# EUROPEAN PATENT APPLICATION

(11) **EP 4 393 916 A1**
(43) Date of publication of application: **03.07.2024**
(21) Application number: 22860666.1
(22) Date of filing: 29.08.2022
(51) Int. Cl.: C07D 401/14, C07D 471/04, C07D 491/04, A61K 31/506, A61P 35/00

(54) **NITROGEN-CONTAINING HETEROCYCLIC DERIVATIVE INHIBITOR, AND PREPARATION METHOD THEREFOR AND USE THEREOF**

(30) Priority: 27.08.2021 CN 202110995982; 11.11.2021 CN 202111334040; 31.12.2021 CN 202111663528
(71) Applicant: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: GAO, Peng, Shanghai 201203 (CN); XIU, Wenhua, Shanghai 201203 (CN); SUN, Guangjun, Shanghai 201203 (CN); CHENG, Fengchang, Shanghai 201203 (CN); YU, Wensheng, Shanghai 201203 (CN)
(74) Representative: EP&C
(86) International application number: PCT/CN2022/115479
(87) International publication number: WO 2023/025320

(57) **Abstract**

Disclosed are a nitrogen-containing heterocyclic derivative inhibitor, and a preparation method therefor and the use thereof. Disclosed are a compound represented by general formula (I), a preparation method therefor, and the use thereof as an EGFR inhibitor in treating cancer.

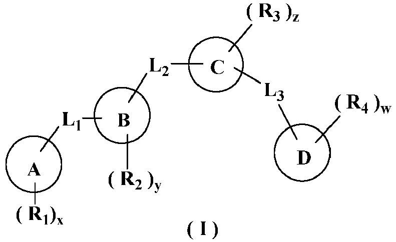

## Description

### Technical Field

The present invention belongs to the field of biomedicine, and specifically relates to a nitrogen-containing heterocyclic derivative inhibitor, and a preparation method therefor and the use thereof.

### Background Art

EGFR (Epidermal Growth Factor Receptor) is a member of the ErbB family of transmembrane receptor tyrosine kinases, which is activated by binding to its ligand epidermal growth factor (EGF) or transforming growth factor α (TGFα). Activated EGFR forms homodimers on the cell membrane, or forms heterodimers with other receptors of the family (such as ErbB-2, ErbB-3, or ErbB-4), resulting in phosphorylation of the key tyrosine residues in EGFR cells, and activation of intracellular downstream signaling pathways, which plays an important role in cell proliferation, survival and anti-apoptosis. Activating mutations, overexpression or gene amplification of EGFR can lead to excessive activation of EGFR, accelerate the transformation of cells into tumor cells, and play an important role in the proliferation, invasion, metastasis and angiopoiesis of tumor cells. It is an important target for the development of anti-cancer drugs, especially drugs for treating lung cancer. First-generation EGFR small molecule inhibitors including gefitinib (Iressa) and erlotinib (Tarceva) have shown good efficacy in the treatment of lung cancer and have been used as first-line drugs for the treatment of non-small cell lung cancer (NSCLC) with EGFR-activating mutations (including L858R and delE746_A750). However, after 10 to 12 months of treatment with first-generation small molecule EGFR inhibitors, almost all NSCLC patients develop resistance to the first-generation small molecule inhibitors, and according to the resistance mechanism, more than half of the cases are due to a secondary mutation in the EGFR gatekeeper gene residue, T790M. Osimertinib (AZD9291) is a third-generation EGFR TKI inhibitor that has a high response rate and good therapeutic effect against drug resistance caused by EGFR T790M mutation. It received accelerated approval from the US FDA in November 2015 and is clinically effective in treating advanced non-small cell lung cancer patients with EGFR T790M drug-resistant mutation. Although osimertinib has achieved great success in clinical treatment of non-small cell lung cancer with EGFR T790M mutation, patients still inevitably develop drug resistance after 9 to 14 months of treatment. Studies have shown that up to 20%-40% of patients are drug-resistant due to the EGFR C797S mutation. The EGFR C797S mutation causes the change of cysteine at position 797 to serine, resulting in the inability of osimertinib to form a covalent bond with the EGFR protein, thus causing drug resistance. Currently, there are no effective clinical inhibitors targeting the EGFR C797S drug-resistant mutation. Therefore, there is an urgent need to develop new highly active EGFR inhibitors to solve the problem of drug resistance caused by EGFR C797S mutation.

Novartis reported compound EAI0450, an EGFR allosteric inhibitor, targeting EGFR C797S drug-resistant mutation. When combined with EGFR monoclonal antibodies such as cetuximab, it showed good anti-tumor effects in a mouse in vivo pharmacodynamic model with L858R/T790M/C797S mutations. However, as a single drug, this compound is ineffective and cannot inhibit C797S drug-resistant mutation (including deIE746_A750), and has not been included in clinical research. In 2017, Ken Uchibori et al. reported that the combination of Brigatinib (AP26113) and EGFR monoclonal antibodies (such as cetuximab) can overcome the problem of drug resistance to third-generation EGFR inhibitors caused by the C797S mutation. In the PC9 (EGFR-C797S/T790M/de119) mouse pharmacodynamic model, it showed good anti-tumor efficacy. However, Brigatinib, as a single drug, also faces the problem of low in vitro activity and no significant anti-tumor activity in vivo, and further clinical research has not been conducted.

Lung cancer is a major disease that threatens human health, and its mortality rate ranks first among all malignant tumors. In China, the incidence of lung cancer is increasing year by year, with about 700,000 new cases every year. The cases of lung cancer with EGFR activating mutations in China account for about 35% of all NSCLC cases. The use of first- or third-generation EGFR inhibitors can achieve good therapeutic effects, but new drug-resistant mutations will occur in the later stages. Therefore, the development of new generation of anti-drug-resistant EGFR inhibitors has huge clinical and market value.

### Summary of the Invention

The objective of the present invention is to provide a compound as represented by general formula (I), or a stereoisomer or pharmaceutically acceptable salt thereof, wherein the compound as represented by general formula (I) has a structure as follows: 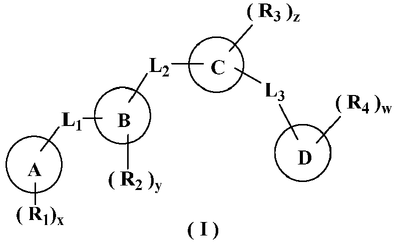 wherein:
ring A, ring B, ring C and ring D are each independently selected from cycloalkyl, heterocyclyl, aryl or heteroaryl;
L₁, L₂ and L₃ are each independently selected from a bond, substituted or unsubstituted alkenyl, substituted or unsubstituted alkynyl, -(CH₂)ₙ-, - (CH₂)ₙC(O)(CRₐₐR_{bb})ₙ₁-, -(CH₂)ₙC(O)NRₐₐ(CH₂)ₙ₁-, -(CH₂)ₙ(CRₐₐR_{bb})ₙ₂-, - (CRₐₐR_{bb})ₙO(CH₂)ₙ₁-, -(CH₂)ₙO(CRₐₐR_{bb})ₙ₁-, -(CRₐₐR_{bb})ₙ₃S(CH₂)ₙ₄-, - (CH₂)ₙS(CRₐₐR_{bb})ₙ₃-, -(CRₐₐR_{bb})ₙ₃(CH₂)ₙNR_{cc}-, -(CH₂)ₙNRₐₐ(CR_{bb}R_{cc})ₙ-, - (CH₂)ₙNRₐₐC(O)-, -(CH₂)ₙP(O)ₚRₐₐ-, -(CH₂)ₙS(O)ₘ-, -(CH₂)ₙS(O)ₘNRₐₐ- or - (CH₂)ₙNRₐₐS(O)ₘ-;
R₁ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, deuteroalkyl, haloalkyl, alkoxy, haloalkoxy, hydroxyalkyl, cycloalkyl, heterocyclyl, aryl and heteroaryl can be optionally further substituted; or, two R₁ are connected to the atoms therebetween to form cycloalkyl, heterocyclyl, aryl and heteroaryl, and the cycloalkyl, heterocyclyl, aryl and heteroaryl can be optionally further substituted;
R₂ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted;
R₃ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted;
R₄ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted;
or, wherein one R₂ and one R₄ are connected to the atoms therebetween to form cycloalkyl, heterocyclyl, aryl or heteroaryl, and the cycloalkyl, heterocyclyl, aryl and heteroaryl can be optionally further substituted;
Rₐ is selected from hydrogen, deuterium, halogen, amino, hydroxyl, cyano, nitro, alkyl, alkenyl, alkynyl, alkoxy, hydroxyalkyl, cycloalkyl, heterocyclyl, aryl or heteroaryl, and the amino, alkyl, alkenyl, alkynyl, alkoxy, hydroxyalkyl, cycloalkyl, heterocyclyl, aryl and heteroaryl can be optionally further substituted;
Rₐₐ, R_{bb} and R_{cc} are each independently selected from hydrogen, deuterium, halogen, amino, hydroxyl, cyano, nitro, alkyl, alkenyl, alkynyl, alkoxy, hydroxyalkyl, cycloalkyl, heterocyclyl, aryl or heteroaryl, and the amino, alkyl, alkenyl, alkynyl, alkoxy, hydroxyalkyl, cycloalkyl, heterocyclyl, aryl and heteroaryl can be optionally further substituted;
x is 0, 1, 2, 3, 4, 5 or 6;
y is 0, 1, 2, 3, 4, 5 or 6;
z is 0, 1, 2, 3, 4, 5 or 6;
w is 0, 1, 2, 3, 4, 5 or 6;
and p, m, n, n1, n2, n3, n4 and n5 are each independently 0, 1, 2 or 3.

In certain embodiments of the present invention, the compound of formula (I) is further as represented by general formula (I-1): 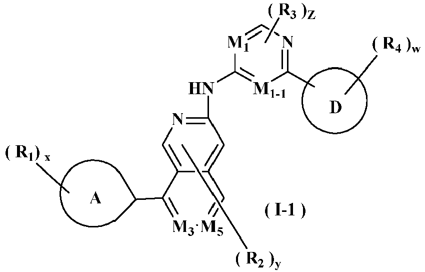 wherein:
M₁ is N or CH;
M₁₋₁ is N or CH;
M₃ is N or CH;
M₅ is N or CH;
ring D is selected from heteroaryl;
ring A is selected from cycloalkyl, heterocyclyl, aryl or heteroaryl;
R₁ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted; or, two R₁ are connected to the atoms therebetween to form cycloalkyl, heterocyclyl, aryl or heteroaryl, and the cycloalkyl, heterocyclyl, aryl and heteroaryl can be optionally further substituted;
R₂ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted;
R₃ is independently selected from hydrogen, deuterium, halogen, amino, hydroxyl, cyano, nitro, alkyl, haloalkyl, deuteroalkyl, alkenyl, alkynyl, alkoxy, deuteroalkoxy, haloalkoxy or hydroxyalkyl; preferably hydrogen, deuterium, halogen, amino, hydroxyl, cyano, nitro, C₁₋₃ alkyl, halo C₁₋₃ alkyl, deuterated C₁₋₃ alkyl, C₂₋₃ alkenyl, C₂₋₃ alkynyl, C₁₋₃ alkoxy, deuterated C₁₋₃ alkoxy, halo C₁₋₃ alkoxy or C₁₋₃ hydroxyalkyl;
R₄ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted;
each Rₐ is independently selected from hydrogen, deuterium, halogen, amino, hydroxyl, cyano, nitro, alkyl, alkenyl, alkynyl, alkoxy, hydroxyalkyl, cycloalkyl, heterocyclyl, aryl or heteroaryl, and the amino, alkyl, alkenyl, alkynyl, alkoxy, hydroxyalkyl, cycloalkyl, heterocyclyl, aryl and heteroaryl can be optionally further substituted;
x is 0, 1, 2, 3, 4, 5 or 6;
y is 0, 1, 2, 3, 4, 5 or 6;
z is 0, 1, 2 or 3;
w is 0, 1, 2, 3, 4, 5 or 6;
and p, m and n5 are each independently 0, 1, 2 or 3.

In certain embodiments of the present invention, the compound of formula (I) is further represented by general formula (II-A), (II-B), (II-C), (II-D), (II-E) or (II-F): 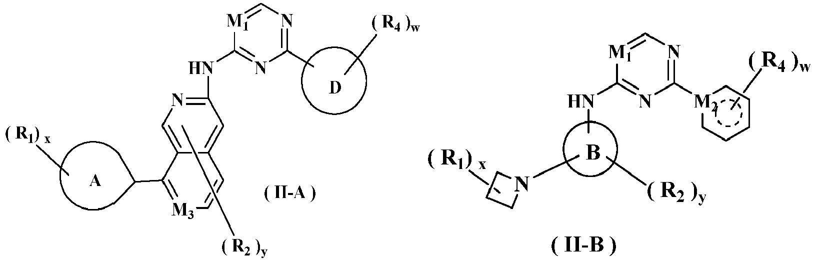 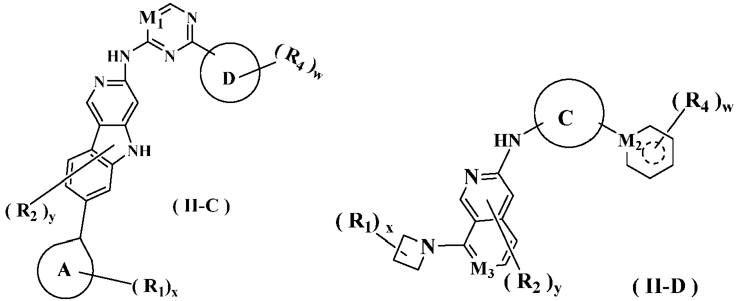 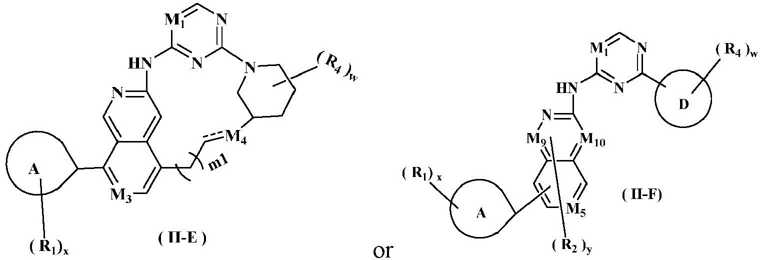 wherein: 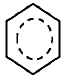
represents a saturated, unsaturated or partially saturated ring;
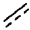 is a single bond or double bond;
M₁ is C, N or CH;
M₂ is N, NH, CH, CH₂, O or S;
M₃ is N or CH;
M₄ is N, NH, CH, CH₂, O or S;
M₅ is N or CH;
M₉ is N or CH;
M₁₀ is N or CH; and
m1 is 0, 1, 2, 3 or 4.

In certain embodiments of the present invention, ring A is selected from C₃₋₁₂ cycloalkyl, 3- to 12-membered heterocyclyl, C₆₋₁₄ aryl or 5- to 14-membered heteroaryl, the heteroatoms in the 3- to 12-membered heterocyclyl and 5- to 14-membered heteroaryl are independently selected from nitrogen, oxygen, sulfur and phosphorus, and the number of the heteroatoms is independently 1, 2, 3 or 4; or, ring A is absent, L₁ and R₁ are directly connected;
preferably, ring A is selected from C₃₋₁₂ cycloalkyl, 3- to 12-membered heterocyclyl, C₆₋₁₄ aryl or 5- to 14-membered heteroaryl; wherein the heteroatoms in the 3- to 12-membered heterocyclyl and 5- to 14-membered heteroaryl are independently selected from nitrogen, oxygen, sulfur and phosphorus, and the number of the heteroatoms is independently 1, 2, 3 or 4; preferably, ring A is 4- to 10-membered heterocyclyl; more preferably, ring A is 4- to 6-membered monocyclic heterocyclyl, 7- to 9-membered spiro heterocyclyl or 8- to 10-membered fused heterocyclyl;
specifically and preferably, ring A is 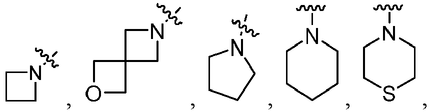 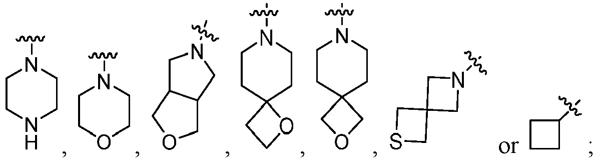 more preferably, ring A is 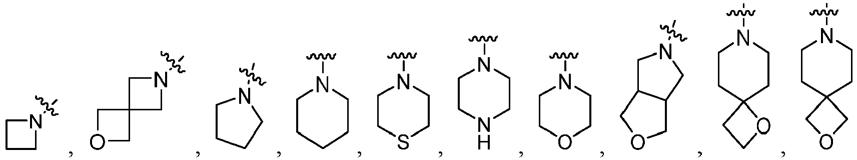 or 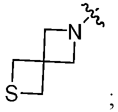
further preferably, ring A is 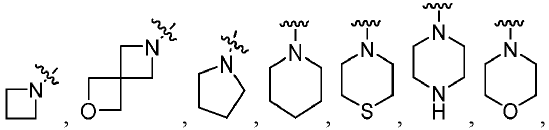 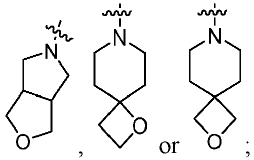
and even further preferably, ring A is

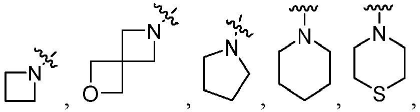

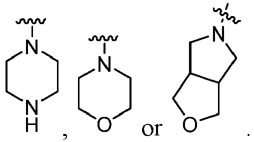

In certain embodiments of the present invention, ring B is selected from C₃₋₁₂ cycloalkyl, 3- to 12-membered heterocyclyl, C₆₋₁₄ aryl or 5- to 14-membered heteroaryl; wherein the heteroatoms in the 3- to 12-membered heterocyclyl and 5- to 14-membered heteroaryl are independently selected from nitrogen, oxygen, sulfur and phosphorus, and the number of the heteroatoms is independently 1, 2, 3 or 4; preferably 5- to 14-membered heteroaryl, and the heteroaryl is monocyclic or fused ring; preferably 8- to 14-membered heteroaryl;
preferably, ring B is 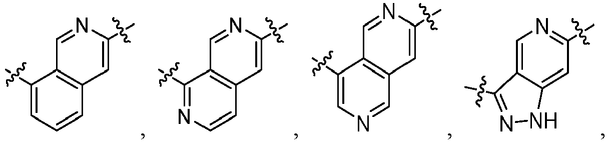 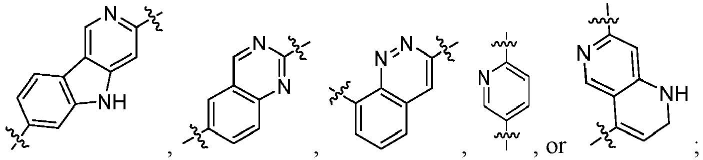
further preferably, ring B is 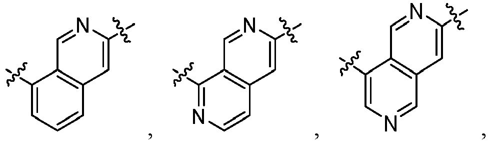 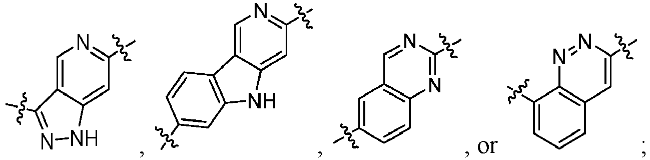
more preferably, ring B is

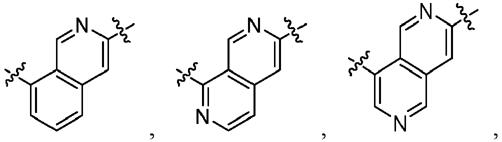

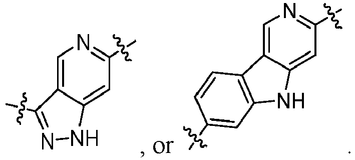

In certain embodiments of the present invention, ring C is selected from C₃₋₁₂ cycloalkyl, 3- to 12-membered heterocyclyl, C₆₋₁₄ aryl or 5- to 14-membered heteroaryl; wherein the heteroatoms in the 3- to 12-membered heterocyclyl and 5- to 14-membered heteroaryl are independently selected from nitrogen, oxygen, sulfur and phosphorus, and the number of the heteroatoms is independently 1, 2, 3 or 4; preferably, ring C is 5- to 6-membered heteroaryl;
more preferably, ring C is 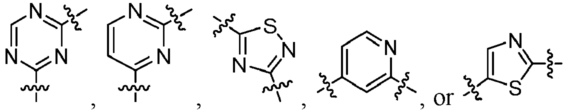
even further preferably, ring C is

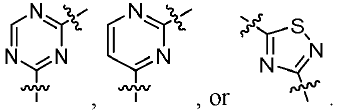

In certain embodiments of the present invention, ring D is selected from C₃₋₁₂ cycloalkyl, 3- to 12-membered heterocyclyl, C₆₋₁₄ aryl or 5- to 14-membered heteroaryl; wherein the heteroatoms in the 3- to 12-membered heterocyclyl and 5- to 14-membered heteroaryl are independently selected from nitrogen, oxygen, sulfur and phosphorus, and the number of the heteroatoms is independently 1, 2, 3 or 4; preferably, ring D is C₃₋₆ cycloalkyl, 5- to 8-membered heterocyclyl, 5- to 8-membered heteroaryl;
more preferably, ring D is C₃₋₆ cycloalkyl, 5- to 8-membered heterocyclyl, 5- to 6-membered heteroaryl; the 5- to 8-membered heterocyclyl is selected from 5- to 6-membered monocyclic heterocyclyl, 7- to 8-membered spiro heterocyclyl or 7- to 8-membered fused heterocyclyl;
more preferably, ring D is 5-membered heteroaryl, 6-membered heteroaryl, 5- to 6-membered monocyclic heterocyclyl, 7- to 8-membered spiro heterocyclyl or 7- to 8-membered fused heterocyclyl;
preferably, ring D is 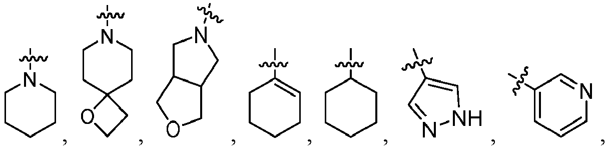 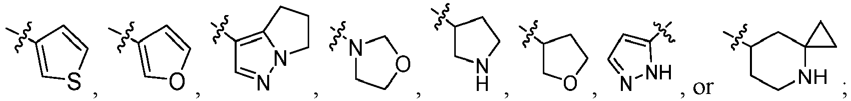
more preferably, ring D is

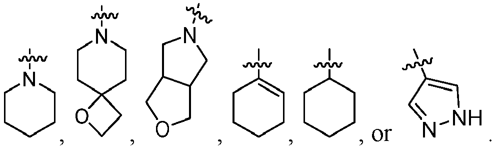

In certain embodiments of the present invention, the compound of formula (I) is further represented by general formula (II), (III), (IV), (V), (VI), (VII), (VIII), (IX) or (X): 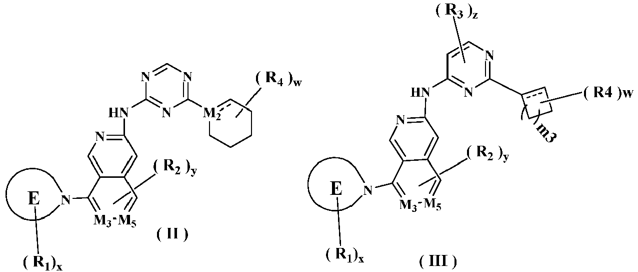 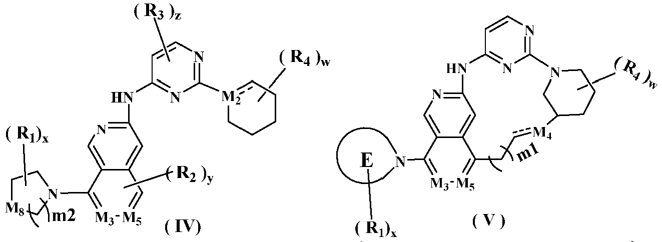 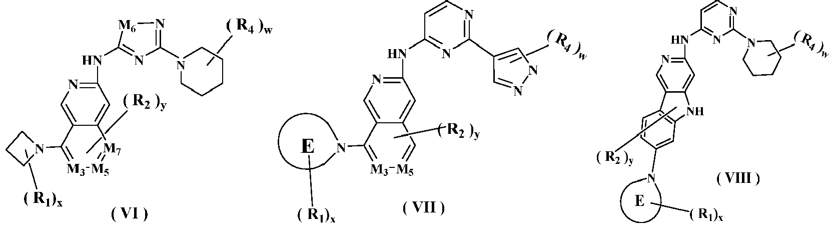 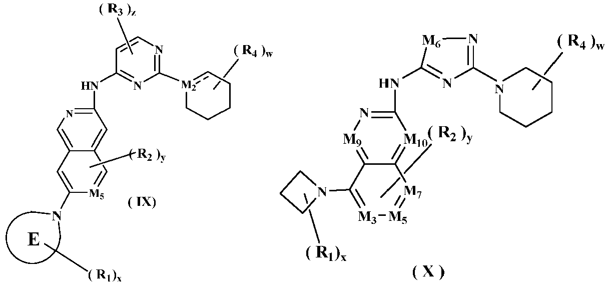
wherein, 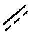 is single bond or double bond;
M₁ is C, N or CH;
M₂ is N, NH, CH, CH₂, O or S;
M₃ is N or CH;
M₄ is N, NH, CH, CH₂, O or S;
M₅ is N or CH;
M₆ is S or CH=CH;
M₇ is a bond, N, NH or CH;
M₈ is NH, CH₂, O or S;
M₉ is N or CH;
M₁₀ is N or CH;
ring E is 4- to 10-membered heterocyclyl; preferably, ring E is 4- to 10-membered heterocyclyl containing 1-4 heteroatoms selected from N, O, S or P;
m1 is 0, 1, 2, 3 or 4;
m2 is 1, 2 or 3;
and m3 is 1, 2, 3, 4, 5 or 6.

In certain embodiments of the present invention, for general formula (VI), when M₆ is CH=CH and M₇ is CH, R₁ is not substituted or unsubstituted -CH₂S(O)₂Rₐ.

In certain embodiments of the present invention, for general formula (VI), when M₆ is CH=CH and M₇ is CH, R₁ is not -CH₂S(O)₂Rₐ.

In certain embodiments of the present invention, for general formula (VI), when M₆ is CH=CH and M₇ is CH, R₁ is not -CH₂S(O)₂CH₃, -CHCH₃S(O)₂CH₃, or - CH₂S(O)₂CH₂CH₃.

In certain embodiments of the present invention, ring E is selected from 4- to 6-membered monocyclic heterocyclyl, 7- to 9-membered spiro heterocyclyl or 8- to 10-membered fused heterocyclyl; preferably, ring E is 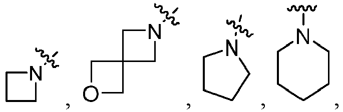 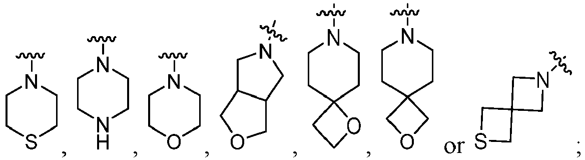 preferably, ring E is 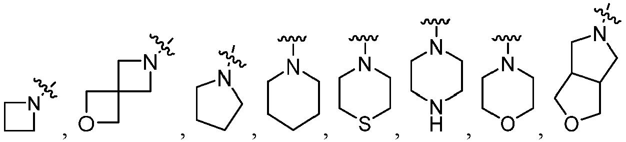 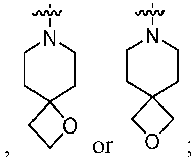 and further preferably, ring E is the group as follows:

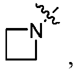

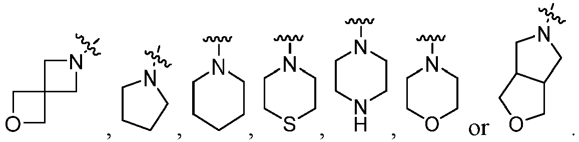

In certain embodiments of the present invention, the compound is further represented by general formula (VII-1) or (VIII-1): 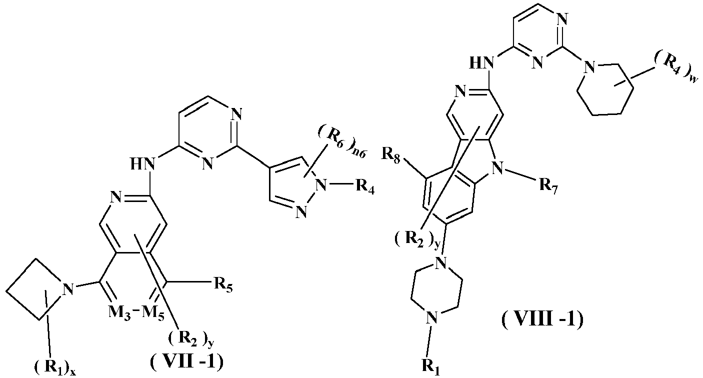
wherein R₅, R₆, R₇ and R₈ are independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ haloalkoxy, or C₁₋₆ hydroxyalkyl; preferably, R₅, R₆, R₇ and R₈ are independently selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ haloalkoxy or C₁₋₆ hydroxyalkyl;
and n6 is 0, 1 or 2.

In certain embodiments of the present invention, R₅ and R₇ are independently methyl, ethyl, n-propyl or isopropyl, preferably isopropyl.

In certain embodiments of the present invention, R₈ is -OCH₂CF₃, -OCH₂CHF₂, - OCH₂CH₂F, -OCHFCF₃, or -OCF₂CF₃, preferably -OCH₂CF₃.

In certain embodiments of the present invention, n6 is 0.

In certain embodiments of the present invention, L₁ is selected from a bond, substituted or unsubstituted C₂₋₆ alkenyl, substituted or unsubstituted C₂₋₆ alkynyl, -(CH₂)ₙ-, - (CH₂)ₙC(O)(CRₐₐR_{bb})ₙ₁-, -(CH₂)ₙC(O)NRₐₐ(CH₂)ₙ₁-, -(CH₂)ₙ(CRₐₐR_{bb})ₙ₂-, - (CRₐₐR_{bb})ₙO(CH₂)ₙ₁-, -(CH₂)ₙO(CRₐₐR_{bb})ₙ₁-, -(CRₐₐR_{bb})ₙ₃S(CH₂)ₙ₄-, - (CH₂)ₙS(CRₐₐR_{bb})ₙ₃-, -(CRₐₐR_{bb})ₙ₃(CH₂)ₙNR_{cc}-, -(CH₂)ₙNRₐₐ(CR_{bb}R_{cc})ₙ-, - (CH₂)ₙNRₐₐC(O)-, -(CH₂)ₙP(O)ₚRₐₐ-, -(CH₂)ₙS(O)ₘ-, -(CH₂)ₙS(O)ₘNRₐₐ- and - (CH₂)ₙNRₐₐS(O)ₘ-; preferably a bond.

In certain embodiments of the present invention, R₁ is independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, - S(O)ₘRₐ or -C(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5-to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)ₚ(Rₐₐ)ₙ₅, -S(O)ₘRₐₐ, -Se(O)ₘRₐₐ or -C(O)Rₐₐ; or, two R₁ are connected to the atoms therebetween to form C₃₋₁₂ cycloalkyl, 3- to 12-membered heterocyclyl, C₆₋₁₄ aryl and 5- to 14-membered heteroaryl, and the C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, or C₁₋₆ hydroxyalkyl;
preferably, R₁ is independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or -C(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)ₚ(Rₐₐ)ₙ₅, -S(O)ₘRₐₐ or -C(O)Rₐₐ;
preferably, R₁ is independently selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₈ cycloalkyl, 3- to 10-membered heterocyclyl, C₆₋₁₀ aryl, 5- to 12-membered heteroaryl, -ORₐ, -P(O)ₚ(Rₐ)ₙ₅, -S(O)ₘRₐ or -C(O)Rₐ, and the amino, C₁₋₃ alkyl, C₂₋₃ alkenyl, C₂₋₃ alkynyl, C₁₋₆ alkoxy, C₁₋₆ hydroxyalkyl, C₃₋₈ cycloalkyl, 3- to 10-membered heterocyclyl, C₆₋₁₀ aryl and 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₈ cycloalkyl, 3- to 8-membered heterocyclyl, C₆₋₁₀ aryl, 5- to 10-membered heteroaryl, -ORₐₐ, -P(O)ₚ(Rₐₐ)ₙ₅, -S(O)ₘRₐₐ or -C(O)Rₐₐ;
or, two R₁ are connected to the atoms therebetween to form C₃₋₁₂ cycloalkyl, 3- to 12-membered heterocyclyl, C₆₋₁₄ aryl or 5- to 14-membered heteroaryl, and the C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, or C₁₋₆ hydroxyalkyl.

Preferably, two R₁ are connected to the atoms therebetween to form C₃₋₁₀ cycloalkyl, 3- to 10-membered heterocyclyl, C₆₋₁₂ aryl or 5- to 12-membered heteroaryl, and the C₃₋₁₀ cycloalkyl, 3- to 10-membered heterocyclyl, C₆₋₁₀ aryl and 5-to 10-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, or C₁₋₃ hydroxyalkyl.

In certain embodiments of the present invention, R₁ is independently selected from - NHS(O)₂CH₃, -NCH₃S(O)₂CH₃, -CH₂S(O)₂N(CH₃)₂, -CH₂Se(O)₂CH₃, -CH₂S(O)₂CH₃, -CH₂SOCH₃, -CH₂NO₂, methyl, hydrogen, 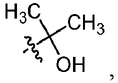 methoxy, cyano, -CH₂OCH₃, - CH₂CN, -CH(CN)₂, -S(O)₂CH₃, oxo, hydroxyl, - CH₂COCH₃, -COCH₃, - CH₂P(O)(CH₃)₂,

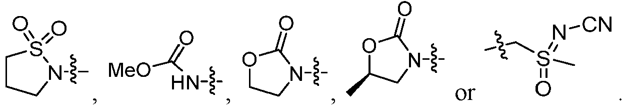

In certain embodiments of the present invention, R₁ is independently - CH₂S(O)₂N(CH₃)₂, -CH₂Se(O)₂CH₃, -CH₂S(O)₂CH₃, methyl, hydrogen, 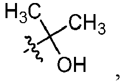 methoxy, cyano, -CH₂OCH₃, -CH₂CN, -CH(CN)₂, -S(O)₂CH₃, oxo, hydroxyl, - CH₂COCH₃, -COCH₃, or -CH₂P(O)(CH₃)₂; or, two R₁ are connected to the atoms therebetween to form 3- to 12-membered heterocyclyl, preferably 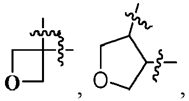 or

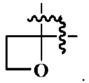

In certain embodiments of the present invention, R₁ is independently -CH₂S(O)₂CH₃, methyl, hydrogen, 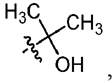 methoxy, cyano, -CH₂OCH₃, -CH₂CN, -S(O)₂CH₃, oxo, hydroxyl, - CH₂COCH₃, -COCH₃, or -CH₂P(O)(CH₃)₂; or, two R₁ are connected to the atoms therebetween to form 3- to 12-membered heterocyclyl, preferably

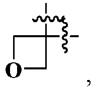

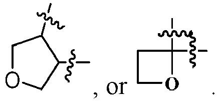

In certain embodiments of the present invention, R₁ is independently -CH₂S(O)₂CH₃, methyl, hydrogen, 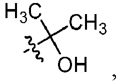 methoxy, cyano, -CH₂OCH₃, -CH₂CN, -S(O)₂CH₃, oxo, hydroxyl, -COCH₃, or -CH₂P(O)(CH₃)₂; or, two R₁ are connected to the atoms therebetween to form 3- to 12-membered heterocyclyl, preferably

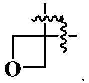

In certain embodiments of the present invention, L₂ is selected from a bond, substituted or unsubstituted C₂₋₆ alkenyl, substituted or unsubstituted C₂₋₆ alkynyl, -(CH₂)ₙ-, - (CH₂)ₙC(O)(CRₐₐR_{bb})ₙ₁-, -(CH₂)ₙC(O)NRₐₐ(CH₂)ₙ₁-, -(CH₂)ₙ(CRₐₐR_{bb})ₙ₂-, - (CRₐₐR_{bb})ₙO(CH₂)ₙ₁-, -(CH₂)ₙO(CRₐₐR_{bb})ₙ₁-, -(CRₐₐR_{bb})ₙ₃S(CH₂)ₙ₄-, - (CH₂)ₙS(CRₐₐR_{bb})ₙ₃-, -(CRₐₐR_{bb})ₙ₃(CH₂)ₙNR_{cc}-, -(CH₂)ₙNRₐₐ(CR_{bb}R_{cc})ₙ-, - (CH₂)ₙNRₐₐC(O)-, -(CH₂)ₙP(O)ₚRₐₐ-, -(CH₂)ₙS(O)ₘ-, -(CH₂)ₙS(O)ₘNRₐₐ- and - (CH₂)ₙNRₐₐS(O)ₘ-; preferably -NH-.

In certain embodiments of the present invention, R₂ is independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, - S(O)ₘRₐ or -C(O)Rₐ, and the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)ₚ(Rₐₐ)ₙ₅, -S(O)ₘRₐₐ or -C(O)Rₐₐ; preferably, R₂ is independently selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, - ORₐ, -P(O)ₚ(Rₐ)ₙ₅, -S(O)ₘRₐ or -C(O)Rₐ, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl and 5- to 12-membered heteroaryl can be optionally further substituted with one or more of hydrogen, deuterium, oxo, thio, halogen, amino, hydroxyl, cyano, nitro, C₁₋₂ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl, 5- to 10-membered heteroaryl, -ORₐₐ, -P(O)ₚ(Rₐₐ)ₙ₅, -S(O)ₘRₐₐ or -C(O)Rₐₐ; more preferably, R₂ is independently isopropyl, cyano, trifluoromethyl, or -OCH₂CF₃; further preferably, R₂ is independently isopropyl or - OCH₂CF₃.

In certain embodiments of the present invention, R₂ is independently oxo, fluorine, isopropyl, cyano, trifluoromethyl, -NHCH(CH)₃, or -OCH₂CF₃.

In certain embodiments of the present invention, L₃ is selected from a bond, substituted or unsubstituted C₂₋₆ alkenyl, substituted or unsubstituted C₂₋₆ alkynyl, -(CH₂)ₙ-, - (CH₂)ₙC(O)(CRₐₐR_{bb})ₙ₁-, -(CH₂)ₙC(O)NRₐₐ(CH₂)ₙ₁-, -(CH₂)ₙ(CRₐₐR_{bb})ₙ₂-, - (CRₐₐR_{bb})ₙO(CH₂)ₙ₁-, -(CH₂)ₙO(CRₐₐR_{bb})ₙ₁-, -(CRₐₐR_{bb})ₙ₃S(CH₂)ₙ₄-, - (CH₂)ₙS(CRₐₐR_{bb})ₙ₃-, -(CRₐₐR_{bb})ₙ₃(CH₂)ₙNR_{cc}-, -(CH₂)ₙNRₐₐ(CR_{bb}R_{cc})ₙ-, - (CH₂)ₙNRₐₐC(O)-, -(CH₂)ₙP(O)ₚRₐₐ-, -(CH₂)ₙS(O)ₘ-, -(CH₂)ₙS(O)ₘNRₐₐ- and - (CH₂)ₙNRₐₐS(O)ₘ-; preferably a bond.

In certain embodiments of the present invention, R₃ is independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, - S(O)ₘRₐ or -C(O)Rₐ, and the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)ₚ(Rₐₐ)ₙ₅, -S(O)ₘRₐₐ or -C(O)Rₐₐ; preferably, R₃ is independently selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, - ORₐ, -P(O)ₚ(Rₐ)ₙ₅, -S(O)ₘRₐ or -C(O)Rₐ, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl and 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl, 5- to 10-membered heteroaryl, -ORₐₐ, -P(O)ₚ(Rₐₐ)ₙ₅, -S(O)ₘRₐₐ or -C(O)Rₐₐ; more preferably, R₃ is hydrogen.

In certain embodiments of the present invention, R₃ is independently selected from hydrogen, methoxy, methyl, fluorine, chlorine, bromine, trifluoromethyl, cyano, cyclopropyl or -COCH₃.

In certain embodiments of the present invention, R₄ is independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, - S(O)ₘRₐ or -C(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5-to 14-membered heteroaryl can be optionally further substituted with one or more R₄₋₁; R₄₋₁ is independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)ₚ(Rₐₐ)ₙ₅, -S(O)ₘRₐₐ or -C(O)Rₐₐ; optionally, R₄₋₁ is substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl, and the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, and C₁₋₆ hydroxyalkyl are optionally substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, or C₁₋₆ hydroxyalkyl.

In certain embodiments of the present invention, R₄ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, -ORₐ, -P(O)ₚ(Rₐ)ₙ₅, - S(O)ₘRₐ or -C(O)Rₐ, and the amino, C₁₋₃ alkyl, C₂₋₄ alkenyl, C₂₋₄ alkynyl, C₁₋₃ alkoxy, C₁₋₃ hydroxyalkyl, C₃₋₁₀ cycloalkyl, 3- to 10-membered heterocyclyl, C₆₋₁₂ aryl and 5-to 12-membered heteroaryl can be optionally further substituted with one or more R₄₋₁; R₄₋₁ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, -ORₐₐ, -P(O)ₚ(Rₐₐ)ₙ₅, -S(O)ₘRₐₐ or -C(O)Rₐₐ; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₂ aryl, or 5- to 12-membered heteroaryl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl.

In certain embodiments of the present invention, R₄ is independently fluorine, methyl, hydroxyl, methoxy, -OCD₃, -CH₂F, -CHF₂, CH₂CF₃, -CH₂CH₂F, hydrogen, - CH₂OCH₃, -CF₃, amino, oxo, -COCH₃, 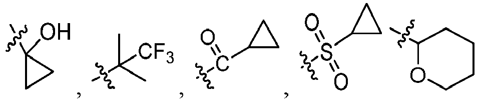 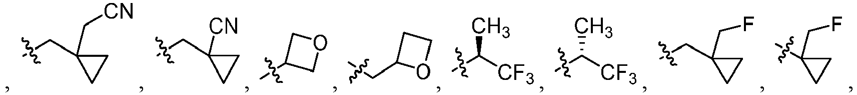 or

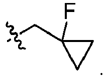

In certain embodiments of the present invention, R₄ is independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, - S(O)ₘRₐ or -C(O)Rₐ, and the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)ₚ(Rₐₐ)ₙ₅, -S(O)ₘRₐₐ or -C(O)Rₐₐ;
preferably, R₄ is independently selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, -ORₐ, -P(O)ₚ(Rₐ)ₙ₅, -S(O)ₘRₐ or -C(O)Rₐ, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl and 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl, 5- to 10-membered heteroaryl, -ORₐₐ, -P(O)ₚ(Rₐₐ)ₙ₅, -S(O)ₘRₐₐ or -C(O)Rₐₐ; more preferably, R₄ is fluorine, methyl, hydroxyl, methoxy, -OCD₃, hydrogen, 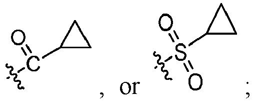 further preferably, R₄ is fluorine, methyl, hydroxyl, methoxy, -OCD₃, hydrogen, or

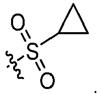

In certain embodiments of the present invention, R₄ is independently selected from fluorine, chlorine, cyano, trifluoromethyl, methyl, ethyl, nitro, hydroxyl, methoxy, - OCD₃, hydrogen, 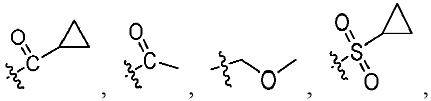 cyclopropyl, 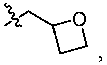 difluoromethyl,

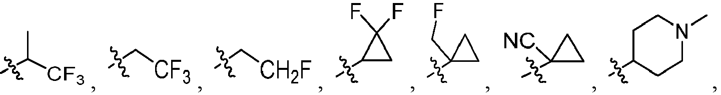

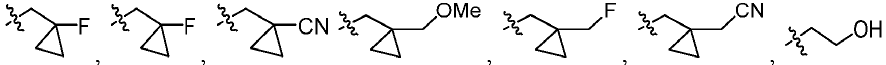

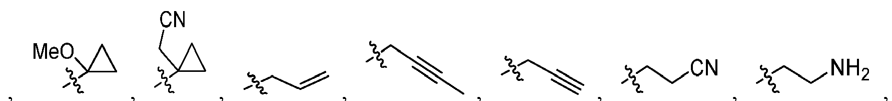

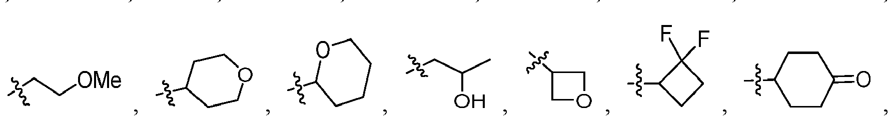

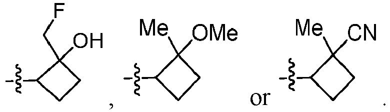

In certain embodiments of the present invention, one R₂ and one R₄ are connected to the atoms therebetween to form C₃₋₁₂ cycloalkyl, 3- to 12-membered heterocyclyl, C₆₋₁₄ aryl or 5- to 14-membered heteroaryl, and the C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl; preferably, -R₂-R₄- is

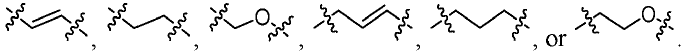

In certain embodiments of the present invention, Rₐ is independently selected from hydrogen, deuterium, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl. Preferably, Rₐ is independently selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, or 5- to 12-membered heteroaryl, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl, or 5- to 10-membered heteroaryl.

In certain embodiments of the present invention, Rₐₐ, R_{bb} and R_{cc} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl, preferably, Rₐₐ and R_{bb} are independently selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, or 5- to 12-membered heteroaryl, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl, or 5- to 10-membered heteroaryl.

In certain embodiments of the present invention, the compound is further represented by general formula (VII-2): 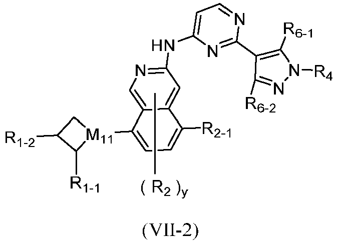 wherein,
M₁₁ is CH or N;
R₁₋₁ is selected from hydrogen, deuterium, fluorine, chlorine, bromine, methyl, ethyl, halomethyl, or haloethyl;
R₁₋₂ is selected from amino, hydroxyl, cyano, C₁₋₃ alkoxy, C₁₋₃ alkyl, C₃₋₆ cycloalkyl, or 3- to 8-membered heterocyclyl, and the amino, C₁₋₃ alkoxy, C₁₋₃ alkyl, C₃₋₆ cycloalkyl, and 3- to 8-membered heterocyclyl can be optionally further substituted with one or more of hydrogen, deuterium, oxo, thio, halogen, amino, hydroxyl, cyano, nitro, C₁₋₃ alkyl, C₁₋₃ deuteroalkyl, C₁₋₃ haloalkyl, C₁₋₃ alkoxy, halo C₁₋₃ alkoxy, C₁₋₃ hydroxyalkyl, -C(O)Rₑₑ, -ORₑₑ, -P(O)ₚ(Rₑₑ)ₙ₅, -S(O)ₘRₑₑ, - S(O)₂N(Rₑₑ)₂ and -S(=O)(=NCN)Rₑₑ; preferably, R₁₋₂ is selected from -NHS(O)₂CH₃, - NCH₃S(O)₂CH₃, -CH₂S(O)₂N(CH₃)₂, -CH₂S(O)₂CH₃, -CH₂SOCH₃, -CH₂NO₂, methyl, hydrogen, 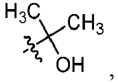 methoxy, cyano, -CH₂OCH₃, -CH₂CN, -CH(CN)₂, hydroxyl, - CH₂COCH₃, 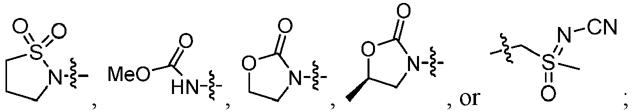
R₂ is independently selected from hydrogen, deuterium, fluorine, chlorine, bromine, methyl, ethyl, halomethyl, or haloethyl;
R₂₋₁ is selected from amino, C₁₋₃ alkyl, halo C₁₋₃ alkyl or deuterated C₁₋₃ alkyl, and the amino, C₁₋₃ alkyl, halo C₁₋₃ alkyl and deuterated C₁₋₃ alkyl can be optionally further substituted with one or more of deuterium, halogen, cyano, hydroxyl, nitro, C₁₋₃ alkyl, halo C₁₋₃ alkyl or deuterated C₁₋₃ alkyl;
R₄ is selected from halogen, amino, hydroxyl, cyano, nitro, C₁₋₃ alkyl, C₁₋₃ alkoxy, C₁₋₃ hydroxyalkyl, C₂₋₄ alkenyl, C₂₋₄ alkynyl, C₃₋₆ cycloalkyl, 3- to 8-membered heterocyclyl, -S(O)ₘR_{d} or -C(O)R_{d}, and the amino, C₁₋₃ alkyl, C₁₋₃ alkoxy, C₁₋₃ hydroxyalkyl, C₂₋₄ alkenyl, C₂₋₄ alkynyl, C₃₋₆ cycloalkyl and 3- to 8-membered heterocyclyl can be optionally further substituted with one or more R₄₋₁;
R₄₋₁ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl or 5- to 12-membered heteroaryl; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl, or 5- to 12-membered heteroaryl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl; preferably, R₄ is selected from fluorine, chlorine, cyano, trifluoromethyl, methyl, ethyl, nitro, hydroxyl, methoxy, -OCD₃, hydrogen, 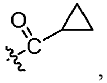 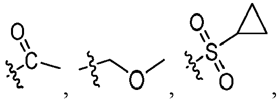 cyclopropyl, 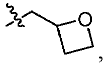 difluoromethyl, 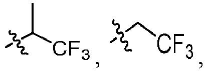 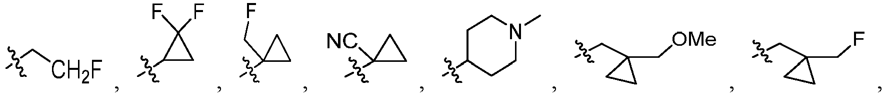 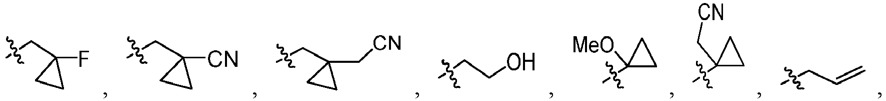 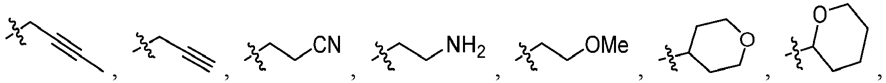 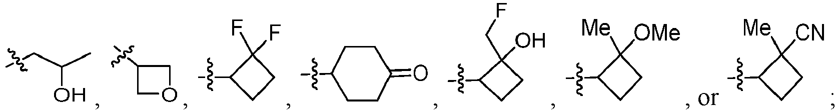
R₆₋₁ is selected from hydrogen, deuterium, oxo, thio, halogen, amino, hydroxyl, cyano, nitro, C₁₋₃ alkyl, C₁₋₃ haloalkyl, C₁₋₃ deuteroalkyl, C₁₋₃ alkoxy, or C₁₋₃ hydroxyalkyl;
R₆₋₂ is selected from hydrogen, deuterium, oxo, thio, halogen, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, -S(O)ₘRₑ or -C(O)Rₑ, and the amino, C₁₋₃ alkyl, C₁₋₃ alkoxy, C₁₋₃ hydroxyalkyl, C₃₋₆ cycloalkyl and 3- to 6-membered heterocyclyl can be optionally further substituted with one or more of deuterium, halogen, amino, hydroxyl, cyano, nitro and C₁₋₃ alkyl;
R_{d} is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
Rₑ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
Rₑₑ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
m is 0, 1 or 2;
p is 0, 1 or 2;
n5 is 0, 1 or 2;
and y is 0, 1, 2, 3 or 4.

In certain embodiments of the present invention, the compound is further represented by general formula (VII-3): 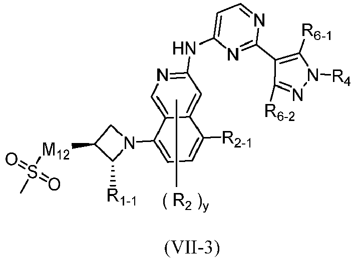 wherein,
M₁₂ is selected from a bond, NR₉ or CR₁₀R₁₁;
R₉ is selected from hydrogen, deuterium, methyl, ethyl, monofluoromethyl, difluoromethyl or trifluoromethyl;
R₁₀ is selected from hydrogen, deuterium, fluorine, chlorine, bromine, methyl, ethyl, monofluoromethyl, difluoromethyl or trifluoromethyl;
R₁₁ is selected from hydrogen, deuterium, fluorine, chlorine, bromine, methyl, ethyl, monofluoromethyl, difluoromethyl or trifluoromethyl;
R₁₋₁ is selected from hydrogen, deuterium, fluorine, chlorine, bromine, methyl, ethyl, monofluoromethyl, difluoromethyl or trifluoromethyl;
R₂ is independently selected from hydrogen, deuterium, fluorine, chlorine, bromine, methyl, ethyl, monofluoromethyl, difluoromethyl or trifluoromethyl;
R₂₋₁ is selected from amino, C₁₋₃ alkyl, halo C₁₋₃ alkyl or deuterated C₁₋₃ alkyl, and the amino, C₁₋₃ alkyl, halo C₁₋₃ alkyl or deuterated C₁₋₃ alkyl can be optionally further substituted with one or more of deuterium, halogen, cyano, hydroxyl, nitro, C₁₋₃ alkyl, halo C₁₋₃ alkyl and deuterated C₁₋₃ alkyl;
preferably, R₂₋₁ is selected from isopropyl, -CH(Me)OMe, or -N(Me)₂;
R₄ is selected from halogen, amino, hydroxyl, cyano, nitro, C₁₋₃ alkyl, C₁₋₃ alkoxy, C₁₋₃ hydroxyalkyl, C₂₋₄ alkenyl, C₂₋₄ alkynyl, C₃₋₆ cycloalkyl, 3- to 8-membered heterocyclyl, -S(O)ₘR_{d} or -C(O)R_{d}, and the amino, C₁₋₃ alkyl, C₁₋₃ alkoxy, C₁₋₃ hydroxyalkyl, C₂₋₄ alkenyl, C₂₋₄ alkynyl, C₃₋₆ cycloalkyl and 3- to 8-membered heterocyclyl can be optionally further substituted with one or more R₄₋₁;
R₄₋₁ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl or 5- to 12-membered heteroaryl; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl, or 5- to 12-membered heteroaryl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl; preferably, R₄ is independently selected from fluorine, chlorine, cyano, trifluoromethyl, methyl, ethyl, nitro, hydroxyl, methoxy, -OCD₃, hydrogen, 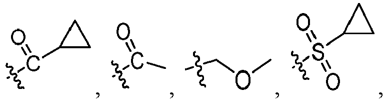 cyclopropyl, 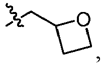 difluoromethyl, 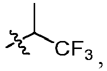 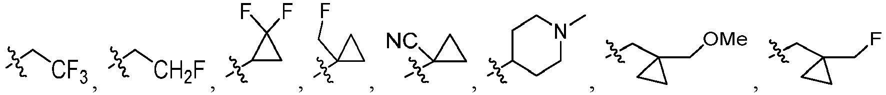 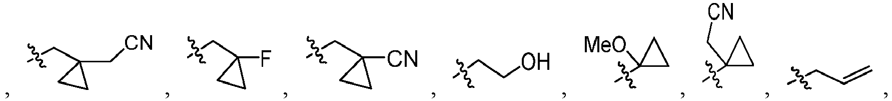 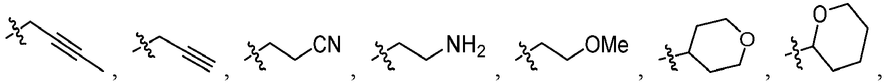 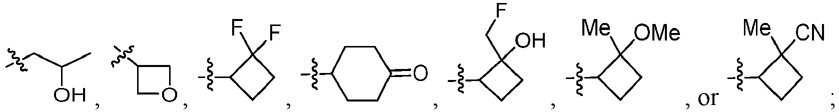
R₆₋₁ is selected from hydrogen, deuterium, oxo, thio, halogen, amino, hydroxyl, cyano, nitro, C₁₋₃ alkyl, C₁₋₃ alkoxy, or C₁₋₃ hydroxyalkyl;
R₆₋₂ is selected from hydrogen, deuterium, oxo, thio, halogen, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, -S(O)ₘRₑ or -C(O)Rₑ, and the amino, C₁₋₃ alkyl, C₁₋₃ alkoxy, C₁₋₃ hydroxyalkyl, C₃₋₆ cycloalkyl and 3- to 8-membered heterocyclyl can be optionally further substituted with one or more of deuterium, halogen, amino, hydroxyl, cyano, nitro, or C₁₋₃ alkyl;
R_{d} is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl, or C₁₋₃ deuteroalkyl;
Rₑ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl, or C₁₋₃ deuteroalkyl;
m is 0, 1 or 2;
and y is 0, 1, 2, 3 or 4.

The present invention further relates to the use of the compounds of various general formulas as mentioned above, or the stereoisomers or pharmaceutically acceptable salts thereof in the preparation of EGFR inhibitor drugs.

The present invention further relates to the use of the compounds of various general formulas as mentioned above, or the stereoisomers or pharmaceutically acceptable salts thereof in the preparation of drugs for treating cancer, wherein, the cancer is non-small cell lung cancer.

The present invention further relates to a method for preventing and/or treating EGFR-related diseases, which comprises administering to the patient a therapeutically effective amount of compounds of various general formulas, or the stereoisomers or pharmaceutically acceptable salts thereof, wherein the EGFR-related disease is non-small cell lung cancer.

In certain embodiments of the present invention, the EGFR is a mutated EGFR, preferably with one or more mutations of Del19, L858R, T790M or C797S, and more preferably with Del19, L858R, L858R/T790M, Del19/T790M, Del19/C797S, L858R/C797S, Del19/T790M/C797S or L858R/T790M/C797S mutation.

In certain embodiments of the present invention, the cancer is a cancer with EGFR Del19, L858R, L858R/T790M, Del19/T790M, Del19/C797S, L858R/C797S, Del19/T790M/C797S or L858R/T790M/C797S mutation.

### Detailed Description of the Invention

Unless stated to the contrary, the terms used in the description and claims have the following meanings.

The term "alkyl" refers to a saturated aliphatic hydrocarbon group, which is a straight or branched group containing 1 to 20 carbon atoms, preferably alkyl containing 1 to 8 carbon atoms, more preferably alkyl containing 1 to 6 carbon atoms, and most preferably alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. More preferably, the alkyl is a lower alkyl group containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl can be substituted or unsubstituted, and when substituted, the substituent can be substituted at any available connection point, and the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or a carboxylate group. In the present invention, alkyl is preferably methyl, ethyl, isopropyl, tert-butyl, haloalkyl, deuteroalkyl, alkoxy-substituted alkyl and hydroxyl-substituted alkyl.

The term "alkylene" refers to one hydrogen atom of alkyl being further substituted, for example: "methylene" refers to -CH₂-, "ethylene" refers to -(CH₂)₂-, "propylene" refers to -(CH₂)₃-, and "butylene" refers to -(CH₂)₄-, etc. The term "alkenyl" refers to an alkyl group as defined above consisting of at least two carbon atoms and at least one carbon-carbon double bond, such as ethenyl, 1-propenyl, 2-propenyl, 1-, 2- or 3 -butenyl, etc. Alkenyl can be substituted or unsubstituted. When the alkenyl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The term "cycloalkyl" refers to a saturated or partially unsaturated monocyclic or polycyclic cyclic hydrocarbon substituent, and a cycloalkyl ring comprises 3 to 20 carbon atoms, preferably 3 to 12 carbon atoms, preferably 3 to 8 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl includes spiro, fused and bridged cycloalkyl, preferably cyclopropyl, cyclobutyl, cyclohexyl, cyclopentyl and cycloheptyl.

The term "spirocycloalkyl" refers to a polycyclic group with 5 to 20 membered monocyclic rings sharing one carbon atom (called a spiro atom). It may contain one or more double bonds, but no ring has a completely conjugated π electron system. Preferably, the spirocycloalkyl is 6- to 14-membered, and more preferably 7- to 10-membered. According to the number of shared spiro atoms between the rings, the spirocycloalkyl is divided into monospirocycloalkyl, bispirocycloalkyl or polyspirocycloalkyl, preferably monospirocycloalkyl and bispirocycloalkyl. More preferably, it is a 3-membered/6-membered, 3-membered/5-membered, 4-membered/4-membered, 4-membered/5-membered, 4-membered/6-membered, 5-membered/5-membered or 5-membered/6-membered monospirocycloalkyl. Non-limiting examples of spirocycloalkyl include: 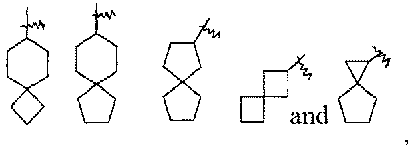 etc.;
Also included are spirocycloalkyl groups in which monospirocycloalkyl and heterocycloalkyl share a spiro atom. Non-limiting examples include: 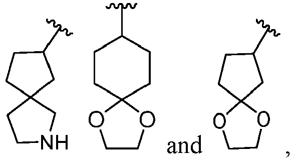 etc.

The term "fused cycloalkyl" refers to a 5- to 20-membered all-carbon polycyclic group in which each ring in the system shares an adjacent pair of carbon atoms with other rings in the system, one or more ring of which may contain one or more double bonds, but no ring has a fully conjugated π electron system. Preferably, the fused cycloalkyl is 6- to 14-membered, and more preferably 7- to 10-membered. According to the number of constituent rings, it can be divided into bicyclic, tricyclic, tetracyclic or polycyclic fused cycloalkyl, preferably bicyclic or tricyclic, and more preferably 5-membered/5-membered or 5-membered/6-membered bicyclic cycloalkyl. Non-limiting examples of fused cycloalkyl include: 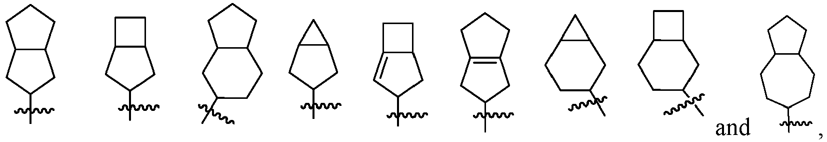 etc.

The term "bridged cycloalkyl" refers to a 5- to 20-membered all-carbon polycyclic group with any two rings sharing two carbon atoms that are not directly connected. It may contain one or more double bonds, but no ring has a fully conjugated π electron system. Preferably, the bridged cycloalkyl is 6- to 14-membered, and more preferably 7- to 10-membered. According to the number of constituent rings, it can be divided into bicyclic, tricyclic, tetracyclic or polycyclic bridged cycloalkyl, preferably bicyclic, tricyclic or tetracyclic, and more preferably bicyclic or tricyclic. Non-limiting examples of bridged cycloalkyl include:

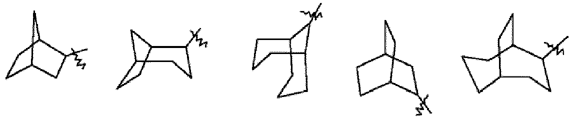

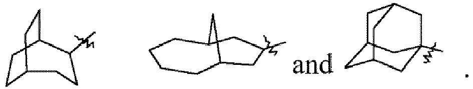

The cycloalkyl ring can be fused to an aryl, heteroaryl or heterocycloalkyl ring, wherein the ring connected to the parent structure is cycloalkyl, and the non-limiting examples include indanyl, tetrahydronaphthyl, benzocycloheptanyl, etc. Cycloalkyl can be optionally substituted or unsubstituted. When the cycloalkyl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or a carboxylate group.

The term "heterocyclyl" refers to a saturated or partially unsaturated monocyclic or polycyclic cyclic hydrocarbon substituent, which comprises 3 to 20 ring atoms, wherein one or more ring atoms are heteroatoms selected from nitrogen, oxygen, P(O)ₚₚ or S(O)ₘₘ (wherein pp and mm are integers of 0 to 2), but excluding ring moieties of -O-O-, -O-S- or -S-S-, and the remaining ring atoms are carbon. Preferably, the heterocyclyl comprises 3 to 12 ring atoms, of which 1-4 are heteroatoms; more preferably, the heterocyclyl comprises 3 to 8 ring atoms or comprises 4 to 10 ring atoms; most preferably, the heterocyclyl comprises 3 to 8 ring atoms; further preferably, the heterocyclyl is a 3- to 8-membered heterocyclyl group comprising 1-3 nitrogen atoms, which is optionally substituted with 1-2 oxygen atoms, sulfur atoms or an oxo group, including nitrogen-containing monocyclic heterocyclyl, nitrogen-containing spiro heterocyclyl or nitrogen-containing fused heterocyclyl.

Non-limiting examples of monocyclic heterocyclyl include azetidinyl, pyrrolidyl, imidazolidinyl, tetrahydrofuryl, tetrahydrothiophenyl, dihydroimidazolyl, dihydrofuryl, dihydropyrazolyl, dihydropyrrolyl, piperidyl, piperazinyl, morpholinyl, thiomorpholinyl, homopiperazinyl, azepinyl, 1,4-diazacycloheptyl, pyranyl, etc., preferably pyrrolidyl, morpholinyl, piperidyl, azepinyl, 1,4-diazacycloheptyl and piperazinyl. Polycyclic heterocyclyl includes spiro, fused and bridged heterocyclyl, wherein the spiro, fused and bridged heterocyclyl groups are optionally connected to other groups through a single bond, or further fused to other cycloalkyl, heterocyclyl, aryl and heteroaryl through any two or more atoms on the ring.

The term "spiroheterocyclyl" refers to a 5- to 20-membered polycyclic heterocyclic group with monocyclic rings sharing one atom (called a spiro atom), wherein one or more ring atoms are heteroatoms selected from nitrogen, oxygen, P(O)ₚₚ or S(O)ₘₘ (wherein pp and mm are integers of 0 to 2), and the remaining ring atoms are carbon. Spiroheterocyclyl may contain one or more double bonds, but no ring has a fully conjugated π electron system. Preferably, the spiro heterocyclyl is 6- to 14-membered, and more preferably 7- to 10-membered. According to the number of shared spiro atoms between the rings, the spiro heterocyclyl is divided into monospiroheterocyclyl, bispiroheterocyclyl or polyspiroheterocyclyl, preferably monospiroheterocyclyl and bispiroheterocyclyl. More preferably, it is a 3-membered/5-membered, 3-membered/6-membered, 4-membered/4-membered, 4-membered/5-membered, 4-membered/6-membered, 5-membered/5-membered or 5-membered/6-membered monospiro heterocyclyl. Non-limiting examples of spiro heterocyclyl include: 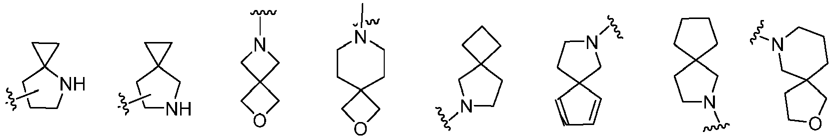 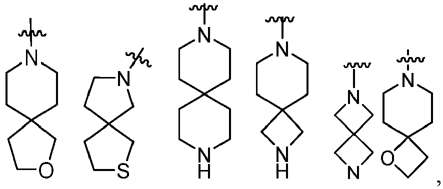 etc.

The term "fused heterocyclyl" refers to a 5- to 20-membered polycyclic heterocyclic group with each ring in the system sharing an adjacent pair of atoms with other rings in the system, and one or more rings may contain one or more double bonds, but no ring has a fully conjugated π electron system, wherein one or more ring atoms are heteroatoms selected from nitrogen, oxygen, P(O)ₚₚ or S(O)ₘₘ (wherein pp and mm are integers of 0 to 2), and the remaining ring atoms are carbon. Preferably, the fused heterocyclyl is 6- to 14-membered, and more preferably 7- to 10-membered. According to the number of constituent rings, it can be divided into bicyclic, tricyclic, tetracyclic or polycyclic fused heterocyclyl, preferably bicyclic or tricyclic, more preferably 5-membered/5-membered or 5-membered/6-membered bicyclic fused heterocyclyl. Non-limiting examples of fused heterocyclyl include: 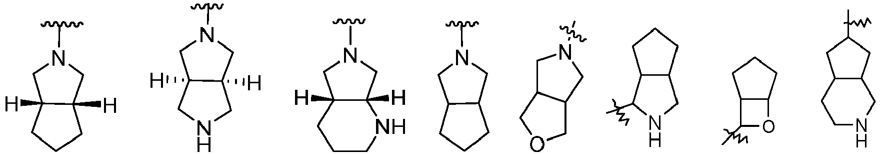 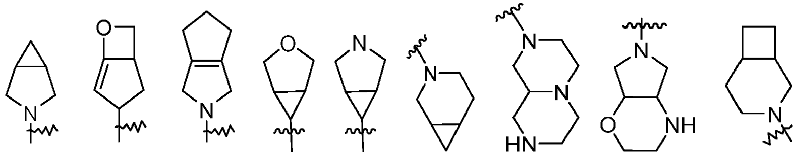 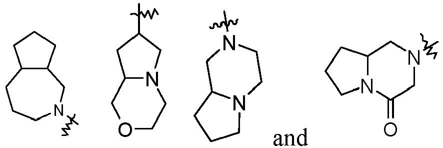 , etc.

The term "bridged heterocyclyl" refers to a 5- to 14-membered polycyclic heterocyclic group with any two rings sharing two atoms that are not directly connected. It may contain one or more double bonds, but no ring has a fully conjugated π electron system, wherein one or more ring atoms are heteroatoms selected from nitrogen, oxygen, P(O)ₚₚ or S(O)ₘₘ (wherein pp and mm are integers of 0 to 2), and the remaining ring atoms are carbon. Preferably, the bridged heterocyclyl is 6- to 14-membered, and more preferably 7- to 10-membered. According to the number of constituent rings, it can be divided into bicyclic, tricyclic, tetracyclic or polycyclic bridged heterocyclyl, preferably bicyclic, tricyclic or tetracyclic, and more preferably bicyclic or tricyclic. Non-limiting examples of bridged heterocyclyl include: 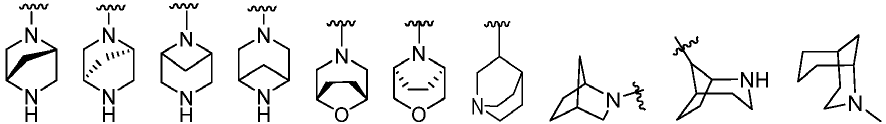 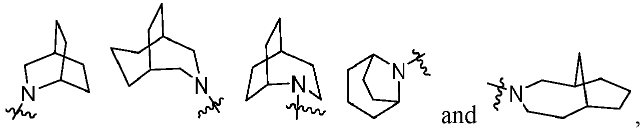 etc.

The heterocyclyl ring can be fused to an aryl, heteroaryl or cycloalkyl ring, wherein the ring connected to the parent structure is heterocyclyl, and the non-limiting examples thereof include: 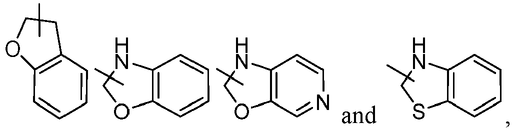 etc.

Heterocyclyl can be optionally substituted or unsubstituted. When the heterocyclyl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or a carboxylate group.

The term "aryl" refers to a 6- to 14-membered all-carbon monocyclic or fused polycyclic (i.e., rings sharing an adjacent pair of carbon atoms) group having a conjugated π electron system, preferably 6- to 12-membered aryl, such as phenyl and naphthyl, and more preferably phenyl. The aryl ring can be fused to heteroaryl, heterocyclyl or cycloalkyl ring, including benzo 5- to 10-membered heteroaryl, benzo 3- to 8-membered cycloalkyl and benzo 3- to 8-membered heteroalkyl, preferably benzo 5- to 6-membered heteroaryl, benzo 3- to 6-membered cycloalkyl and benzo 3-to 6-membered heteroalkyl, wherein the heterocyclyl is heterocyclyl containing 1-3 nitrogen atoms, oxygen atoms, or sulfur atoms; or further including a three-membered nitrogen-containing fused ring containing a benzene ring,
wherein the ring connected to the parent structure is aryl ring, and the non-limiting examples thereof include: 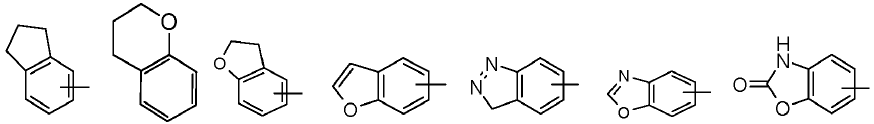 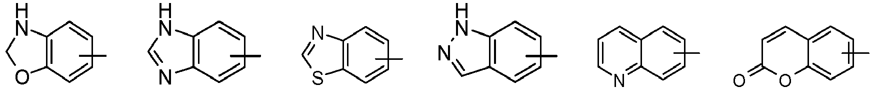 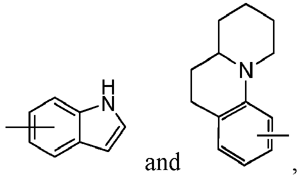 etc.

Aryl can be substituted or unsubstituted. When the aryl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

The term "heteroaryl" refers to a heteroaromatic system containing 1 to 4 heteroatoms and 5 to 14 ring atoms, wherein the heteroatoms are selected from oxygen, sulfur and nitrogen. The heteroaryl is preferably 5- to 12-membered, more preferably 8- to 11-membered, 5-membered or 6-membered, such as imidazolyl, furyl, thienyl, thiazolyl, pyrazolyl, oxazolyl, pyrrolyl, triazolyl, tetrazolyl, pyridyl, pyrimidinyl, thiadiazole, pyrazinyl, etc., preferably triazolyl, thienyl, imidazolyl, pyrazolyl, oxazolyl, pyrimidinyl or thiazolyl; more preferably, pyrazolyl, pyrrolyl and oxazolyl. The heteroaryl ring can be fused to an aryl, heterocyclyl or cycloalkyl ring, wherein the ring connected to the parent structure is heteroaryl ring, and the non-limiting examples thereof include: 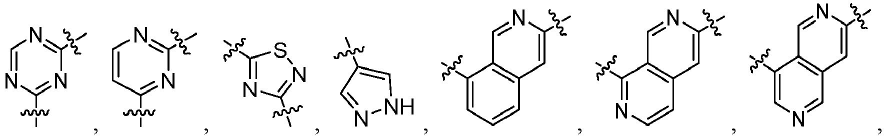 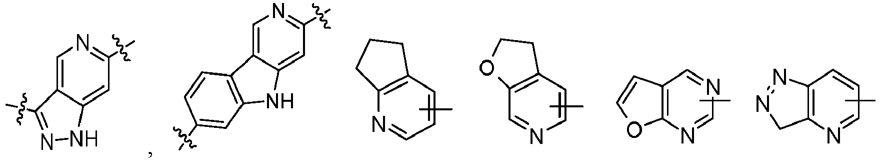 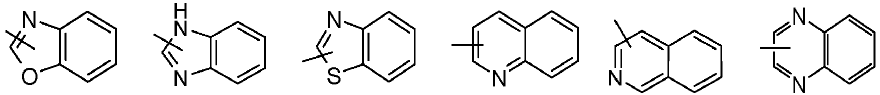 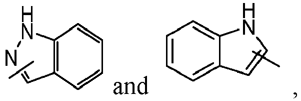 etc.

Heteroaryl can be optionally substituted or unsubstituted. When the heteroaryl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

The term "alkoxy" refers to -O- (alkyl) and -O- (unsubstituted cycloalkyl), where alkyl is as defined above, preferably alkyl containing 1 to 8 carbon atoms, more preferably alkyl containing 1 to 6 carbon atoms, and most preferably alkyl containing 1 to 3 carbon atoms. Non-limiting examples of alkoxy include: methoxy, ethoxy, propoxy, butoxy, cyclopropoxy, cyclobutoxy, cyclopentoxy, and cyclohexoxy. Alkoxy can be optionally substituted or unsubstituted. When the alkoxy is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

"Haloalkyl" refers to alkyl substituted with one or more halogen, wherein alkyl is defined as above.

"Haloalkoxy" refers to alkoxy substituted with one or more halogen, wherein alkoxy is defined as above.

"Hydroxyalkyl" refers to alkyl substituted with hydroxy, wherein alkyl is defined as above.

"Alkenyl" refers to a chain alkenyl group, also known as an alkene group, preferably alkyl containing 2 to 8 carbon atoms, more preferably alkyl containing 2 to 6 carbon atoms, and most preferably alkyl containing 2 to 3 carbon atoms, wherein the alkenyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

"Alkynyl" refers to CH=C-, preferably alkyl containing 2 to 8 carbon atoms, more preferably alkyl containing 2 to 6 carbon atoms, and most preferably alkyl containing 2 to 3 carbon atoms, wherein the alkynyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

The term "alkenylcarbonyl" refers to -C(O)-(alkenyl), wherein alkenyl is defined as above. Non-limiting examples of alkenylcarbonyl include: ethenylcarbonyl, propenylcarbonyl, and butenylcarbonyl. Alkenylcarbonyl can be optionally substituted or unsubstituted. When the alkenylcarbonyl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.
"Hydroxyl" refers to an -OH group.
"Halogen" refers to fluorine, chlorine, bromine or iodine.
"Amino" refers to -NH₂.
"Cyano" refers to -CN.
"Nitro" refers to -NO₂.
"Carbonyl" refers to -C(O)-.
"Carboxyl" refers to -C(O)OH.
"THF" refers to tetrahydrofuran.
"EtOAc" refers to ethyl acetate.
"MeOH" refers to methanol.
"DMF" refers to N,N-dimethylformamide.
"DIPEA" refers to diisopropylethylamine.
"TFA" refers to trifluoroacetic acid.
"MeCN" refers to acetonitrile.
"DMA" refers to N,N-dimethylacetamide.
"Et₂O" refers to diethyl ether.
"DCE" refers to 1,2-dichloroethane.
"DIPEA" refers to N,N-diisopropylethylamine.
"NBS" refers to N-bromosuccinimide.
"NIS" refers to N-iodosuccinimide.
"Cbz-Cl" refers to benzyl chloroformate.
"Pd₂(dba)₃" refers to tris(dibenzylideneacetone)dipalladium.
"Dppf' refers to 1,1'-bisdiphenylphosphine ferrocene.
"HATU" refers to 2-(7-azabenzotriazol-1-yl)-N,N,N',N'-tetramethyluronium hexafluorophosphate.
"KHMDS" refers to potassium hexamethyldisilamide.
"LiHMDS" lithium bistrimethylsilylamide.
"MeLi" refers to lithium methyl.
"n-BuLi" refers to n-butyl lithium.
"NaBH(OAc)₃" refers to sodium triacetoxyborohydride.

Different terms such as "X is selected from A, B, or C", "X is selected from A, B and C", "X is A, B or C", "X is A, B and C" all express the same meaning, i.e., X can be any one or more of A, B, and C.

The hydrogen atoms described in the present invention can be replaced with its isotope deuterium, and any hydrogen atom in the example compounds involved in the present invention can also be replaced with a deuterium atom.

"Optional" or "optionally" means that the event or circumstance subsequently described may but need not to occur, and the description includes the occasions where the events or circumstances occur or do not occur. For example, "heterocyclic group optionally substituted with alkyl" means the alkyl may but need not be present, and the description includes the case where the heterocyclic group is substituted with alkyl and the case where the heterocyclic group is not substituted with alkyl.

"Substituted" refers to one or more hydrogen atoms in the group, preferably at most 5, more preferably 1-3 hydrogen atoms each independently substituted with a corresponding number of substituents. It goes without saying, the substituents may be only in their possible chemical positions, a person skilled in the art can determine the possible or impossible substitutions (by experiment or theory) without paying too much effort. For example, the amino group having a free hydrogen or a hydroxyl group may be unstable when combined the carbon atoms having an unsaturated (e.g., olefinic) bond.

The "more" in "substituted with one or more of..." can mean 2, 3, 4, 5, 6 or more than 6.

In various parts of the present invention, linking substituents are described. When it is clear that a linking group is required for the structure, the markush variables listed for that group should be understood as referring to the linking group. For example, if a linking group is required for the structure and the markush group definition for that variable lists "alkyl" or "aryl," it should be understood that the "alkyl" or "aryl" respectively represents the attached alkylene group or arylene group.

"Pharmaceutically acceptable salts" refer to salts of the compounds of the present invention, which are safe and effective when used in mammals, and have appropriate biological activity.

### Detailed Description of Embodiments

The present invention will be further described below in conjunction with examples, but these examples are not meant to limit the scope of the present invention.

### Example

The structure of the compound of the present invention is determined by nuclear magnetic resonance (NMR) or/and liquid chromatography-mass spectrometry (LC-MS). NMR chemical shift (δ) is given in the unit of 10⁻⁶ (ppm). NMR is determined with Bruker AVANCE-400 nuclear magnetic resonance instrument; the solvents for determination are deuterated dimethyl sulfoxide (DMSO-*d₆*), deuterated methanol (CD₃OD) and deuterated chloroform (CDCl₃); and the internal standard is tetramethylsilane (TMS).

Agilent 1200 Infinity Series mass spectrometer is used for LC-MS. HPLC uses Agilent 1200DAD high-pressure liquid chromatograph (Sunfire C18 150 × 4.6 mm column) and Waters 2695-2996 high-pressure liquid chromatograph (Gimini C₁₈ 150 × 4.6 mm column).

Yantai Huanghai HSGF254 or Qingdao GF254 silica gel plate is used as a thin layer chromatography silica plate, and TLC is of the specification of 0.15 mm-0.20 mm, and the specification when separating and purifying a product by thin layer chromatography is 0.4 mm - 0.5 mm. For the column chromatography, Yantai Huanghai silica gel of 200-300 mesh silica gel is generally used as a carrier.

The starting materials in the examples of the present invention are known and can be purchased on the market, or can be synthesized using or according to methods known in the art.

Unless otherwise specified, all reactions of the present invention are carried out under continuous magnetic stirring in a dry nitrogen or argon atmosphere, the solvent is a dry solvent, and the reaction temperature unit is degrees Celsius.

### Intermediate 1 3-((methanesulfonyl)methyl)azetidine

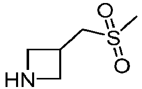

### Step 1 Synthesis of tert-butyl 3-((methylthio)methyl)azetidine-1-carboxylate

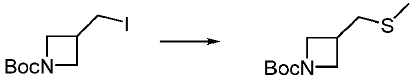

Tert-butyl 3-(iodomethyl)azetidine-1-carboxylate (2 g, 6.73 mmol) and sodium methyl mercaptide (970 mg, 13.50 mmol) were dissolved in ACN (15 mL) and H₂O (5 mL), and the mixture was warmed to 60°C and reacted for 12 hours. The reaction solution was cooled to room temperature, concentrated under reduced pressure, and then separated by column chromatography to obtain the target compound tert-butyl 3-((methylthio)methyl)azetidine-1-carboxylate (1.30 g, 88.9%).

MS m/z (ESI) : 162.0 [M+H-56]⁺.

### Step 2 Synthesis of tert-butyl 3-((methanesulfonyl)methyl)azetidine-1-carboxylate

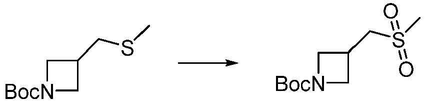

Tert-butyl 3-((methylthio)methyl)azetidine-1-carboxylate (1.30 g, 5.99 mmol) was dissolved in dichloromethane (15 mL), m-chloroperoxybenzoic acid (2 g, 12 mmol) was added and the mixture was stirred overnight. The reaction solution was washed with saturated sodium thiosulfate solution and saturated sodium chloride solution, the organic phase was dried over anhydrous sodium sulfate, concentrated under reduced pressure and separated by column chromatography to obtain the target compound tert-butyl 3-((methanesulfonyl)methyl)azetidine-1-carboxylate (1.30 g, 87.1%).

MS m/z (ESI) :194.0 [M+H-56]⁺.

### Step 3 Synthesis of 3-((methanesulfonyl)methyl)azetidine trifluoroacetate

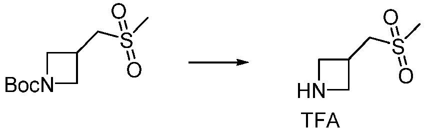

Tert-butyl 3-((methanesulfonyl)methyl)azetidine-1-carboxylate (1.30 g, 5.22 mmol) was dissolved in dichloromethane (15 mL), TFA (3 mL) was added, and the mixture was stirred for 4 hours. The reaction solution was concentrated under reduced pressure to obtain the crude target compound 3-((methanesulfonyl)methyl)azetidine trifluoroacetate (740 mg).

MS m/z (ESI) :150.0 [M+H]⁺.

### Intermediate 2 (2S,3R)-2-methyl-3-((methanesulfonyl)methyl)azetidine

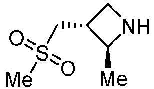

### Step 1 Synthesis of (2S,3R)-1-diphenylmethyl-2-methylazetidin-3-yl methanesulfonate

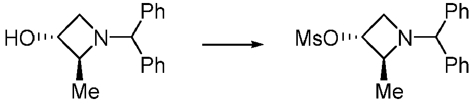

(2S,3R)-1-Diphenylmethyl-2-methylazetidin-3-ol (10 g, 39.50 mmol) was dissolved in dichloromethane (100 mL), triethylamine (4.80 g, 47.30 mmol) was added, the reaction solution was cooled to 0°C, methanesulfonyl chloride (5 g, 43.40 mmol) was slowly added dropwise, and the mixture was warmed to room temperature and allowed to react overnight. The reaction was quenched by adding water, the reaction solution was subjected to liquid separation with dichloromethane and water, the organic phase was dried over anhydrous sodium sulfate, and concentrated under reduced pressure to obtain the crude target compound (2S, 3R)-1-diphenylmethyl-2-methylazetidin-3-yl methanesulfonate (13 g).

MS m/z (ESI) :332.2 [M+H]⁺.

### Step 2 Synthesis of methyl (R)-2-((2S,3R)-1-diphenylmethyl-2-methylazetidin-3-yl)-2-(methanesulfonyl)acetate

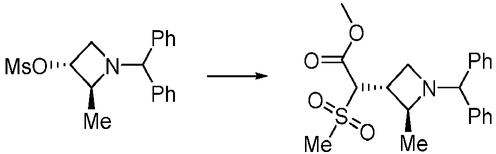

(2S,3R)-1-Diphenylmethyl-2-methylazetidin-3-ylmethanesulfonate (13 g, 39.20 mmol) and methyl 2-(methylsulfonyl)acetate (7.70 g, 50.60 mmol) were dissolved in DMF (100 mL), sodium hydride (2.20 g, 60% in mineral oil, 55.50 mmol) was added in batches, and the mixture was reacted at room temperature for 15 minutes, and then warmed to 80°C and reacted overnight. The reaction solution was cooled to room temperature and quenched with a saturated ammonium chloride solution, the reaction solution was subjected to liquid separation with ethyl acetate and water, and the organic phase was dried over anhydrous sodium sulfate, concentrated under reduced pressure and separated by column chromatography to obtain the target compound methyl (R)-2-((2S,3R)-1-diphenylmethyl-2-methyl azetidin-3-yl)-2-(methanesulfonyl)acetate (12 g, 79.0%).

MS m/z (ESI) :388.2 [M+H]⁺.

### Step 3 Synthesis of (2S,3R)-1-diphenylmethyl-2-methyl-3-((methanesulfonyl) methyl)azetidine

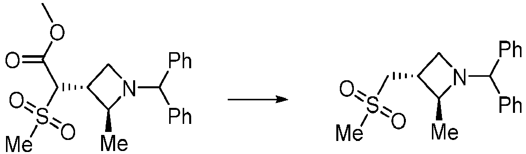

Methyl (R)-2-((2S,3R)-1-diphenylmethyl-2-methylazetidin-3-yl)-2-(methanesulfonyl)acetate (12 g, 30.97 mmol) was dissolved in DMA (120 mL), lithium chloride (10.50 g, 247.50 mmol) was added, and the mixture was warmed to 150°C and reacted for 2 hours. The mixture was cooled to room temperature, the reaction solution was subjected to liquid separation with ethyl acetate and water, the organic phase was dried over anhydrous sodium sulfate, concentrated under reduced pressure and separated by column chromatography to obtain the target compound (2S,3R)-1-diphenylmethyl-2-methyl-3-((methanesulfonyl)methyl)azetidine (9.50 g, 93.1%).

MS m/z (ESI) :330.0 [M+H]⁺.

### Step 4 Synthesis of (2S,3R)-2-methyl-3-((methanesulfonyl)methyl)azetidine

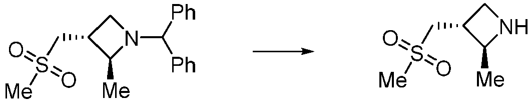

(2S,3R)-1-Diphenylmethyl-2-methyl-3-((methanesulfonyl)methyl)azetidine (9.50 g, 28.60 mmol) was dissolved in methanol (120 mL), TFA (5 mL) and palladium hydroxide (2.80 g) were added, the reaction system was evacuated, and filled with hydrogen, the operations were repeated three times, and the reaction solution was reacted overnight under a hydrogen atmosphere. The reaction solution was filtered, and the filtrate was concentrated under reduced pressure to obtain crude target compound (2S,3R)-2-methyl-3-((methanesulfonyl)methyl)azetidine (4.30 g).

MS m/z (ESI) :164.0 [M+H]⁺.

### Intermediate 3 (3S,4R)-1-(4-amino-1,3,5-triazin-2-yl)-3-fluoro-3-methylpiperidin-4-ol

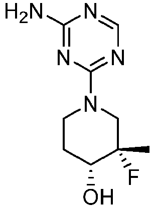

### Step 1 Synthesis of tert-butyl 5-methyl-4-((trimethylsilyl)oxo)-3,6-dihydropyridine-1(2H)-carboxylate

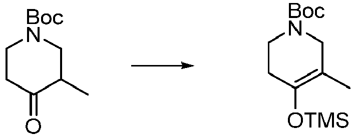

Tert-butyl 3-methyl-4-carbonylpiperidine-1-carboxylate (10 g, 46.90 mmol) was dissolved in toluene (100 mL), TEA (11.90 g, 117.30 mmol) was added, and the reaction solution was cooled to 0°C. TMSOTf (12.50 g, 56.30 mmol) was slowly added dropwise, and the mixture was reacted at 0°C for 6 hours. The reaction solution was subjected to liquid separation with ethyl acetate and water, and the organic phase was dried over anhydrous sodium sulfate, and concentrated under reduced pressure to obtain the crude target compound tert-butyl 5-methyl-4-((trimethylsilyl)oxo)-3,6-dihydropyridine-1(2H)-carboxylate (10.70 g).

### Step 2 Synthesis of tert-butyl 3-fluoro-3-methyl-4-carbonylpiperidine -1-carboxylate

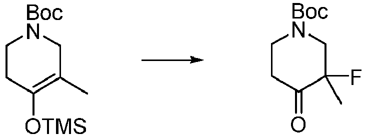

Tert-butyl 5-methyl-4-((trimethylsilyl)oxo)-3,6-dihydropyridine-1(2H)-carboxylate (10.70 g, 37.50 mmol) was dissolved in acetonitrile (100 mL), the reaction solution was cooled to 0°C, selectFluor (14.60 g, 41.30 mmol) was slowly added, and the mixture was reacted at 0°C for 2 hours. The reaction solution was subjected to liquid separation with ethyl acetate and water, and the organic phase was dried over anhydrous sodium sulfate, and concentrated under reduced pressure to obtain the crude target compound tert-butyl 3-fluoro-3-methyl-4-carbonylpiperidine -1-carboxylate (7.50 g).

### Step 3 Synthesis of tert-butyl 3-fluoro-4-hydroxy-3-methylpiperidine-1-carboxylate

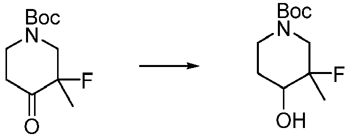

Tert-butyl 3-fluoro-3-methyl-4-carbonylpiperidine-1-carboxylate (7.50 g, 32.40 mmol) was dissolved in methanol (75 mL). The reaction solution was cooled to 0°C and NaBH₄ (1.50 g, 38.90 mmol) was slowly added, and the mixture was warmed to room temperature and reacted for 4 hours. The reaction solution was subjected to liquid separation with ethyl acetate and water, and the organic phase was dried over anhydrous sodium sulfate, concentrated under reduced pressure and separated by column chromatography to obtain the target compound tert-butyl 3-fluoro-4-hydroxy-3-methylpiperidine-1-carboxylate (6.80 g, 90.0%).

MS m/z (ESI) : 178.0 [M+H-56]⁺.

### Step 4 Synthesis of 3-fluoro-3-methylpiperidin-4-ol

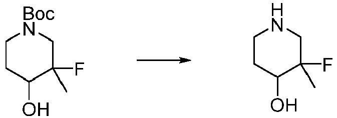

Tert-butyl 3-fluoro-4-hydroxy-3-methylpiperidine-1-carboxylate (6.80 g, 29.20 mmol) was dissolved in dioxane (70 mL), 4 M hydrogen chloride in dioxane (10 mL) was added, and the mixture was reacted for 4 hours. The reaction solution was concentrated under reduced pressure to obtain the crude target product 3-fluoro-3-methylpiperidin-4-ol (4.60 g).

MS m/z (ESI) :134.0 [M+H]⁺.

### Step 5 Synthesis of 1-(4-amino-1,3,5-triazin-2-yl)-3-fluoro-3-methylpiperidin-4-ol

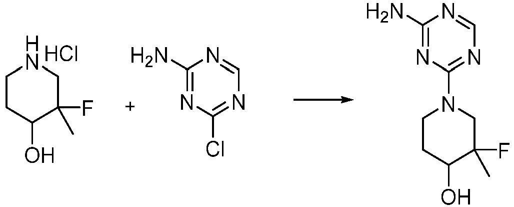

4-Chloro-1,3,5-triazin-2-amine (4 g, 30.80 mmol) and 3-fluoro-3-methylpiperidin-4-ol hydrochloride (5.70 g, 33.60 mmol) were dissolved in isopropanol (100 mL), triethylamine (9.30 g, 92.40 mmol) was added, and the mixture was warmed to 130°C and reacted under sealed conditions for 6 hours. The reaction solution was cooled to room temperature, and filtered, and the filtrate was concentrated under reduced pressure and separated by column chromatography to obtain the target compound 1-(4-amino-1,3,5-triazin-2-yl)-3-fluoro-3-methylpiperidin-4-ol (6 g, 85.7%).

MS m/z (ESI) :228.0 [M+H]⁺.

### Step 6 Preparation of (3S,4R)-1-(4-amino-1,3,5-triazin-2-yl)-3-fluoro-3-methylpiperidin-4-ol

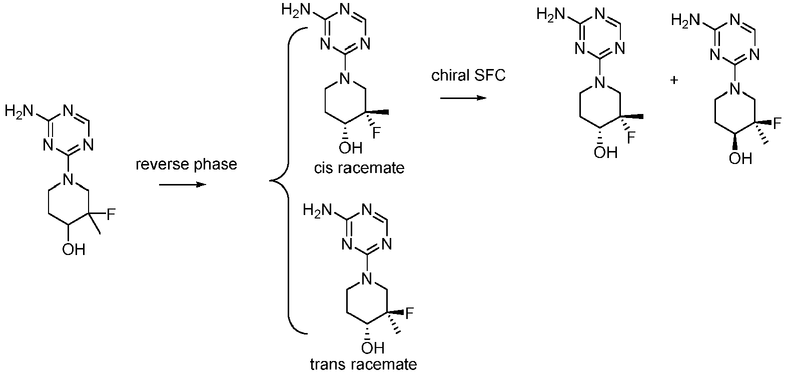

The purified product 1-(4-amino-1,3,5-triazin-2-yl)-3-fluoro-3-methylpiperidin-4-ol was purified through a reversed-phase column to obtain two components which were concentrated and freeze-dried to obtain cis racemate (3S,4R)-1-(4-amino-1,3,5-triazin-2-yl)-3-fluoro-3-methylpiperidin-4-ol (2.20 g) and trans racemate (3R,4R)-1-(4-amino-1,3,5-triazin-2-yl)-3-fluoro-3-methylpiperidin-4-ol (1.40 g), respectively.

The cis racemate (3S,4R)-1-(4-amino-1,3,5-triazin-2-yl)-3-fluoro-3-methylpiperidin-4-ol (2.20 g) obtained through reversed-phase purification was subjected to chiral resolution to give (3S,4R)-1-(4-amino-1,3,5-triazin-2-yl)-3-fluoro-3-methylpiperidin-4-ol (850 mg) and (3R,4S)-1-(4-amino-1,3,5-triazin-2-yl)-3-fluoro-3-methylpiperidin-4-ol (760 mg).

MS m/z (ESI) :228.0 [M+H]⁺.

### Intermediate 4 (2R,3S)-2-methyl-3-((methanesulfonyl)methyl)azetidine

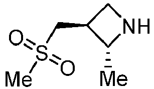

### Step 1 Synthesis of (2R,3S)-1-diphenylmethyl-2-methylazetidin-3-yl methanesulfonate

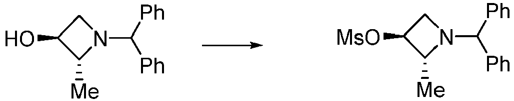

(2R,3S)-1-Diphenylmethyl-2-methylazetidin-3-ol (10 g, 39.50 mmol) was dissolved in dichloromethane (100 mL), triethylamine (4.80 g, 47.30 mmol) was added, the reaction solution was cooled to 0°C, methanesulfonyl chloride (5 g, 43.40 mmol) was slowly added dropwise, and the mixture was allowed to react at room temperature overnight. The reaction was quenched by adding water, the reaction solution was subjected to liquid separation with dichloromethane and water, the organic phase was dried over anhydrous sodium sulfate, and concentrated under reduced pressure to obtain the crude target compound (2R,3S)-1-diphenylmethyl-2-methylazetidin-3-yl methanesulfonate (12.80 g).

MS m/z (ESI) :332.2 [M+H]⁺.

### Step 2 Synthesis of methyl (S)-2-((2R,3S)-1-diphenylmethyl-2-methylazetidin-3-yl)-2-(methanesulfonyl)acetate

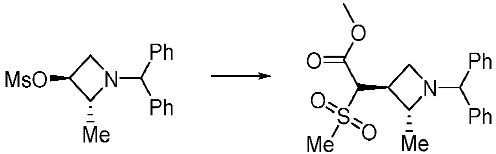

(2R,3S)-1-Diphenylmethyl-2-methylazetidin-3-ylmethanesulfonate (12.80 g, 38.62 mmol) and methyl 2-(methylsulfonyl)acetate (7.70 g, 50.60 mmol) were dissolved in DMF (100 mL), sodium hydride (2.20 g, 60% in mineral oil, 55.50 mmol) was added in batches, and the mixture was reacted at room temperature for 15 minutes, and then warmed to 80°C and reacted overnight. The reaction solution was cooled to room temperature and quenched with a saturated ammonium chloride solution, the reaction solution was subjected to liquid separation with ethyl acetate and water, and the organic phase was dried over anhydrous sodium sulfate, concentrated and separated by column chromatography to obtain the target compound methyl (S)-2-((2R,3S)-1-diphenylmethyl-2-methyl azetidin-3-yl)-2-(methanesulfonyl)acetate (11.60 g, 77.5%).

MS m/z (ESI) :388.2 [M+H]⁺.

### Step 3 Synthesis of (2R,3S)-1-diphenylmethyl-2-methyl-3-((methanesulfonyl)methyl)azetidine

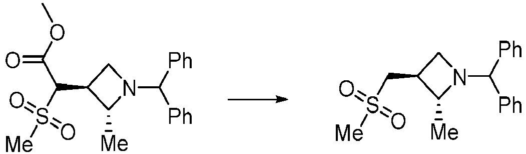

Methyl (S)-2-((2R,3 S)-1-diphenylmethyl-2-methylazetidin-3-yl)-2-(methanesulfonyl)acetate (11.60 g, 29.94 mmol) was dissolved in DMA (120 mL), lithium chloride (10.50 g, 247.50 mmol) was added, and the mixture was warmed to 150°C and reacted for 2 hours. The mixture was cooled to room temperature, the reaction solution was subjected to liquid separation with ethyl acetate and water, the organic phase was dried over anhydrous sodium sulfate, concentrated under reduced pressure and separated by column chromatography to obtain the target compound (2R,3S)-1-diphenylmethyl-2-methyl-3-((methanesulfonyl)methyl)azetidine (9.20 g, 93.3%).

MS m/z (ESI) :330.0 [M+H]⁺.

### Step 4 Synthesis of (2R,3 S)-2-methyl-3-((methanesulfonyl)methyl)azetidine

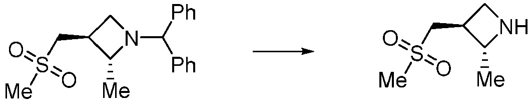

(2R,3 S)-1-Diphenylmethyl-2-methyl-3-((methanesulfonyl)methyl)azetidine (9.20 g, 27.92 mmol) was dissolved in methanol (120 mL), TFA (5 mL) and palladium hydroxide (2.80 g) were added, and the reaction system was evacuated and filled with hydrogen. The operations were repeated three times, and the reaction solution was reacted overnight under a hydrogen atmosphere. The reaction solution was filtered, and the filtrate was concentrated under reduced pressure to obtain the crude target compound (2R,3S)-2-methyl-3-((methanesulfonyl)methyl)azetidine (4 g).

MS m/z (ESI) :164.0 [M+H]⁺.

### Intermediate 5 8-bromo-3-chloro-6-fluoro-5-isopropylisoquinoline

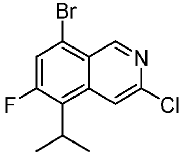

### Step 1 Synthesis of 3-chloro-6-fluoroisoquinoline

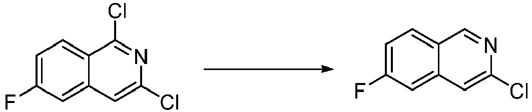

1,3-Dichloro-6-fluoroisoquinoline (7.50 g, 34.72 mmol) was dissolved in glacial acetic acid (40 mL) and hydriodic acid (20 mL, 45% aqueous), and the temperature was warmed to 100°C and the mixture was reacted for 4 hours. The reaction solution was cooled to room temperature, concentrated, diluted with dichloromethane (100 mL), and washed with saturated aqueous sodium carbonate solution, the organic phase was separated, dried over anhydrous sodium sulfate, and filtered, and the organic solvent was concentrated under reduced pressure and separated by column chromatography to obtain the target compound 3-chloro-6-fluoroisoquinoline (4.90 g, 77.8%).

MS m/z (ESI) :182.0 [M+H]⁺.

### Step 2 Synthesis of 5-bromo-3-chloro-6-fluoroisoquinoline

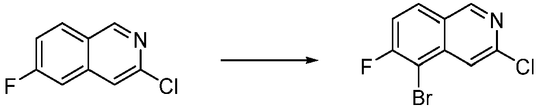

3-Chloro-6-fluoroisoquinoline (3.20 g, 17.62 mmol) was dissolved in concentrated sulfuric acid (20 mL), NBS (3.45 g, 19.38 mmol) was added, and the mixture was stirred at room temperature overnight. The reaction solution was slowly added to ice water, the reaction solution was subjected to liquid separation with ethyl acetate and water, and the organic phase was separated and dried over anhydrous sodium sulfate. After filtration, the filtrate was concentrated under reduced pressure and separated by column chromatography to obtain the target compound 5-bromo-3-chloro-6-fluoroisoquinoline (4.0 g, 87.1%).

MS m/z (ESI) :260.0 [M+H]⁺.

### Step 3 Synthesis of 3-chloro-6-fluoro-5-(prop-1-en-2-yl)isoquinoline

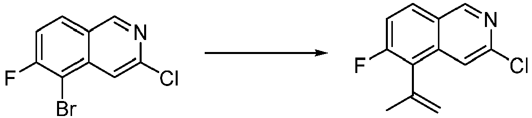

5-Bromo-3-chloro-6-fluoroisoquinoline (4.0 g, 15.36 mmol) and 4,4,5,5-tetramethyl-2-(prop-1-en-2-yl)-1,3,2-dioxaborolane (2.71 g, 16.13 mmol) was dissolved in 1,4-dioxane (20 mL) and water (3 mL), and [1,1'-bis(diphenylphosphine)ferrocene]palladium dichloride (562 mg, 0.77 mmol) and cesium carbonate (10.01 g, 30.71 mmol) were added, and the mixture was warmed to 80°C and reacted for 2 hours. The reaction solution was cooled to room temperature, concentrated under reduced pressure, and then separated by column chromatography to obtain the target compound 3-chloro-6-fluoro-5-(prop-1-en-2-yl)isoquinoline (3.0 g, 88.1%).

MS m/z (ESI) :222.0 [M+H]⁺. Step 4 Synthesis of 3-chloro-6-fluoro-5-i sopropyli soquinoline

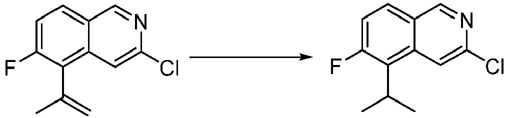

3-Chloro-6-fluoro-5-(prop-1-en-2-yl)isoquinoline (3.0 g, 13.53 mmol) was dissolved in ethyl acetate (20 mL), platinum dioxide (615 mg, 2.71 mmol) was added, and the mixture was stirred at room temperature for 4 hours under a hydrogen atmosphere. The reaction solution was filtered, and the filtrate was concentrated under reduced pressure and separated by column chromatography to obtain the target compound 3-chloro-6-fluoro-5-isopropylisoquinoline (2.10 g, 69.4%).

MS m/z (ESI) :224.1 [M+H]⁺.

### Step 5 Synthesis of 8-bromo-3-chloro-6-fluoro-5-isopropylisoquinoline

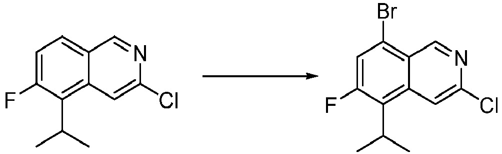

In an ice bath, to a solution of 3-chloro-6-fluoro-5-isopropylisoquinoline (800 mg, 3.58 mmol) in concentrated sulfuric acid (5 mL) was slowly added dibromohydantoin (1.12 g, 3.93 mmol) in batches, and the mixture was stirred in the ice bath for additional 0.5 hours. The reaction solution was carefully added to ice water and the reaction solution was subjected to liquid separation with dichloromethane and water. The organic phase was dried over anhydrous sodium sulfate. After filtration, the filtrate was concentrated under reduced pressure and separated by column chromatography to obtain the target compound 8-bromo-3-chloro-6-fluoro-5-isopropylisoquinoline (320 mg, 29.6%).

MS m/z (ESI) :302.0 [M+H]⁺.

### Reference example 1

### (3S,4R)-3-fluoro-1-(4-((5-isopropyl-8-(3-((methanesulfonyl)methyl)azetidin-1-yl)isoquinolin-3-yl)amino)-1,3,5-triazin-2-yl)-3-methylpiperidin-4-ol

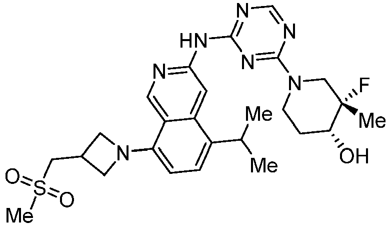

### Step 1 Synthesis of 8-bromo-3-chloroisoquinolin-5-yltrifluoromethanesulfonic acid

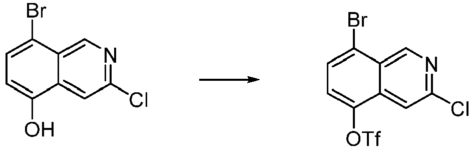

8-Bromo-3-chloroisoquinolin-5-ol (5 g, 19.50 mmol) was dissolved in dichloromethane (50 mL), TEA (7.90 g, 78 mmol) was added, and the reaction solution was cooled to - 60°C, (TfO)₂O (16.50 g, 58.50 mmol) was slowly added dropwise, and the mixture was warmed to room temperature and reacted for 4 hours. The reaction solution was concentrated under reduced pressure and separated by column chromatography to obtain the target compound 8-bromo-3-chloroisoquinolin-5-yltrifluoromethanesulfonic acid (6.50 g, 85.3%).

MS m/z (ESI) :390.0 [M+H]⁺.

### Step 2 Synthesis of 8-bromo-3-chloro-5-(prop-1-en-2-yl)isoquinoline

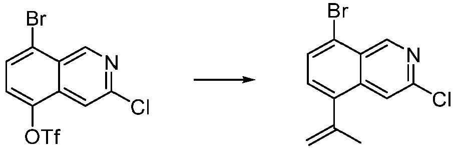

8-Bromo-3-chloroisoquinolin-5-yltrifluoromethanesulfonic acid (6.50 g, 16.64 mmol) was dissolved in dioxane (60 mL) and H₂O (6 mL), isopropenylboronic acid pinacol ester (4.20 g, 25 mmol), potassium carbonate (4.60 g, 33.40 mmol), and PdCl₂(dppf)

(610 mg, 0.84 mmol) were added, and the mixture was warmed to 100°C and reacted for 4 hours. The reaction solution was cooled to room temperature, and concentrated, the reaction solution was subjected to liquid separation with dichloromethane and water, and the organic phase was dried over anhydrous sodium sulfate, concentrated under reduced pressure and separated by column chromatography to obtain the target compound 8-bromo-3-chloro-5-(prop-1-en-2-yl)isoquinoline (2.90 g, 61.7%).

MS m/z (ESI) :282.0 [M+H]⁺.

### Step 3 Synthesis of 8-bromo-3-chloro-5-isopropylisoquinoline

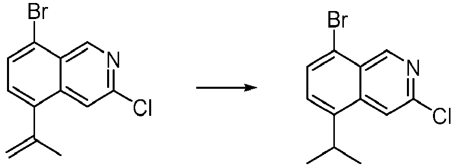

8-Bromo-3-chloro-5-(prop-1-en-2-yl)isoquinoline (2.90 g, 10.26 mmol) was dissolved in ethyl acetate (100 mL), PtO₂ (700 mg, 3.10 mmol) was added, the reaction system was evacuated, and filled with hydrogen, the operations were repeated 3 times, and the mixture was stirred under hydrogen atmosphere for 12 hours. The reaction solution was filtered, and the filtrate was concentrated under reduced pressure and separated by column chromatography to obtain the target compound 8-bromo-3-chloro-5-isopropylisoquinoline (2.70 g, 92.5%).

MS m/z (ESI) :284.0 [M+H]⁺.

### Step 4 Synthesis of 3-chloro-5-isopropyl-8-(3-((methanesulfonyl)methyl) azetidin-1-yl)isoquinoline

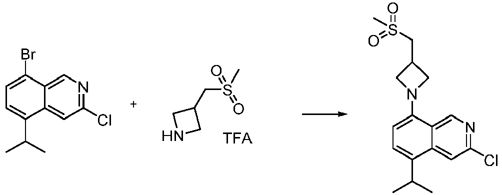

8-Bromo-3-chloro-5-isopropylisoquinoline (500 mg, 1.76 mmol) and 3-((methanesulfonyl)methyl)azetidine trifluoroacetate (435 mg, 1.77 mmol) were dissolved in dioxane (10 mL), cesium carbonate (1.15 g, 3.54 mmol) and Xantphos Pd G4 (164 mg, 0.17 mmol) were added, and the mixture was warmed to 100°C and stirred for 12 hours. The reaction solution was cooled to room temperature, and concentrated under reduced pressure, the reaction solution was subjected to liquid separation with dichloromethane and water, and the organic phase was dried over anhydrous sodium sulfate, concentrated under reduced pressure and separated by column chromatography to obtain the target compound 3-chloro-5-isopropyl-8-(3-((methanesulfonyl)methyl)azetidin-1-yl)isoquinoline (286 mg, 46.0%).

MS m/z (ESI) :353.0 [M+H]⁺.

### Step 5 Synthesis of (3S,4R)-3-fluoro-1-(4-((5-isopropyl-8-(3-((methanesulfonyl)methyl)azetidin-1-yl)isoquinolin-3-yl)amino)-1,3,5-triazin-2-yl)-3-methylpiperidin-4-ol

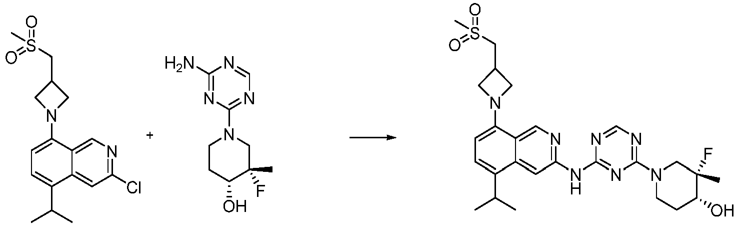

3-Chloro-5-isopropyl-8-(3-((methanesulfonyl)methyl)azetidin-1-yl)isoquinoline (100 mg, 0.28 mmol) and (3S,4R)-1-(4-amino-1,3,5-triazin-2-yl)-3-fluoro-3-methylpiperidin-4-ol (64 mg, 0.28 mmol) were dissolved in dioxane (5 mL), cesium carbonate (274 mg, 0.84 mmol) and BrettPhos Pd G3 (24 mg, 27 µmol) were added, and the mixture was warmed to 100°C and stirred for 12 hours. The reaction solution was cooled to room temperature, and concentrated under reduced pressure, the reaction solution was subjected to liquid separation with dichloromethane and water, and the organic phase was dried over anhydrous sodium sulfate, concentrated under reduced pressure and separated by column chromatography to obtain the target compound (3S,4R)-3-fluoro-1-(4-((5-isopropyl-8-(3-((methanesulfonyl)methyl) azetidin-1-yl)isoquinolin-3-yl)amino)-1,3,5-triazin-2-yl)-3-methyl piperidin-4-ol (23 mg, 15.1%).

¹H NMR (400 MHz, DMSO-*d*₆) *δ* 9.93 (s , 1H), 9.08 (s, 1H), 8.33 (s, 1H), 7.44 (d, *J* = 8.0 Hz, 1H), 6.45 (d, *J* = 8.1 Hz, 1H), 5.47 (s, 1H), 4.40 (t, *J* = 7.7 Hz, 2H), 4.25 (s, 1H), 3.97 (t, *J* = 6.9 Hz, 2H), 3.76 (s, 2H), 3.69 (s, 1H), 3.59 (d, *J* = 7.4 Hz, 3H), 3.01 (d, *J=* 4.8 Hz, 3H), 1.35 (s, 2H), 1.33-1.27 (m, 12H);

MS m/z (ESI) :544.2 [M+H]⁺.

### Example 1 N-(2-(1-(cyclopropylsulfonyl)-1H-pyrazol-4-yl)pyrimidin-4-yl)-5-isopropyl-8-(3-((methanesulfonyl)methyl)azetidin-1-yl)isoquinolin-3-amine

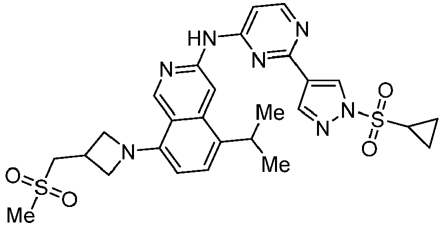

N-(2-(1-(cyclopropylsulfonyl)-1H-pyrazol-4-yl)pyrimidin-4-yl)-5-isopropyl-8-(3-((methanesulfonyl)methyl)azetidin-1-yl)isoquinolin-3-amine was prepared with reference to example 1
or synthesized according to the following steps:

### Step 1 Synthesis of 1-(cyclopropylsulfonyl)-4-(4,4,5,5-tetramethyl-1,3,2-dioxaborolan-2-yl)-1H-pyrazole

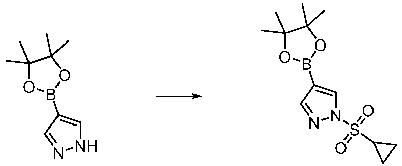

4-(4,4,5,5-Tetramethyl-1,3,2-dioxaborolan-2-yl)-1H-pyrazole (1.02 g, 5.26 mmol) was dissolved in DMF (15 mL), the mixture was cooled to 0°C, NaH (60%, 0.32 g, 8 mmol) and cyclopropanesulfonyl chloride (0.8 g, 5.78 mmol) were added, and the mixture was reacted at room temperature for 5 hours. The reaction solution was subjected to liquid separation with ethyl acetate and saturated sodium chloride solution, the organic phase was dried with anhydrous sodium sulfate, and the organic solvent was concentrated under reduced pressure and separated by column chromatography to obtain the target compound 1-(cyclopropylsulfonyl)-4-(4,4,5,5-tetramethyl-1,3,2-dioxaborolan-2-yl)-1H-pyrazole (787.4 mg, 50.2%).

MS m/z (ESI) :299.1 [M+H]⁺.

### Step 2 Synthesis of 2-(1-(cyclopropylsulfonyl)-1H-pyrazol-4-yl)pyrimidin-4-amine

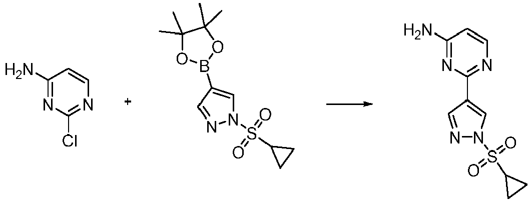

2-Chloropyrimidin-4-amine (323 mg, 2.49 mmol), 1-(cyclopropylsulfonyl)-4-(4,4,5,5-tetramethyl-1,3,2-dioxaborolan-2-yl)-1H-pyrazole (787.4 mg, 2.64 mmol) and potassium carbonate (691 mg, 5 mmol) were mixed with dioxane (5 mL) and water (1 mL), [1,1'-bis(diphenylphosphino)ferrocene]palladium dichloride (247.4 mg, 0.3 mmol) was added, and the mixture was warmed to 100°C and stirred for 12 hours. The reaction solution was cooled to room temperature, and concentrated under reduced pressure, the reaction solution was subjected to liquid separation with dichloromethane and water, the organic phase was dried over anhydrous sodium sulfate, and the organic solution was concentrated under reduced pressure and separated by column chromatography to obtain the target compound 2-(1-(cyclopropylsulfonyl)-1H-pyrazol-4-yl)pyrimidin-4-amine (500 mg, 75.7%).

MS m/z (ESI) :266.1 [M+H]⁺.

### Step 3 Synthesis of N-(2-(1-(cyclopropylsulfonyl)-1H-pyrazol-4-yl)pyrimidin-4-yl)-5-isopropyl-8-(3-((methanesulfonyl)methyl)azetidin-1-yl)isoquinolin-3-amine

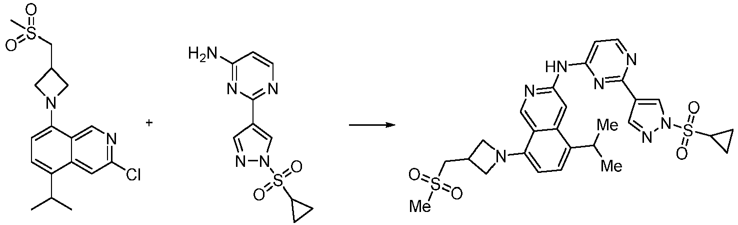

3-Chloro-5-isopropyl-8-(3-((methanesulfonyl)methyl)azetidin-1-yl)isoquinoline (100 mg, 0.28 mmol) and 2-(1-(cyclopropylsulfonyl)-1H-pyrazol-4-yl)pyrimidin-4-amine (74.3 mg, 0.28 mmol) were dissolved in dioxane (5 mL), cesium carbonate (274 mg, 0.84 mmol) and BrettPhos Pd G3 (24 mg, 27 µmol) were added, and the mixture was warmed to 105°C and stirred for 12 hours. The reaction solution was cooled to room temperature, and concentrated under reduced pressure, the reaction solution was subjected to liquid separation with dichloromethane and water, and the organic phase was dried over anhydrous sodium sulfate, concentrated under reduced pressure and separated by column chromatography to obtain the target compound N-(2-(1-(cyclopropylsulfonyl)-1H-pyrazol-4-yl)pyrimidin-4-yl)-5-isopropyl-8-(3-((methanesulfonyl)methyl)azetidin-1-yl)isoquinolin-3-amine (31.2 mg, 19.2%).

¹H NMR (400 MHz, DMSO-*d*₆) *δ* 10.37 (s, 1H), 9.10 (s, 1H), 8.80-8.74 (m, 1H), 8.69 (s, 1H), 8.51 (s, 1H), 8.43 (d, *J=* 5.9 Hz, 1H), 7.45 (d, *J=* 8.0 Hz, 1H), 7.29-7.20 (m, 1H), 6.45 (d, *J=* 8.0 Hz, 1H), 4.41 (t, *J=* 7.6 Hz, 2H), 3.98 (t, *J=* 6.9 Hz, 2H), 3.60 (d, *J* = 7.4 Hz, 3H), 3.30-3.25 (m, 2H), 3.02 (s, 3H), 1.39-1.34 (m, 8H), 1.29-1.26 (m, 2H);

MS m/z (ESI) :582.2 [M+H]⁺.

### Example 2

### N-(2-(1-(cyclopropylsulfonyl)-1H-pyrazol-4-yl)pyrimidin-4-yl)-5-isopropyl-8-((2S,3R)-2-methyl-3-((methanesulfonyl)methyl)azetidin-1-yl)isoquinolin-3-amine

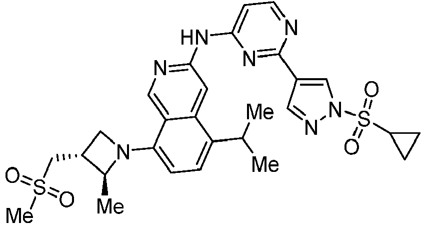

N-(2-(1-(cyclopropylsulfonyl)-1H-pyrazol-4-yl)pyrimidin-4-yl)-5-isopropyl-8-((2S,3R)-2-methyl-3-((methanesulfonyl)methyl)azetidin-1-yl)isoquinolin-3-amine was prepared with reference to reference example 1 or example 1.

MS m/z (ESI) :596.2 [M+H]⁺.

### Example 3

Preparation of N-(2-(1-(cyclopropylsulfonyl)-1H-pyrazol-4-yl)pyrimidin-4-yl)-5-isopropyl-8-(3-((methanesulfonyl)methyl)azetidin-1-yl)-2,7-diazanaphthalen-3-amine

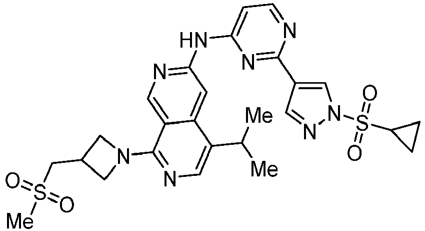

N-(2-(1-(cyclopropylsulfonyl)-1H-pyrazol-4-yl)pyrimidin-4-yl)-5-isopropyl-8-(3-((methanesulfonyl)methyl)azetidin-1-yl)-2,7-diazanaphthalen-3-amine was prepared with reference to reference example 1 or example 1.

¹H NMR (400 MHz, DMSO-*d*₆) *δ* 10.56 (s, 1H), 9.08 (s, 1H), 8.70 (s, 1H), 8.69-8.63 (m, 1H), 8.52-8.47 (m, 2H), 8.01 (s, 1H), 4.62-4.55 (m, 2H), 4.27-4.21 (m 2H), 3.60 (d, *J* = 7.5 Hz, 2H), 3.44-3.39 (m, 1H), 3.31-3.27 (m, 2H), 3.01 (s, 3H), 1.41-1.34 (m, 8H), 1.30-1.25 (m, 2H);

MS m/z (ESI): 583.1 [M+H]⁺.

### Example 4

### Preparation of N-(2-(1-(cyclopropylsulfonyl)-1H-pyrazol-4-yl)pyrimidin-4-yl)-5-isopropyl-8-((2S,3R)-2-methyl-3-((methanesulfonyl)methyl)azetidin-1-yl)-2,7-diazanaphthalen-3-amine

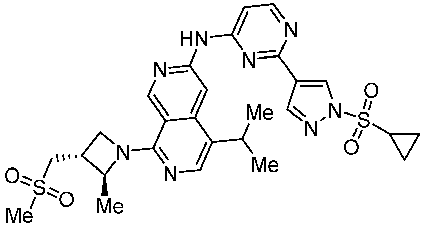

N-(2-(1-(cyclopropylsulfonyl)-1H-pyrazol-4-yl)pyrimidin-4-yl)-5-isopropyl-8-((2S,3R)-2-methyl-3-((methanesulfonyl)methyl)azetidin-1-yl)-2,7-diazanaphthalen-3-amine was prepared with reference to reference example 1 or example 1.

MS m/z (ESI): 597.1 [M+H]⁺.

### Example 5

### Preparation of N-(2-(1-(cyclopropylsulfonyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

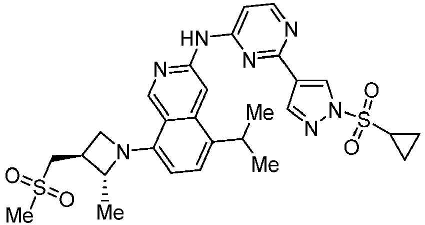

N-(2-(1-(cyclopropylsulfonyl)-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine was prepared with reference to reference example 1 or example 1.

MS m/z (ESI): 596.1 [M+H]⁺.

### Example 6

### Preparation of N-(2-(1-(cyclopropylsulfonyl)-1H-pyrazol-4-yl)pyrimidin-4-yl)-5-isopropyl-8-((2R,3 S)-2-methyl-3-((methanesulfonyl)methyl)azetidin-1-yl)-2,7-diazanaphthalen-3-amine

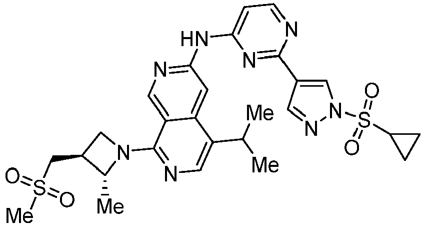

N-(2-(1-(cyclopropylsulfonyl)-1H-pyrazol-4-yl)pyrimidin-4-yl)-5-isopropyl-8-((2R,3 S)-2-methyl-3-((methanesulfonyl)methyl)azetidin-1-yl)-2,7-diazanaphthalen-3-amine was prepared with reference to reference example 1 or example 1.

MS m/z (ESI): 597.1 [M+H]⁺.

### Example 7

### Preparation of N-(2-(1-(cyclopropylsulfonyl)-1H-pyrazol-4-yl)pyrimidin-4-yl)-5-isopropyl-8-(3-((methanesulfonyl)methyl)azetidin-1-yl)-2,6-diazanaphthalen-3-amine

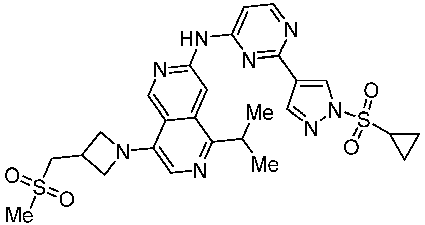

N-(2-(1-(cyclopropylsulfonyl)-1H-pyrazol-4-yl)pyrimidin-4-yl)-5-isopropyl-8-(3-((methanesulfonyl)methyl)azetidin-1-yl)-2,6-diazanaphthalen-3-amine was prepared with reference to reference example 1 or example 1.

MS m/z (ESI): 583.1 [M+H]⁺.

### Example 8

### Preparation of N-(2-(1-(cyclopropylsulfonyl)-1H-pyrazol-4-yl)pyrimidin-4-yl)-5-isopropyl-8-((2R,3 S)-2-methyl-3-((methanesulfonyl)methyl)azetidin-1-yl)-2,6-diazanaphthalen-3-amine

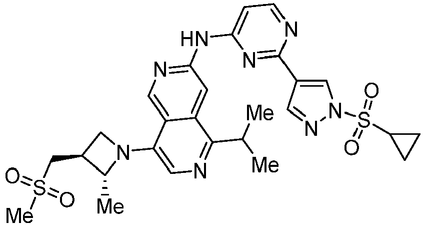

N-(2-(1-(cyclopropylsulfonyl)-1H-pyrazol-4-yl)pyrimidin-4-yl)-5-isopropyl-8-((2R,3 S)-2-methyl-3-((methanesulfonyl)methyl)azetidin-1-yl)-2,6-diazanaphthalen-3 -amine was prepared with reference to reference example 1 or example 1.

MS m/z (ESI): 597.1 [M+H]⁺.

### Example 9

### Preparation of cyclopropyl (4-(4-((5-isopropyl-8-((2R,3 S)-2-methyl-3-((methanesulfonyl)methyl)azetidin-1-yl)isoquinolin-3-yl)amino)pyrimidin-2-yl)-1H-pyrazol-1-yl)methanone

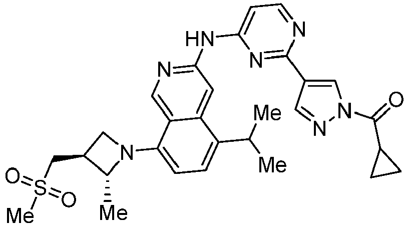

Cyclopropyl (4-(4-((5-isopropyl-8-((2R,3 S)-2-methyl-3-((methanesulfonyl) methyl)azetidin-1-yl)isoquinolin-3-yl)amino)pyrimidin-2-yl)-1H-pyrazol-1-yl)methanone was prepared with reference to reference example 1.

MS m/z (ESI): 560.1 [M+H]⁺.

### Example 10

### Preparation of N-(2-(3-chloro-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine

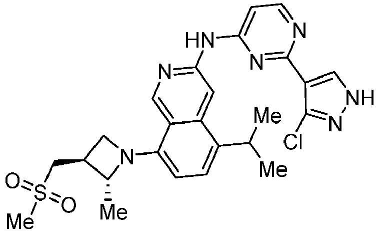

### Step 1 Synthesis of 4-bromo-3-chloro-1-((2-(trimethylsilyl)ethoxy)methyl)-1H-pyrazole

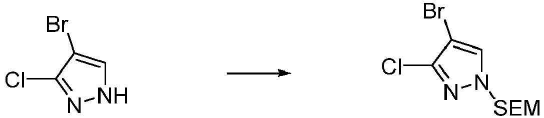

4-Bromo-3-chloro-1H-pyrazole (1.09 g, 6 mmol) and potassium carbonate (2.49 g, 18 mmol) were mixed in acetonitrile (20 mL), 2-(trimethylsilyl)ethoxymethyl chloride (1.50 g, 9 mmol) was added dropwise, and the mixture was reacted at room temperature for 3 hours. The reaction solution was diluted with dichloromethane and washed with saturated sodium chloride solution. The organic phase was dried over anhydrous sodium sulfate, filtered, and concentrated under reduced pressure to remove the solvent, and the residue was separated by silica gel column chromatography to obtain the title compound 4-bromo-3-chloro-1-((2-(trimethylsilyl)ethoxy)methyl)-1H-pyrazole (1.59 g, 85.0%).

MS m/z (ESI) :311.0 [M+H]⁺.

### Step 2 Synthesis of 3-chloro-4-(4,4,5,5-tetramethyl-1,3,2-dioxaborolan-2-yl)-1-((2-(trimethylsilyl)ethoxy)methyl)-1H-pyrazole

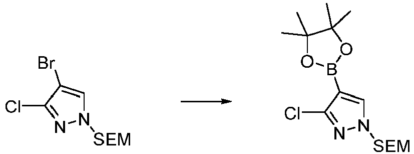

4-Bromo-3-chloro-1-((2-(trimethylsilyl)ethoxy)methyl)-1H-pyrazole (1.59 g, 5.1 mmol) and 4,4,4',4',5,5,5',5'-octamethyl-2,2'-di(1,3,2-dioxaborolane) (2.59 g, 10.2 mmol) were dissolved in dioxane (30 mL), potassium phosphate (3.18 g, 15 mmol) and [1,1'-bis(diphenylphosphino)ferrocene]palladium dichloride (412.4 mg, 0.5 mmol) were added, and the mixture was warmed to 95°C and stirred for 12 hours. The mixture was cooled to room temperature, and concentrated under reduced pressure to remove the solvent, the reaction solution was subjected to liquid separation with dichloromethane and water, the organic phase was dried over anhydrous sodium sulfate, filtered, and concentrated under reduced pressure, and the residue was separated by silica gel column chromatography to obtain the title compound 3-chloro-4-(4,4,5,5-tetramethyl-1,3,2-dioxaborolan-2-yl)-1-((2-(trimethylsilyl)ethoxy)methyl)-1H-pyrazole (1.22 g, 66.7%).

MS m/z (ESI) :359.2 [M+H]⁺.

### Step 3 Synthesis of 2-(3-chloro-1-((2-(trimethylsilyl)ethoxy)methyl)-1H-pyrazol-4-yl)pyrimidin-4-amine

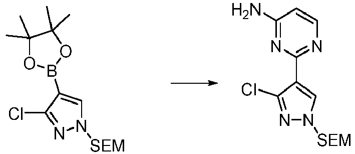

2-Chloropyrimidin-4-amine (388.6 mg, 3 mmol), 3-chloro-4-(4,4,5,5-tetramethyl-1,3,2-dioxaborolan-2-yl)-1-((2-(trimethylsilyl)ethoxy)methyl)-1H-pyrazole (1.22 g, 3.4 mmol) and potassium carbonate (1.38 g, 10 mmol) were added to a mixed solvent of dioxane (10 mL) and water (2 mL), [1,1'-bis(diphenylphosphino)ferrocene]palladium dichloride (247.4 mg, 0.3 mmol) was added and the reaction solution was warmed to 100°C and stirred for 12 hours. The reaction solution was cooled to room temperature, and concentrated under reduced pressure to remove the solvent, and the residue was separated by silica gel column chromatography to obtain the title compound 2-(3-chloro-1-((2-(trimethylsilyl)ethoxy)methyl)-1H-pyrazol-4-yl)pyrimidin-4-amine (490 mg, 50.1%).

MS m/z (ESI) :326.1 [M+H]⁺.

### Step 4 Synthesis of N-(2-(3-chloro-1-((2-(trimethylsilyl)ethoxy)methyl)-1H-pyrazol-4-yl)pyrimidin-4-yl)-5-isopropyl-8-((2R,3S)-2-methyl-3-((methanesulfonyl) methyl)azetidin-1-yl)isoquinolin-3-amine

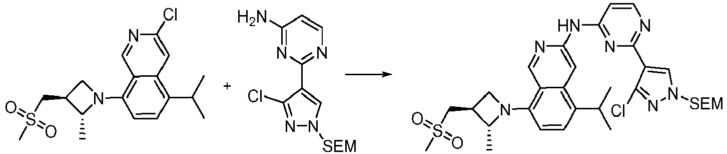

3-Chloro-5-isopropyl-8-((2R,3S)-2-methyl-3-((methanesulfonyl)methyl) azetidin-1-yl)isoquinoline (100 mg, 0.27 mmol) and 2-(3-chloro-1-((2-(trimethylsilyl)ethoxy)methyl)-1H-pyrazol-4-yl)pyrimidin-4-amine (88 mg, 0.27 mmol) were dissolved in dioxane (3 mL), cesium carbonate (274 mg, 0.84 mmol) and BrettPhos Pd G3 (24 mg, 27 µmol) were added, and the mixture was warmed to 105°C and stirred for 12 hours. The reaction solution was cooled to room temperature, and concentrated under reduced pressure, the reaction solution was subjected to liquid separation with dichloromethane and water, the organic phase was dried over anhydrous sodium sulfate, the organic solvent was concentrated under reduced pressure and separated by column chromatography to obtain the target compound N-(2-(3-chloro-1-((2-(trimethylsilyl)ethoxy)methyl)-1H-pyrazol-4-yl)pyrimidin-4-yl)-5-isopropyl-8-((2R,3 S)-2-methyl-3-((methanesulfonyl)methyl) azetidin-1-yl)isoquinolin-3-amine (81 mg, 45.7%).

MS m/z (ESI) :656.3 [M+H]⁺.

### Step 5 Synthesis of N-(2-(3-chloro-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

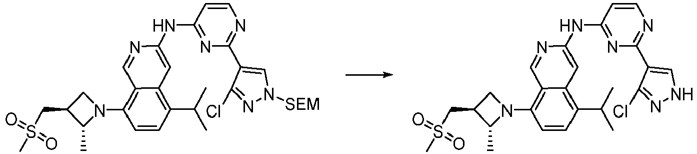

N-(2-(3-chloro-1-((2-(trimethylsilyl)ethoxy)methyl)-1H-pyrazol-4-yl)pyrimidin-4-yl)-5-isopropyl-8-((2R,3S)-2-methyl-3-((methanesulfonyl)methyl)azetidin-1-yl) isoquinolin-3-amine (81 mg, 0.12 mmol) was dissolved in THF (2 mL), 1 M TBAF solution (2 mL, 2 mmol) was added, and the mixture was warmed to reflux and stirred for 12 hours. The reaction solution was cooled to room temperature, and concentrated under reduced pressure, the reaction solution was subjected to liquid separation with dichloromethane and water, the organic phase was dried over anhydrous sodium sulfate, the organic solvent was concentrated under reduced pressure and separated by column chromatography to obtain the target compound N-(2-(3-chloro-1H-pyrazol-4-yl)pyrimidin-4-yl)-5-isopropyl-8-((2R,3S)-2-methyl-3-((methanesulfonyl)methyl)azetidin-1-yl)isoquinolin-3-amine (31 mg, 49.1%).

¹H NMR (400 MHz, DMSO-*d*₆) *δ* 13.50 (s, 1H), 10.22 (s, 1H), 9.09 (s, 1H), 8.68 (s, 1H), 8.41 (d, *J* = 6.0 Hz, 1H), 8.35 (s, 1H), 7.44 (d, *J* = 8.0 Hz, 1H), 7.25-7.18 (m, 1H), 6.58 (d, *J* = 8.2 Hz, 1H), 4.68 (t, *J* = 7.6 Hz, 1H), 4.25-4.15 (m, 1H), 3.65 (t, *J* = 7.1 Hz, 1H), 3.61-3.49 (m, 3H), 3.00 (s, 3H), 2.95-2.84 (m, 1H), 1.43 (d, *J* = 6.0 Hz, 3H), 1.36-1.26 (m, 6H);

MS m/z (ESI): 526.2 [M+H]⁺.

### Example 11

### Preparation of 2-(3-chloro-4-(4-((5-isopropyl-8-((2R,3 S)-2-methyl-3-((methanesulfonyl)methyl)azabut-1-yl)isoquinolin-3-yl)amino)pyrimidin-2-yl)-1H-pyrazol-1-yl)ethanol

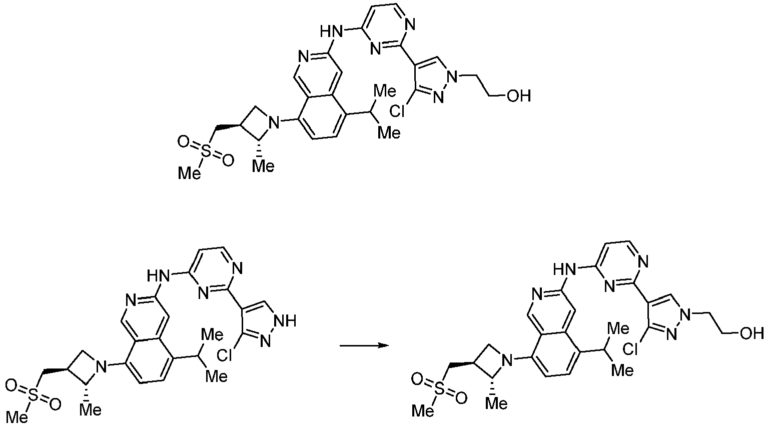

N-(2-(3-chloro-1H-pyrazol-4-yl)pyrimidin-4-yl)-5-isopropyl-8-((2R,3S)-2-methyl-3-((methanesulfonyl)methyl)azetidin-1-yl)isoquinolin-3-amine (57.9 mg, 0.11 mmol) was dissolved in DMF (2 mL), cesium carbonate (107.5 mg, 0.33 mmol) was added, and the mixture was warmed to 40°C and reacted for 12 hours. The reaction solution was cooled to room temperature, and concentrated under reduced pressure, the reaction solution was subjected to liquid separation with dichloromethane and water, the organic phase was dried over anhydrous sodium sulfate, the organic solvent was concentrated under reduced pressure and separated by column chromatography to obtain the target compound 2-(3-chloro-4-(4-((5-isopropyl-8-((2R,3 S)-2-methyl-3-((methanesulfonyl)methyl)azabut-1-yl)isoquinolin-3-yl)amino)pyrimidin-2-yl)-1H-pyrazol-1-yl)ethanol (21.1 mg, 33.6%).

¹H NMR (400 MHz, DMSO-*d*₆) *δ* 10.19 (s, 1H), 9.10 (s, 1H), 8.53 (s, 1H), 8.41 (d, *J* = 6.0 Hz, 1H), 8.32 (s, 1H), 7.43 (d, *J* = 7.8 Hz, 1H), 7.32-7.25 (m, 1H), 6.58 (d, *J* = 8.0 Hz, 1H), 5.03 (t, *J* = 5.2 Hz, 1H), 4.72-4.64 (m, 1H), 4.24-4.15 (m, 3H), 3.82-3.75 (m, 2H), 3.68-3.63 (m, 1H), 3.60-3.51 (m, 3H), 3.00 (s, 3H), 2.96-2.86 (m, 1H), 1.43 (d, *J* = 6.0 Hz, 3H), 1.35-1.27 (m, 6H);

MS m/z (ESI): 570.2 [M+H]⁺.

### Example 12

### Preparation of N-(2-(1-(2,2-difluorocyclopropyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

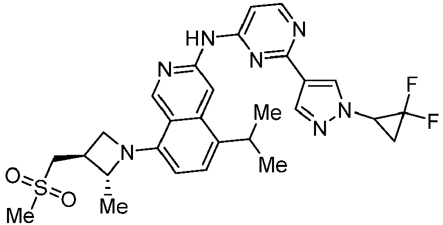

N-(2-(1-(2,2-difluorocyclopropyl)-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine was prepared with reference to example 11.

¹H NMR (400 MHz, DMSO-*d*₆) *δ* 10.27 (s, 1H), 9.10 (s, 1H), 8.81 (s, 1H), 8.45 (s, 1H), 8.37 (d, *J* = 5.8 Hz, 1H), 8.16 (s, 1H), 7.46 (d, *J* = 7.9 Hz, 1H), 7.13 (d, *J* = 5.2 Hz, 1H), 6.59 (d, *J* = 8.1 Hz, 1H), 4.68 (t, *J* = 7.4 Hz, 2H), 4.21 (t, *J* = 6.3 Hz, 1H), 3.68-3.49 (m, 4H), 3.00 (s, 3H), 2.93-2.86 (m, 1H), 2.63-2.55 (m, 1H), 2.43-2.35 (m, 1H), 1.43 (d, *J* = 6.0 Hz, 3H), 1.40-1.33 (m, 6H);

MS m/z (ESI): 568.2 [M+H]⁺.

### Example 13

### 1-(4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)cyclopropane-1-carbonitrile

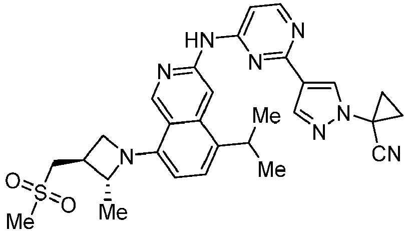

1-(4-(4-((5-Isopropyl-8-((2R,3 S)-2-methyl-3-((methanesulfonyl)methyl) azetidin-1-yl)isoquinolin-3-yl)amino)pyrimidin-2-yl)-1H-pyrazol-1-yl)cyclopropane-1-carbonitrile was prepared with reference to example 11.

¹H NMR (400 MHz, DMSO-*d*₆) *δ* 10.25 (s, 1H), 9.10 (s, 1H), 8.77 (s, 1H), 8.49 (s, 1H), 8.36 (d, *J* = 5.9 Hz, 1H), 8.11 (s, 1H), 7.46 (d, *J* = 8.0 Hz, 1H), 7.14 (d, *J* = 5.8 Hz, 1H), 6.59 (d, *J* = 8.1 Hz, 1H), 4.72-4.63 (m, 2H), 4.21 (t, *J* = 6.2 Hz, 1H), 3.68-3.50 (m, 4H), 3.00 (s, 3H), 2.92-2.85 (m, 1H), 2.56-2.52 (m, 1H), 2.09-2.02 (m, 1H), 1.87-1.80 (m, 1H), 1.43 (d, *J* = 6.0 Hz, 3H), 1.40-1.34 (m, 6H);MS m/z (ESI): 557.2 [M+H]⁺.

### Example 14

### 5-isopropyl-8-((2R,3 S)-2-methyl-3-((methanesulfonyl)methyl)azetidin-1-yl)-N-(2-(1-(1-methylpiperidin-4-yl)-1H-pyrazol-4-yl)pyrimidin-4-yl)isoquinolin-3-amine

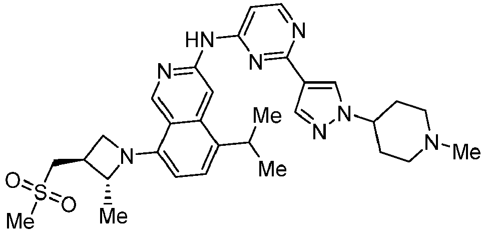

5-Isopropyl-8-((2R,3 S)-2-methyl-3-((methanesulfonyl)methyl)azetidin-1-yl)-N-(2-(1-(1-methylpiperidin-4-yl)-1H-pyrazol-4-yl)pyrimidin-4-yl)isoquinolin-3-amine was prepared with reference to example 11.

¹H NMR (400 MHz, DMSO-*d*₆) *δ* 10.13 (s, 1H), 9.03 (s, 1H), 8.70 (br s, 1H), 8.30-8.25 (m, 2H), 8.03 (s, 1H), 7.39 (d, *J* = 8.0 Hz, 1H), 7.06-7.03 (m ,1H), 6.52 (d, *J* = 8.0 Hz, 1H), 4.63-4.60 (m, 1H), 4.14-4.11 (m, 2H), 3.60- 3.45 (m, 4H), 2.93 (s, 3H), 2.85-2.81 (m, 3H), 2.15 (s, 3H), 1.99- 1.92 (m, 6H), 1.36 (d, J=6.0 Hz, 3H), 1.31-1.27 (m, 6H);

MS m/z (ESI): 589.3 [M+H]⁺.

### Example 15

### N-(2-(1-cyclopropyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

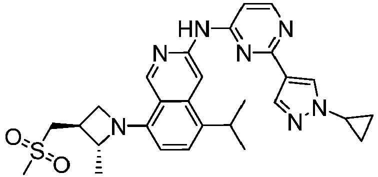

### Step 1 Synthesis of 3-chloro-5-isopropyl-8-((2R,3S)-2-methyl-3-((methanesulfonyl)methyl)azetidin-1-yl)isoquinoline

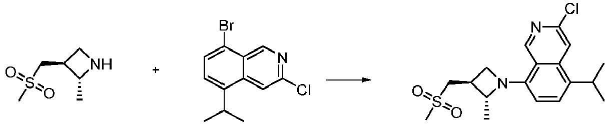

8-Bromo-3-chloro-5-isopropylisoquinoline (500 mg, 1.76 mmol) and (2R,3S)-2-methyl-3-((methanesulfonyl)methyl)azetidine (462 mg, 2.83 mmol) were dissolved in dioxane (10 mL), cesium carbonate (1.15 g, 3.54 mmol) and Xantphos Pd G4 (164 mg, 0.17 mmol) were added, and the mixture was warmed to 100°C and stirred for 12 hours. The reaction solution was cooled to room temperature, and concentrated under reduced pressure, the reaction solution was subjected to liquid separation with dichloromethane and water, the organic phase was dried over anhydrous sodium sulfate, the organic solvent was concentrated under reduced pressure and separated by column chromatography to obtain the target compound 3-chloro-5-isopropyl-8-((2R,3 S)-2-methyl-3-((methanesulfonyl)methyl)azetidin-1-yl)isoquinoline (318 mg, 49.2%).

MS m/z (ESI) :367.1 [M+H]⁺.

### Step 2 Synthesis of 2-(1-cyclopropyl-1H-pyrazol-4-yl)pyrimidin-4-amine

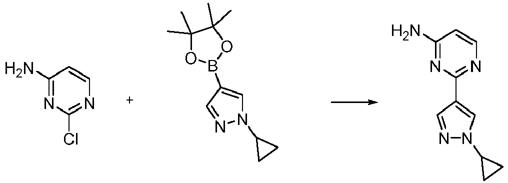

2-Chloropyrimidin-4-amine (1.29 g, 10 mmol), 1-cyclopropyl-4-(4,4,5,5-tetramethyl-1,3,2-dioxaborolan-2-yl)-1H-pyrazole (2.58 g, 11 mmol) and potassium carbonate (3.46 g, 25 mmol) were mixed in dioxane (20 mL) and water (2 mL), bis(triphenylphosphine)palladium dichloride (702 mg, 1 mmol) was added, and the mixture was warmed to 100°C and stirred for 12 hours. The reaction solution was cooled to room temperature, and concentrated under reduced pressure, the reaction solution was subjected to liquid separation with dichloromethane and water, the organic phase was dried over anhydrous sodium sulfate, and the organic solution was concentrated under reduced pressure and separated by column chromatography to obtain the target compound 2-(1-cyclopropyl-1H-pyrazol-4-yl)pyrimidin-4-amine (1.05 g, 52.2%).

MS m/z (ESI) :202.1 [M+H]⁺.

### Step 3 Synthesis of N-(2-(1-cyclopropyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

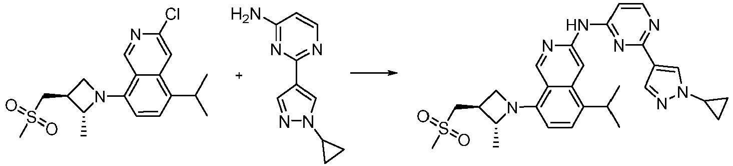

3-Chloro-5-isopropyl-8-((2R,3S)-2-methyl-3-((methanesulfonyl)methyl) azetidin-1-yl)isoquinoline (100 mg, 0.27 mmol) and 2-(1-cyclopropyl-1H-pyrazol-4-yl)pyrimidin-4-amine (54 mg, 0.27 mmol) were dissolved in dioxane (5 mL), cesium carbonate (274 mg, 0.84 mmol) and BrettPhos Pd G3 (24 mg, 27 µmol) were added, and the mixture was warmed to 100°C and stirred for 12 hours. The reaction solution was cooled to room temperature, concentrated under reduced pressure, the reaction solution was subjected to liquid separation with dichloromethane and water, the organic phase was dried over anhydrous sodium sulfate, the organic solvent was concentrated under reduced pressure and separated by column chromatography to obtain the target compound N-(2-(1-cyclopropyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine (31 mg, 21.6%).

¹H NMR (400 MHz, DMSO-*d*₆) *δ* 10.22 (s, 1H), 9.09 (s, 1H), 8.82 (s, 1H), 8.36-8.32 (m, 2H), 8.05 (s, 1H), 7.46 (d, *J=* 7.9 Hz, 1H), 7.15-7.05 (m, 1H), 6.59 (d, *J=* 8.1 Hz, 1H), 4.68 (t, *J=* 7.5 Hz, 1H), 4.25-4.16 (m, 1H), 3.87-3.80 (m, 1H), 3.68-3.51 (m, 4H), 3.00 (s, 3H), 2.95-2.85 (m, 1H), 1.43 (d, *J=* 6.0 Hz, 3H), 1.38 (dd, *J=* 6.8, 3.9 Hz, 6H), 1.17-1.11 (m, 2H), 1.06-1.00 (m, 2H);

MS m/z (ESI) :532.2 [M+H]⁺.

### Example 16

### 5-isopropyl-8-((2R,3 S)-2-methyl-3-((methanesulfonyl)methyl)azetidin-1-yl)-N-(2-(1-(tetrahydro-2H-pyran-2-yl)-1H-pyrazol-4-yl)pyrimidin-4-yl)isoquinolin-3-amine

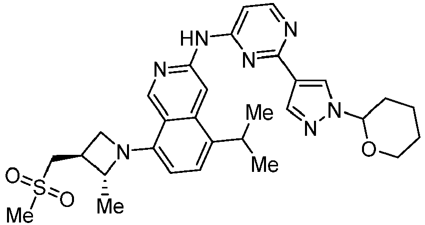

5-Isopropyl-8-((2R,3 S)-2-methyl-3-((methanesulfonyl)methyl)azetidin-1-yl)-N-(2-(1-(tetrahydro-2H-pyran-2-yl)-1H-pyrazol-4-yl)pyrimidin-4-yl)isoquinolin-3-amine was prepared with reference to example 15 or 11.

MS m/z (ESI): 576.2 [M+H]⁺.

### Example 17

### N-(2-(1H-pyrazol-4-yl)pyrimidin-4-yl)-5-isopropyl-8-((2R,3S)-2-methyl-3-((methanesulfonyl)methyl)azetidin-1-yl)isoquinolin-3-amine

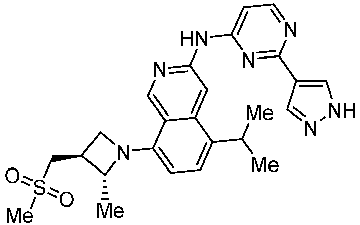

N-(2-(1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to example 15 or 10.

MS m/z (ESI): 492.2 [M+H]⁺.

### Example 18

### 2-(1-((4-(4-((5-isopropyl-8-((2R,3 S)-2-methyl-3-((methanesulfonyl)methyl)azetidin-1-yl)isoquinolin-3-yl)amino)pyrimidin-2-yl)-1H-pyrazol-1-yl)methyl)cyclopropyl)acetonitrile

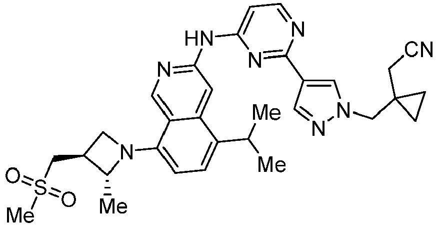

2-(1-((4-(4-((5-Isopropyl-8-((2R,3S)-2-methyl-3-((methanesulfonyl)methyl) azetidin-1-yl)isoquinolin-3-yl)amino)pyrimidin-2-yl)-1H-pyrazol-1-yl)methyl)cyclopropyl) acetonitrile was prepared with reference to example 15 or 11.

MS m/z (ESI): 585.2 [M+H]⁺.

### Example 19

### 1-((4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)methyl)cyclopropane-1-carbonitrile

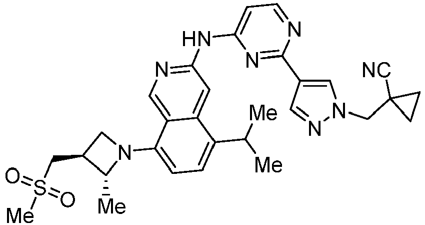

1-((4-(4-((5-Isopropyl-8-((2R,3 S)-2-methyl-3-((methanesulfonyl)methyl) azetidin-1-yl)isoquinolin-3-yl)amino)pyrimidin-2-yl)-1H-pyrazol-1-yl)methyl)cyclopropane-1-carbonitrile was prepared with reference to example 15 or 11.

MS m/z (ESI): 571.2 [M+H]⁺.

### Example 20

### 5-isopropyl-8-((2R,3 S)-2-methyl-3-((methanesulfonyl)methyl)azetidin-1-yl)-N-(2-(1-(oxetan-3-yl)-1H-pyrazol-4-yl)pyrimidin-4-yl)isoquinolin-3-amine

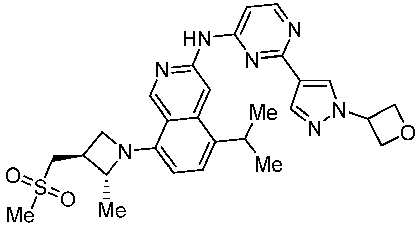

5-Isopropyl-8-((2R,3 S)-2-methyl-3-((methanesulfonyl)methyl)azetidin-1-yl)-N-(2-(1-(oxetan-3-yl)-1H-pyrazol-4-yl)pyrimidin-4-yl)isoquinolin-3-amine was prepared with reference to example 15 or 11.

MS m/z (ESI): 548.2 [M+H]⁺.

### Example 21

### 5-isopropyl-8-((2R,3 S)-2-methyl-3-((methanesulfonyl)methyl)azetidin-1-yl)-N-(2-(1-(oxetan-2-ylmethyl)-1H-pyrazol-4-yl)pyrimidin-4-yl)isoquinolin-3-amine

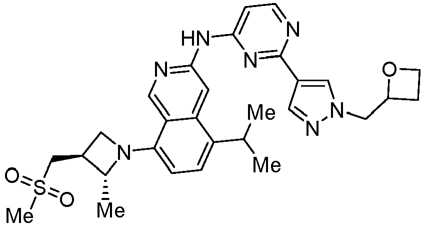

5-Isopropyl-8-((2R,3 S)-2-methyl-3-((methanesulfonyl)methyl)azetidin-1-yl)-N-(2-(1-(oxetan-2-ylmethyl)-1H-pyrazol-4-yl)pyrimidin-4-yl)isoquinolin-3-amine was prepared with reference to example 15 or 11.

¹H NMR (400 MHz, DMSO-*d*₆) *δ* 10.23 (s, 1H), 9.09 (s, 1H), 8.78 (s, 1H), 8.38-8.28 (m, 2H), 8.12 (s, 1H), 7.45 (d, *J* = 8.0 Hz, 1H), 7.13 (d, *J* = 5.4 Hz, 1H), 6.58 (d, *J* = 8.1 Hz, 1H), 5.11-5.02 (m, 1H), 4.68 (t, *J =* 7.5 Hz, 1H), 4.53-4.39 (m, 3H), 4.34-4.25 (m, 1H), 4.23-4.16 (m, 1H), 3.67-3.49 (m, 4H), 3.00 (s, 3H), 2.92-2.86 (m, 1H), 2.72-2.64 (m, 1H), 2.47-2.40 (m, 1H), 1.43 (d, *J=* 6.0 Hz, 3H), 1.39-1.33 (m, 6H);

MS m/z (ESI): 562.2 [M+H]⁺.

### Example 22A

### 5-isopropyl-8-((2R,3 S)-2-methyl-3-((methanesulfonyl)methyl)azetidin-1-yl)-N-(2-(1-((S)-1,1,1-trifluoropropan-2-yl)-1H-pyrazol-4-yl)pyrimidin-4-yl)isoquinolin-3-amine

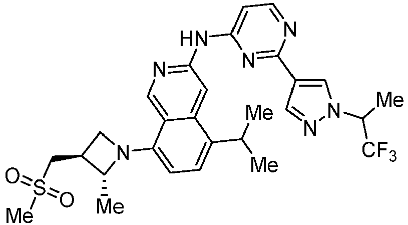

5-Isopropyl-8-((2R,3 S)-2-methyl-3-((methanesulfonyl)methyl)azetidin-1-yl)-N-(2-(1-((S)-1,1,1-trifluoropropan-2-yl)-1H-pyrazol-4-yl)pyrimidin-4-yl)isoquinolin-3-amine was prepared with reference to example 15 or 11.

MS m/z (ESI): 588.2 [M+H]⁺.

### Example 22

### 5-isopropyl-8-((2R,3 S)-2-methyl-3-((methanesulfonyl)methyl)azetidin-1-yl)-N-(2-(1-((S)-1,1,1-trifluoropropan-2-yl)-1H-pyrazol-4-yl)pyrimidin-4-yl)isoquinolin-3-amine

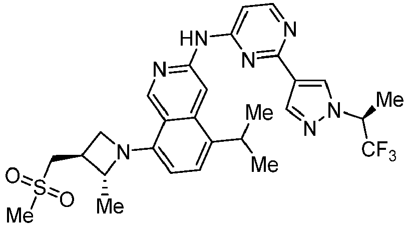

5-Isopropyl-8-((2R,3 S)-2-methyl-3-((methanesulfonyl)methyl)azetidin-1-yl)-N-(2-(1-((S)-1,1,1-trifluoropropan-2-yl)-1H-pyrazol-4-yl)pyrimidin-4-yl)isoquinolin-3-amine was prepared with reference to example 15 or 11.

¹H NMR (400 MHz, DMSO-*d*₆) *δ* 10.26 (s, 1H), 9.11 (s, 1H), 8.69 (s, 1H), 8.49 (s, 1H), 8.38 (d, *J* = 5.9 Hz, 1H), 8.20 (s, 1H), 7.45 (d, *J* = 8.0 Hz, 1H), 7.21 (d, *J* = 5.8 Hz, 1H), 6.59 (d, *J* = 8.1 Hz, 1H), 5.53 (p, *J* = 7.2 Hz, 1H), 4.69 (t, *J* = 7.5 Hz, 1H), 4.21 (p, *J* = 6.1 Hz, 1H), 3.70-3.47 (m, 4H), 3.00 (s, 3H), 2.94-2.86 (m, 1H), 1.76 (d, *J* = 7.0 Hz, 3H), 1.44 (d, *J=* 6.0 Hz, 3H), 1.35 (m, 6H);

MS m/z (ESI): 588.2 [M+H]⁺.

### Example 23

### 5-isopropyl-8-((2R,3 S)-2-methyl-3-((methanesulfonyl)methyl)azetidin-1-yl)-N-(2-(1-((R)-1,1,1-trifluoropropan-2-yl)-1H-pyrazol-4-yl)pyrimidin-4-yl)isoquinolin-3-amine

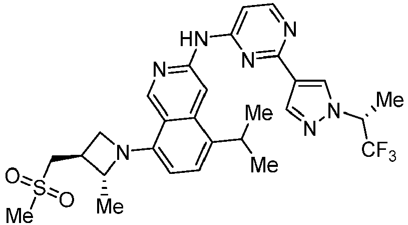

5-Isopropyl-8-((2R,3S)-2-methyl-3-((methanesulfonyl)methyl)azetidin-1-yl)-N-(2-(1-((R)-1,1,1-trifluoropropan-2-yl)-1H-pyrazol-4-yl)pyrimidin-4-yl)isoquinolin-3-amine was prepared with reference to example 15 or 11.

¹H NMR (400 MHz, DMSO-*d*₆) *δ* 10.26 (s, 1H), 9.11 (s, 1H), 8.69 (s, 1H), 8.49 (s, 1H), 8.38 (d, *J* = 5.9 Hz, 1H), 8.20 (s, 1H), 7.45 (d, *J* = 8.0 Hz, 1H), 7.21 (d, *J* = 5.8 Hz, 1H), 6.59 (d, *J* = 8.1 Hz, 1H), 5.53 (p, *J* = 7.2 Hz, 1H), 4.69 (t, *J* = 7.5 Hz, 1H), 4.21 (p, *J* = 6.1 Hz, 1H), 3.70-3.47 (m, 4H), 3.00 (s, 3H), 2.94-2.86 (m, 1H), 1.76 (d, *J* = 7.0 Hz, 3H), 1.44 (d, *J=* 6.0 Hz, 3H), 1.35 (m, 6H);

MS m/z (ESI): 588.2 [M+H]⁺.

### Example 24

### 5-isopropyl-N-(2-(1-methyl-1H-pyrazol-4-yl)pyrimidin-4-yl)-8-((2R,3 S)-2-methyl-3-((methanesulfonyl)methyl)azetidin-1-yl)isoquinolin-3-amine

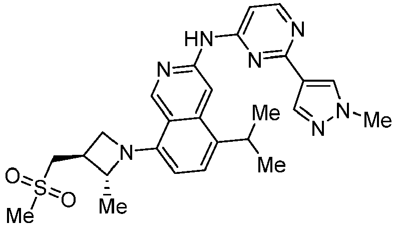

5-Isopropyl-N-(2-(1-methyl-1H-pyrazol-4-yl)pyrimidin-4-yl)-8-((2R,3 S)-2-methyl-3-((methanesulfonyl)methyl)azetidin-1-yl)isoquinolin-3-amine was prepared with reference to example 15.

¹H NMR (400 MHz, DMSO-*d*₆) *δ* 10.21 (s, 1H), 9.10 (s, 1H), 8.82 (s, 1H), 8.34 (d, *J* = 5.9 Hz, 1H), 8.28 (s, 1H), 8.07 (s, 1H), 7.46 (d, *J* = 8.0 Hz, 1H), 7.10 (d, *J* = 5.8 Hz, 1H), 6.59 (d, *J* = 8.0 Hz, 1H), 4.68 (t, *J* = 7.5 Hz, 1H), 4.34-4.18 (m, 1H), 3.94 (s, 3H), 3.70-3.44 (m, 4H), 3.00 (s, 3H), 2.90 (q, *J=* 7.3 Hz, 1H), 1.43 (d, *J=* 6.0 Hz, 3H), 1.37 (m, 6H);

MS m/z (ESI): 506.2 [M+H]⁺.

### Example 25

### N-(2-(1-(difluoromethyl)-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine

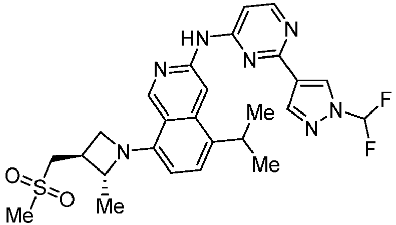

N-(2-(1-(difluoromethyl)-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine was prepared with reference to example 15 or 11.

¹H NMR (400 MHz, DMSO-*d*₆) *δ* 10.35 (s, 1H), 9.11 (s, 1H), 8.79 (br s, 1H), 8.73 (s, 1H), 8.42 (d, *J* = 8.0 Hz, 1H), 8.36 (s, 1H), 7.94 (t, *J* = 60 Hz, 1H), 7.46 (d, *J* = 8.0 Hz, 1H), 7.21 (s, 1H), 6.60 (d, *J* = 8.0 Hz, 1H), 4.69 (t, *J* = 7.6 Hz, 1H), 4.21 (t, *J* = 7.6 Hz, 1H), 3.69-3.40 (m, 4H), 3.00 (s, 3H), 2.92-2.87 (m, 1H), 1.44 (d, *J* = 8.0 Hz, 3H), 1.35(d, *J* = 8.0Hz, 6H);

MS m/z (ESI): 542.2 [M+H]⁺.

### Example 26

### 5-isopropyl-8-((2R,3 S)-2-methyl-3-((methanesulfonyl)methyl)azetidin-1-yl)-N-(2-(1-(2,2,2-trifluoroethyl)-1H-pyrazol-4-yl)pyrimidin-4-yl)isoquinolin-3-amine

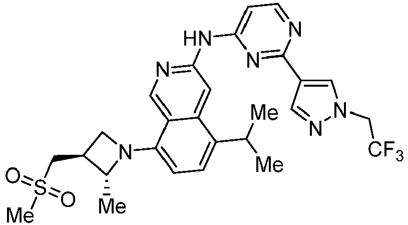

5-Isopropyl-8-((2R,3 S)-2-methyl-3-((methanesulfonyl)methyl)azetidin-1-yl)-N-(2-(1-(2,2,2-trifluoroethyl)-1H-pyrazol-4-yl)pyrimidin-4-yl)isoquinolin-3-amine was prepared with reference to example 15 or 11.

¹H NMR (400 MHz, DMSO-*d*₆) *δ* 10.26 (s, 1H), 9.10 (s, 1H), 8.70 (br s, 1H), 8.44 (s, 1H), 8.37 (d, *J* = 7.2Hz, 1H), 8.20 (s, 1H), 7.46 (d, *J* = 8.0 Hz, 1H), 7.20 (s, 1H), 6.60 (d, *J* = 8.0 Hz, 1H),5.32-5.25 (m, 2H), 4.68 (t, *J* = 7.6 Hz, 1H), 4.21 (t, *J* = 7.6 Hz, 1H), 3.68-3.34 (m, 4H), 3.00 (s, 3H), 2.92-2.87 (m, 1H), 1.44-1.31(m, 9H);

MS m/z (ESI): 574.2 [M+H]⁺.

### Example 27

### N-(2-(1-(2-fluoroethyl)-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine

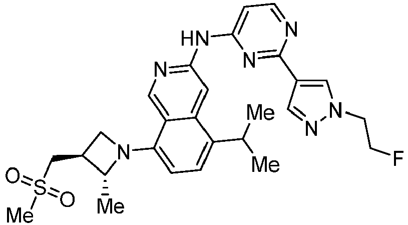

N-(2-(1-(2-fluoroethyl)-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine was prepared with reference to example 15 or 11.

¹H NMR (400 MHz, DMSO-*d*₆) *δ* 10.26 (s, 1H), 9.10 (s, 1H), 8.79 (br s, 1H), 8.37-8.35 m, 2H), 8.14 (s, 1H), 7.46 (d, *J* = 8.0 Hz, 1H), 7.13 (s, 1H), 6.60 (d, *J* = 8.0 Hz, 1H),4.89 (m, 1H), 4.78 (m, 1H), 4.68 (m, 1H), 4.57 (m, 1H), 4.50 (m,1H), 4.21 (m, 1H), 3.67-3.31 (m, 4H), 3.00 (s, 3H), 2.92-2.88 (m, 1H), 1.44-1.33(m, 9H);

MS m/z (ESI): 538.2 [M+H]⁺.

### Example 28

### N-(2-(1-((1-(fluoromethyl)cyclopropyl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

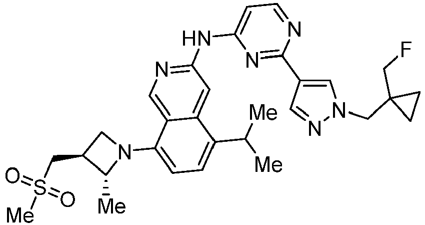

N-(2-(1-((1-(fluoromethyl)cyclopropyl)methyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to example 15 or 11.

MS m/z (ESI): 578.2 [M+H]⁺.

### Example 29

### N-(2-(1-(1-(fluoromethyl)cyclopropyl)-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine

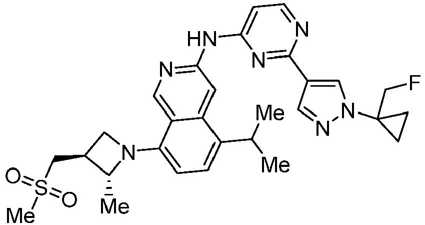

N-(2-(1-(1-(fluoromethyl)cyclopropyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to example 15 or 11.

¹H NMR (400 MHz, DMSO-*d*₆) *δ* 10.25 (s, 1H), 9.10 (s, 1H), 8.81 (s, 1H), 8.38-8.32 (m, 2H), 8.13 (s, 1H), 7.46 (d, *J* = 8.0 Hz, 1H), 7.09 (s, 1H), 6.59 (d, *J* = 8.1 Hz, 1H), 4.74 (s, 1H), 4.68 (t, *J=* 7.6 Hz, 1H), 4.62 (s, 1H), 4.24-4.17 (m, 1H), 3.68-3.51 (m, 4H), 3.00 (s, 3H), 2.93-2.85 (m, 1H), 1.46-1.36 (m, 11H), 1.31-1.26 (m, 2H);

MS m/z (ESI): 564.2 [M+H]⁺.

### Example 30

### N-(2-(1-((1-fluorocyclopropyl)methyl)-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine

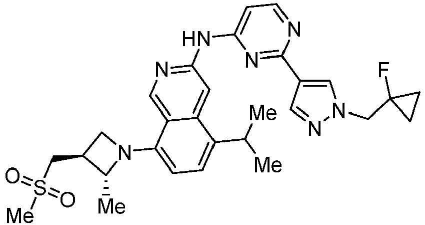

N-(2-(1-((1-fluorocyclopropyl)methyl)-1H-pyrazol-4-yl)pyrimidin-4-yl)-5-isopropyl-8-((2R,3 S)-2-methyl-3-((methanesulfonyl)methyl)azetidin-1-yl)isoquinolin-3 -amine was prepared with reference to example 15 or 11.

MS m/z (ESI): 564.2 [M+H]⁺.

### Example 31

### N-(2-(3-(cyclopropylsulfonyl)-1-methyl-1H-pyrazol-5-yl)pyrimidin-4-yl)-5-isopropyl-8-((2R,3S)-2-methyl-3-((methanesulfonyl)methyl)azetidin-1-yl)isoquinolin-3-amine

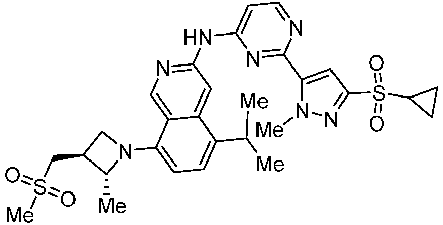

N-(2-(3-(cyclopropylsulfonyl)-1-methyl-1H-pyrazol-5-yl)pyrimidin-4-yl)-5-isopropyl-8-((2R,3 S)-2-methyl-3-((methanesulfonyl)methyl)azetidin-1-yl)isoquinolin-3 -amine was prepared with reference to example 15.

MS m/z (ESI): 610.2 [M+H]⁺.

### Example 32

### Preparation of 5-isopropyl-8-((2R,3 S)-2-methyl-3-((methanesulfonyl)methyl)azetidin-1-yl)-N-(2-(5-(trifluoromethyl)thien-3-yl)pyrimidin-4-yl)isoquinolin-3-amine

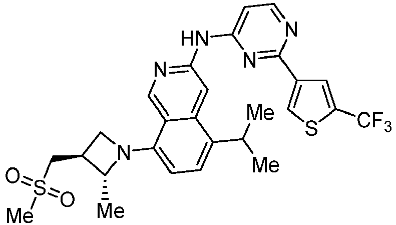

5-Isopropyl-8-((2R,3 S)-2-methyl-3-((methanesulfonyl)methyl)azetidin-1-yl)-N-(2-(5-(trifluoromethyl)thien-3-yl)pyrimidin-4-yl)isoquinolin-3-amine was prepared with reference to example 15.

MS m/z (ESI): 576.2 [M+H]⁺.

### Example 33

### Preparation of 1-(4-(4-((5-isopropyl-8-((2R,3S)-2-methyl-3-((methanesulfonyl)methyl)azetidin-1-yl)isoquinolin-3-yl)amino)pyrimidin-2-yl)thien-2-yl)ethan-1-one

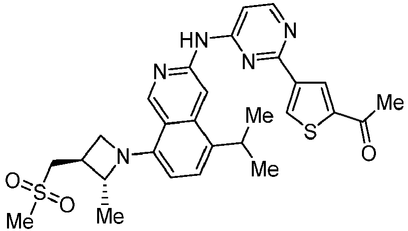

1-(4-(4-((5-Isopropyl-8-((2R,3 S)-2-methyl-3-((methanesulfonyl)methyl) azetidin-1-yl)isoquinolin-3-yl)amino)pyrimidin-2-yl)thien-2-yl)ethan-1-one was prepared with reference to example 15.

¹H NMR (400 MHz, DMSO-*d*₆) *δ* 10.36 (s, 1H), 9.12 (s, 1H), 8.64 (s, 1H), 8.60 (s, 1H), 8.52-8.40 (m, 2H), 7.43 (d, *J* = 8.0 Hz, 1H), 7.40-7.34 (m, 1H), 6.58 (d, *J* = 8.0 Hz, 1H), 4.69 (t, *J* = 7.5 Hz, 1H), 4.23-4.19 (m, 1H), 3.66 (t, *J* = 7.2 Hz, 1H), 3.58-3.51 (m, 2H), 3.01 (s, 3H), 2.90-2.85 (m, 1H), 2.62 (s, 3H), 1.52-1.22 (m, 10H);

MS m/z (ESI): 550.2 [M+H]⁺.

### Example 34

### Preparation of 5-isopropyl-8-((2R,3 S)-2-methyl-3-((methanesulfonyl)methyl)azetidin-1-yl)-N-(2-(5-(trifluoromethyl)furan-3-yl)pyrimidin-4-yl)isoquinolin-3-amine

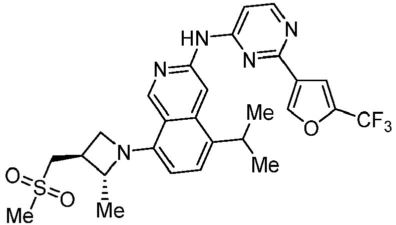

5-Isopropyl-8-((2R,3 S)-2-methyl-3-((methanesulfonyl)methyl)azetidin-1-yl)-N-(2-(5-(trifluoromethyl)furan-3-yl)pyrimidin-4-yl)isoquinolin-3-amine was prepared with reference to example 15.

MS m/z (ESI): 560.2 [M+H]⁺.

### Example 35

### Preparation of 1-(4-(4-((5-isopropyl-8-((2R,3S)-2-methyl-3-((methanesulfonyl)methyl)azetidin-1-yl)isoquinolin-3-yl)amino)pyrimidin-2-yl)furan-2-yl)ethan-1-one

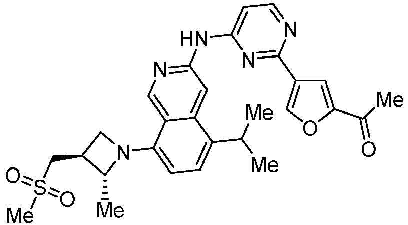

1-(4-(4-((5-Isopropyl-8-((2R,3 S)-2-methyl-3-((methanesulfonyl)methyl) azetidin-1-yl)isoquinolin-3-yl)amino)pyrimidin-2-yl)furan-2-yl)ethan-1-one was prepared with reference to example 15.

¹H NMR (400 MHz, DMSO-*d*₆) *δ* 10.31 (d, *J* = 2.6 Hz, 1H), 9.04 (d, *J* = 2.6 Hz, 1H), 8.67 (s, 1H), 8.54 (d, *J* = 2.7 Hz, 1H), 8.38-8.33 (m, 1H), 7.82 (d, *J* = 2.6 Hz, 1H), 7.43-7.35 (m, 1H), 7.19 (s, 1H), 6.55-6.50 (m, 1H), 4.63 (d, *J* = 8.6 Hz, 1H), 4.14 (s, 1H), 3.62-3.41 (m, 7H), 2.93 (d, *J* = 2.6 Hz, 3H), 1.40-1.25 (m, 9H), 1.17 (s, 1H);

MS m/z (ESI): 534.2 [M+H]⁺.

### Example 36

### Preparation of N-(2-(1-cyclopropyl-1H-pyrazol-4-yl)pyrimidin-4-yl)-7-fluoro-5-isopropyl-8-((2R,3S)-2-methyl-3-((methanesulfonyl)methyl)azetidin-1-yl)isoquinolin-3-amine

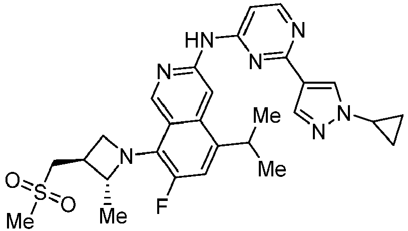

N-(2-(1-cyclopropyl-1H-pyrazol-4-yl)pyrimidin-4-yl)-7-fluoro-5-isopropyl-8-((2R,3 S)-2-methyl-3-((methanesulfonyl)methyl)azetidin-1-yl)isoquinolin-3-amine was prepared with reference to example 15.

MS m/z (ESI): 550.2 [M+H]⁺.

### Example 37

### Preparation of N-(2-(1-cyclopropyl-1H-pyrazol-4-yl)pyrimidin-4-yl)-6-fluoro-5-isopropyl-8-((2R,3S)-2-methyl-3-((methanesulfonyl)methyl)azetidin-1-yl)isoquinolin-3-amine

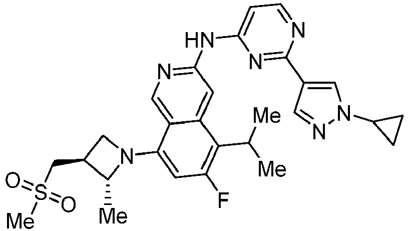

N-(2-(1-cyclopropyl-1H-pyrazol-4-yl)pyrimidin-4-yl)-6-fluoro-5-isopropyl-8-((2R,3 S)-2-methyl-3-((methanesulfonyl)methyl)azetidin-1-yl)isoquinolin-3-amine was prepared with reference to example 15.

MS m/z (ESI): 550.2 [M+H]⁺.

### Example 38

### Preparation of 7-fluoro-5-isopropyl-N-(2-(1-methyl-1H-pyrazol-4-yl)pyrimidin-4-yl)-8-((2R,3S)-2-methyl-3-((methanesulfonyl)methyl)azetidin-1-yl)isoquinolin-3-amine

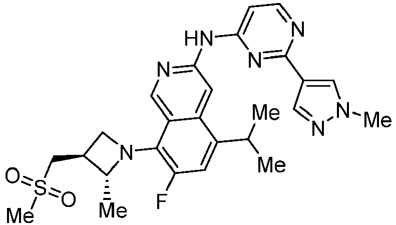

7-Fluoro-5-isopropyl-N-(2-(1-methyl-1H-pyrazol-4-yl)pyrimidin-4-yl)-8-((2R,3 S)-2-methyl-3-((methanesulfonyl)methyl)azetidin-1-yl)isoquinolin-3-amine was prepared with reference to example 15.

MS m/z (ESI): 524.2 [M+H]⁺.

### Example 39

### Preparation of 6-fluoro-5-isopropyl-N-(2-(1-methyl-1H-pyrazol-4-yl)pyrimidin-4-yl)-8-((2R,3S)-2-methyl-3-((methanesulfonyl)methyl)azetidin-1-yl)isoquinolin-3-amine

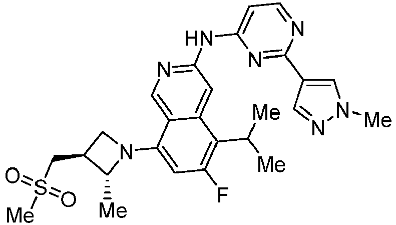

6-Fluoro-5-isopropyl-N-(2-(1-methyl-1H-pyrazol-4-yl)pyrimidin-4-yl)-8-((2R,3 S)-2-methyl-3-((methanesulfonyl)methyl)azetidin-1-yl)isoquinolin-3-amine was prepared with reference to example 15.

¹H NMR (400 MHz, DMSO-*d*₆) *δ* 10.24 (s, 1H), 9.03 (s, 1H), 8.85 (s, 1H), 8.36 (d, *J* = 5.8 Hz, 1H), 8.26 (s, 1H), 8.04 (d, *J* = 0.8 Hz, 1H), 7.10 (d, *J* = 5.8 Hz, 1H), 6.35 (d, *J* = 14.4 Hz, 1H), 4.73 (t, *J* = 7.8 Hz, 1H), 4.31-4.20 (m, 1H), 3.93 (s, 3H), 3.76 (t, *J* = 7.2 Hz, 1H), 3.63-3.48 (m, 3H), 3.00 (s, 3H), 2.96-2.82 (m, 1H), 1.49-1.39 (m, 9H);

MS m/z (ESI): 524.2 [M+H]⁺.

### Example 40

### Preparation of 2-(1-(3-((2-(1-(cyclopropylsulfonyl)-1H-pyrazol-4-yl)pyrimidin-4-yl)amino)-5-isopropylisoquinolin-8-yl)azetidin-3-yl)acetonitrile

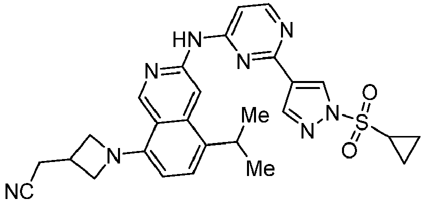

2-(1-(3-((2-(1-(Cyclopropylsulfonyl)-1H-pyrazol-4-yl)pyrimidin-4-yl)amino)-5-isopropylisoquinolin-8-yl)azetidin-3-yl)acetonitrile was prepared with reference to example 15 or 1.

¹H NMR (400 MHz, DMSO- *d₆*) *δ* 10.38 (s, 1H), 9.12 (s, 1H), 8.81-8.74 (m, 1H), 8.70 (s, 1H), 8.52 (s, 1H), 8.44 (d, *J* = 5.8 Hz, 1H), 7.46 (d, *J* = 7.8 Hz, 1H), 7.28-7.22 (m, 1H), 6.47 (d, *J* = 8.0 Hz, 1H), 4.39-4.33 (m, 2H), 3.96-3.89 (m, 2H), 3.63-3.56 (m, 1H), 3.16-3.07 (m, 1H), 3.01-2.94 (m, 2H), 1.42-1.33 (m, 7H), 1.32-1.21 (m, 4H);

MS m/z (ESI): 529.2 [M+H]⁺.

### Example 41

### Preparation of 1-(3-((2-(1-(cyclopropylsulfonyl)-1H-pyrazol-4-yl)pyrimidin-4-yl)amino)-5-isopropylisoquinolin-8-yl)azetidin-3-carbonitrile

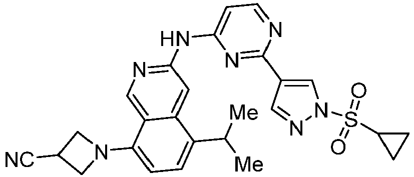

1-(3-((2-(1-(Cyclopropylsulfonyl)-1H-pyrazol-4-yl)pyrimidin-4-yl)amino)-5-isopropylisoquinolin-8-yl)azetidin-3-carbonitrile was prepared with reference to example 15 or 1.

MS m/z (ESI): 515.2 [M+H]⁺.

### Example 42

### Preparation of 2-((2R,3S)-1-(3-((2-(1-(cyclopropylsulfonyl)-1H-pyrazol-4-yl)pyrimidin-4-yl)amino)-5-isopropylisoquinolin-8-yl)-2-methylazetidin-3-yl)acetonitrile

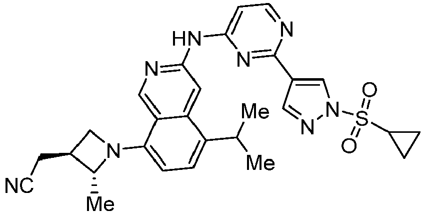

2-((2R,3 S)-1-(3-((2-(1-(Cyclopropylsulfonyl)-1H-pyrazol-4-yl)pyrimidin-4-yl)amino)-5-isopropylisoquinolin-8-yl)-2-methylazetidin-3-yl)acetonitrile was prepared with reference to example 15 or 1.

MS m/z (ESI): 543.2 [M+H]⁺.

### Example 43

### Preparation of (2R,3S)-1-(3-((2-(1-(cyclopropylsulfonyl)-1H-pyrazol-4-yl)pyrimidin-4-yl)amino)-5-isopropylisoquinolin-8-yl)-2-methylazetidin-3-carbonitrile

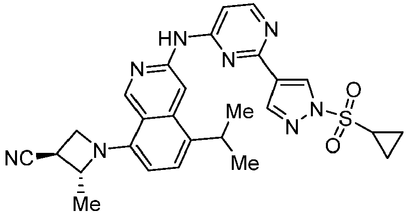

(2R,3 S)-1-(3-((2-(1-(Cyclopropylsulfonyl)-1H-pyrazol-4-yl)pyrimidin-4-yl)amino)-5-isopropylisoquinolin-8-yl)-2-methylazetidin-3-carbonitrile was prepared with reference to example 15 or 1.

MS m/z (ESI): 529.2 [M+H]⁺.

### Example 44

### Preparation of 2-(1-(5-isopropyl-3-((2-(1-methyl-1H-pyrazol-4-yl)pyrimidin-4-yl)amino)isoquinolin-8-yl)azetidin-3-yl)acetonitrile

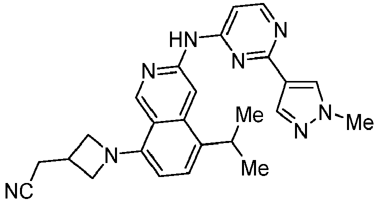

2-(1-(5-Isopropyl-3-((2-(1-methyl-1H-pyrazol-4-yl)pyrimidin-4-yl)amino) isoquinolin-8-yl)azetidin-3-yl)acetonitrile was prepared with reference to example 15.

¹H NMR (400 MHz, DMSO-*d*₆) *δ* 10.19 (s, 1H), 9.10 (s, 1H), 8.81 (s, 1H), 8.34 (d, *J* = 5.8 Hz, 1H), 8.28 (s, 1H), 8.07 (s, 1H), 7.46 (d, *J* = 8.0 Hz, 1H), 7.14-7.08 (m, 1H), 6.45 (d, *J* = 8.0 Hz, 1H), 4.34 (t, *J* = 7.8 Hz, 2H), 3.98-3.87 (m, 5H), 3.65-3.60 (m, 1H), 3.15-3.04 (m, 1H), 2.97 (d, *J* = 6.8 Hz, 2H), 1.36 (d, *J* = 6.8 Hz, 6H);

MS m/z (ESI): 439.2 [M+H]⁺.

### Example 45

### Preparation of 2-((2R,3S)-1-(5-isopropyl-3-((2-(1-methyl-1H-pyrazol-4-yl)pyrimidin-4-yl)amino)isoquinolin-8-yl)-2-methylazetidin-3-yl)acetonitrile

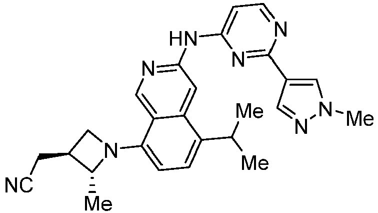

2-((2R,3 S)-1-(5-Isopropyl-3-((2-(1-methyl-1H-pyrazol-4-yl)pyrimidin-4-yl)amino)isoquinolin-8-yl)-2-methylazetidin-3-yl)acetonitrile was prepared with reference to example 15.

MS m/z (ESI): 453.2 [M+H]⁺.

### Example 46

### Preparation of 2-(1-(3-((2-(1-cyclopropyl-1H-pyrazol-4-yl)pyrimidin-4-yl)amino)-5-isopropylisoquinolin-8-yl)azetidin-3-yl)acetonitrile

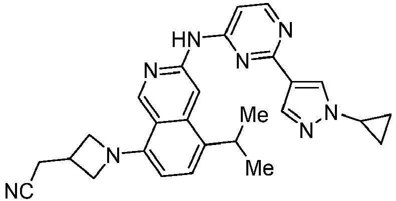

2-(1-(3-((2-(1-Cyclopropyl-1H-pyrazol-4-yl)pyrimidin-4-yl)amino)-5-isopropylisoquinolin-8-yl)azetidin-3-yl)acetonitrile was prepared with reference to example 15.

MS m/z (ESI): 465.2 [M+H]⁺.

### Example 47

### Preparation of 2-((2R,3S)-1-(3-((2-(1-cyclopropyl-1H-pyrazol-4-yl)pyrimidin-4-yl)amino)-5-isopropylisoquinolin-8-yl)-2-methylazetidin-3-yl)acetonitrile

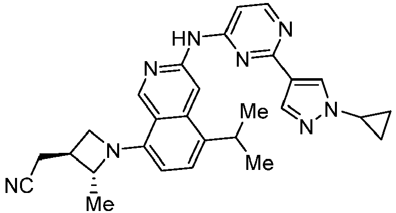

2-((2R,3 S)-1-(3-((2-(1-cyclopropyl-1H-pyrazol-4-yl)pyrimidin-4-yl)amino)-5-isopropylisoquinolin-8-yl)-2-methylazetidin-3-yl)acetonitrile was prepared with reference to example 15.

MS m/z (ESI): 479.2 [M+H]⁺.

### Example 48

### Preparation of 2-(4-(4-((5-isopropyl-8-((2R,3 S)-2-methyl-3-((methanesulfonyl)methyl)azetidin-1-yl)isoquinolin-3-yl)amino)pyrimidin-2-yl)-1H-pyrazol-1-yl)ethan-1-ol

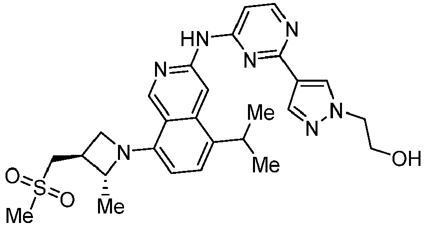

2-(4-(4-((5-Isopropyl-8-((2R,3S)-2-methyl-3-((methanesulfonyl)methyl) azetidin-1-yl)isoquinolin-3-yl)amino)pyrimidin-2-yl)-1H-pyrazol-1-yl)ethan-1-ol was prepared with reference to example 11 or 15.

¹H NMR (400 MHz, DMSO-*d*₆) *δ* 10.22 (s, 1H), 9.10 (s, 1H), 8.80 (s, 1H), 8.35 (d, *J* = 7.2 Hz, 1H), 8.29 (s, 1H), 8.09 (s, 1H), 7.46 (d, *J* = 7.2 Hz, 1H), 7.11 (br s, 1H), 6.59 (d, *J* = 7.2 Hz, 1H), 4.70-4.66 (m, 1H), 4.23-4.19 (m, 3H), 3.87-3.51 (m, 6H), 3.00 (s, 3H), 2.91-2.88 (m, 1H), 1.43 (d, *J* = 6.4 Hz, 3H), 1.31-1.26 (m, 6H);

MS m/z (ESI): 536.2 [M+H]⁺.

### Example 49

### Preparation of 5-isopropyl-N-(2-(1-(1-methoxycyclopropyl)-1H-pyrazol-4-yl)pyrimidin-4-yl)-8-((2R,3 S)-2-methyl-3-((methanesulfonyl)methyl)azetidin-1-yl)isoquinolin-3 -amine

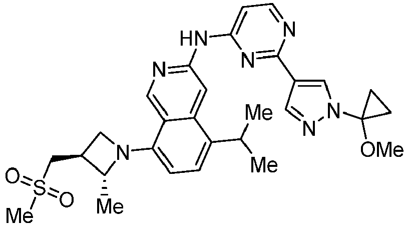

5-Isopropyl-N-(2-(1-(1-methoxycyclopropyl)-1H-pyrazol-4-yl)pyrimidin-4-yl)-8-((2R,3 S)-2-methyl-3-((methanesulfonyl)methyl)azetidin-1-yl)isoquinolin-3-amine was prepared with reference to example 11 or 15.

¹H NMR (400 MHz, DMSO-*d*₆) *δ* 10.21 (s, 1H), 9.03 (s, 1H), 8.79 (s, 1H), 8.41 (s, 1H), 8.30 (d, *J* = 6.0 Hz, 1H), 8.10 (s, 1H), 7.40 (d, *J* = 8.0 Hz, 1H), 7.06-7.02 (m, 1H), 6.53 (d, *J* = 8.2 Hz, 1H), 4.61 (t, *J* = 7.6 Hz, 1H), 4.18-4.12(m, 1H), 3.62-3.54 (m, 2H), 3.55-3.44 (m, 3H), 3.20 (s, 3H), 2.93 (s, 3H), 2.87-2.78 (m, 1H), 1.37-1.31 (m, 12H);

MS m/z (ESI): 562.3 [M+H]⁺.

### Example 50

### Preparation of 2-(1-(4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)cyclopropyl)acetonitrile

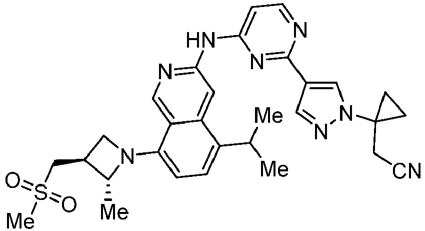

2-(1-(4-(4-((5-Isopropyl-8-((2R,3S)-2-methyl-3-((methanesulfonyl)methyl) azetidin-1-yl)isoquinolin-3-yl)amino)pyrimidin-2-yl)-1H-pyrazol-1-yl)cyclopropyl) acetonitrile was prepared with reference to example 11 or 15.

¹H NMR (400 MHz, DMSO-*d*₆) *δ* 10.18 (s, 1H), 9.03 (s, 1H), 8.70 (s, 1H), 8.33 (s, 1H), 8.29 (d, *J* = 6.0 Hz, 1H), 8.09 (s, 1H), 7.40 (d, *J* = 7.8 Hz, 1H), 7.08-7.04 (m, 1H), 6.53 (d, *J* = 8.0 Hz, 1H), 4.61 (t, *J* = 7.6 Hz, 1H), 4.18-4.11 (m, 1H), 3.61-3.56 (m, 1H), 3.52-3.45 (m, 3H), 3.17 (s, 2H), 2.93 (s, 3H), 2.86-2.79 (m, 1H), 1.38-1.30 (m, 10H), 1.19-1.13 (m, 3H);

MS m/z (ESI): 571.3 [M+H]⁺.

### Example 51

### Preparation of N-(2-(1-allyl-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine

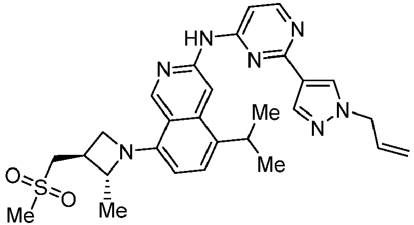

N-(2-(1-allyl-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine was prepared with reference to example 11 or 15.

¹H NMR (400 MHz, DMSO-*d*₆) *δ* 10.23 (s, 1H), 9.10 (s, 1H), 8.79 (br s, 1H), 8.37-8.30 (m, 2H), 8.11 (s, 1H), 7.46 (d, *J* = 7.2 Hz, 1H), 7.15 (br s, 1H), 6.59 (d, *J* = 7.2 Hz, 1H), 5.34-5.15 (m, 3H), 4.86 (d, J = 7.2 Hz, 2H), 4.70-4.66 (m, 1H), 4.22-4.19 (m, 1H), 3.66-3.52 (m, 3H), 3.00 (s, 3H), 2.90-2.87 (m, 1H), 2.01-1.99 (m, 1H), 1.43 (d, *J* = 6.4 Hz, 3H), 1.39-1.36 (m, 6H);

MS m/z (ESI): 532.2 [M+H]⁺.

### Example 52

### Preparation of N-(2-(1-(but-2-yn-1-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

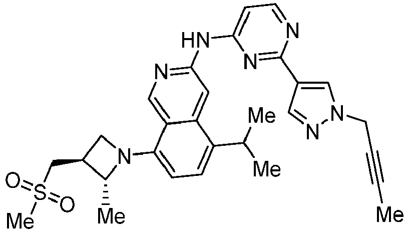

N-(2-(1-(but-2-yn-1-yl)-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine was prepared with reference to example 11 or 15.

¹H NMR (400 MHz, DMSO-*d*₆) *δ* 10.22 (s, 1H), 9.10 (s, 1H), 8.79 (s, 1H), 8.37-8.35 (m, 2H), 8.09 (s, 1H), 7.46 (d, *J* = 7.2 Hz, 1H), 7.13 (br s, 1H), 6.59 (d, *J* = 7.2 Hz, 1H), 5.08 (s, 2H), 4.70-4.66 (m, 1H), 4.25-4.19 (m, 1H), 3.66-3.51 (m, 3H), 3.00 (s, 3H), 2.91-2.87 (m, 1H), 2.01-1.99 (m, 1H), 1.86 (s, 3H), 1.43 (d, *J* = 6.4 Hz, 3H), 1.39-1.36 (m, 6H);

MS m/z (ESI): 544.2 [M+H]⁺.

### Example 53

### Preparation of 5-isopropyl-8-((2R,3 S)-2-methyl-3-((methanesulfonyl)methyl)azetidin-1-yl)-N-(2-(1-(prop-2-yn-1-yl)-1H-pyrazol-4-yl)pyrimidin-4-yl)isoquinolin-3-amine

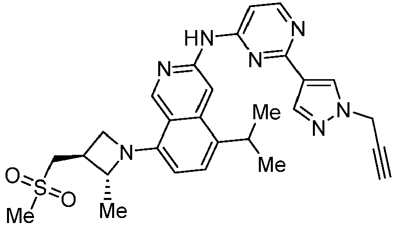

5-Isopropyl-8-((2R,3 S)-2-methyl-3-((methanesulfonyl)methyl)azetidin-1-yl)-N-(2-(1-(prop-2-yn-1-yl)-1H-pyrazol-4-yl)pyrimidin-4-yl)isoquinolin-3-amine was prepared with reference to example 11 or 15.

¹H NMR (400 MHz, DMSO-*d*₆) *δ* 10.23 (s, 1H), 9.10 (s, 1H), 8.77 (s, 1H), 8.37-8.35 (m, 2H), 8.11 (s, 1H), 7.46 (d, *J* = 7.2 Hz, 1H), 7.15 (br s, 1H), 6.59 (d, *J* = 7.2 Hz, 1H), 5.15 (s, 2H), 4.70-4.66 (m, 1H), 4.22-4.19 (m, 1H), 3.66-3.52 (m, 4H), 3.00 (s, 3H), 2.90-2.87 (m, 1H), 2.01-1.99 (m, 1H), 1.43 (d, *J* = 6.4 Hz, 3H), 1.39-1.36 (m, 6H);

MS m/z (ESI): 530.2 [M+H]⁺.

### Example 54

### Preparation of 5-isopropyl-8-((2R,3 S)-2-methyl-3-((methanesulfonyl)methyl)azetidin-1-yl)-N-(2-(thiazol-5-yl)pyrimidin-4-yl)isoquinolin-3-amine

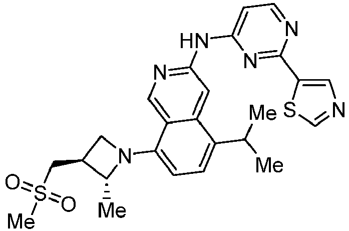

5-Isopropyl-8-((2R,3 S)-2-methyl-3-((methanesulfonyl)methyl)azetidin-1-yl)-N-(2-(thiazol-5-yl)pyrimidin-4-yl)isoquinolin-3-amine was prepared with reference to example 15.

¹H NMR (400 MHz, DMSO-*d*₆) *δ* 10.46 (s, 1H), 9.29 (s, 1H), 9.12 (s, 1H), 8.76 (s, 1H), 8.66 (s, 1H), 8.43 (d, *J* = 5.8 Hz, 1H), 7.48 (d, *J* = 8.0 Hz, 1H), 7.26 (s, 1H), 6.61 (d, *J* = 8.0 Hz, 1H), 4.69-4.67 (m, 1H), 4.21-4.20 (m, 1H), 3.70-3.68 (m, 2H), 3.63-3.45 (m, 2H), 3.00 (s, 3H), 2.90-2.89 (m, 1H), 1.44 (d, *J* = 6.0 Hz, 3H), 1.38-1.35 (m, 6H);

MS m/z (ESI): 509.2 [M+H]⁺.

### Example 55

### Preparation of 4-(4-((5-isopropyl-8-((2R,3S)-2-methyl-3-((methanesulfonyl)methyl)azetidin-1-yl)isoquinolin-3-yl)amino)pyrimidin-2-yl)-1-methyl-1H-pyrazole-3-carbonitrile

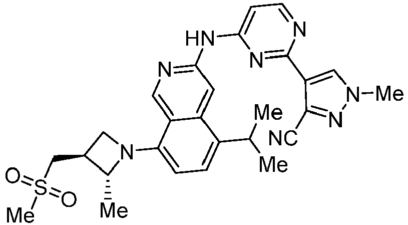

4-(4-((5-Isopropyl-8-((2R,3 S)-2-methyl-3-((methanesulfonyl)methyl)azetidin-1-yl)isoquinolin-3-yl)amino)pyrimidin-2-yl)-1-methyl-1H-pyrazole-3-carbonitrile was prepared with reference to example 15.

¹H NMR (400 MHz, DMSO-*d*₆) *δ* 10.30 (s, 1H), 9.11 (s, 1H), 8.58 (s, 1H), 8.48-8.44 (m, 2H), 7.44 (d, *J* = 8.0 Hz, 1H), 7.34 (s, 1H), 6.59 (d, *J* = 8.1 Hz, 1H), 4.69 (t, *J* = 7.5 Hz, 1H), 4.23-4.17 (m, 1H), 4.05 (s, 3H), 3.68-3.49 (m, 4H), 3.00 (s, 3H), 2.93-2.85 (m, 1H), 1.43 (d, *J* = 6.1 Hz, 3H), 1.35-1.29 (m, 6H);

MS m/z (ESI): 531.2 [M+H]⁺.

### Example 56

### Preparation of 5-isopropyl-8-((2R,3S)-2-methyl-3-((methanesulfonyl)methyl)azetidin-1-yl)-N-(2-(1-methyl-3-(trifluoromethyl)-1H-pyrazol-4-yl)pyrimidin-4-yl)isoquinolin-3-amine

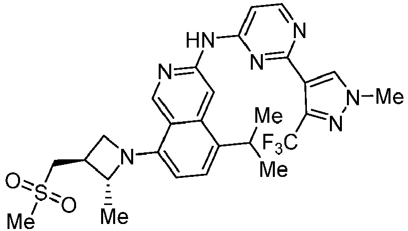

5-Isopropyl-8-((2R,3S)-2-methyl-3-((methanesulfonyl)methyl)azetidin-1-yl)-N-(2-(1-methyl-3-(trifluoromethyl)-1H-pyrazol-4-yl)pyrimidin-4-yl)isoquinolin-3-amine was prepared with reference to example 15.

¹H NMR (400 MHz, DMSO-*d*₆) *δ* 10.21 (s, 1H), 9.10 (s, 1H), 8.59 (s, 1H), 8.50-8.33 (m, 2H), 7.44 (d, *J* = 7.6 Hz, 1H), 7.31 (d, *J* = 5.8 Hz, 1H), 6.58 (d, *J* = 8.0 Hz, 1H), 4.69-4.67 (m, 1H), 4.21-4.19 (m, 1H), 4.02 (s, 3H), 3.69-3.59 (m, 1H), 3.59-3.48 (m, 3H), 3.00 (s, 3H), 2.89-2.86 (m, 1H), 1.43 (d, *J* = 6.0 Hz, 3H), 1.36-1.32 (m, 6H);

MS m/z (ESI): 574.2 [M+H]⁺.

### Example 57

### Preparation of N-(2-(3-ethyl-1-methyl-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine

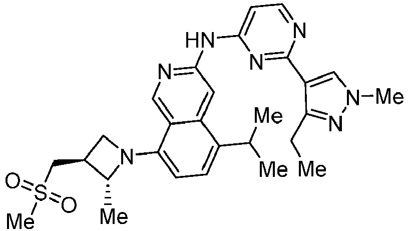

N-(2-(3-ethyl-1-methyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to example 15.

¹H NMR (400 MHz, DMSO-*d*₆) *δ* 10.08 (s, 1H), 9.10 (s, 1H), 8.58 (s, 1H), 8.36 (d, *J* = 5.8 Hz, 1H), 8.18 (s, 1H), 7.44 (d, *J* = 8.0 Hz, 1H), 7.17 (d, *J* = 5.6 Hz, 1H), 6.58 (d, *J* = 8.0 Hz, 1H), 4.68 (t, *J* = 7.4 Hz, 1H), 4.21 (t, *J* = 6.2 Hz, 1H), 3.86 (s, 3H), 3.65 (t, *J* = 7.1 Hz, 1H), 3.59-3.49 (m, 3H), 3.04-2.99 (m, 5H), 2.93-2.86 (m, 1H), 1.43 (d, *J* = 6.0 Hz, 3H), 1.36-1.32 (m, 6H), 1.22-1.17 (m, 3H);

MS m/z (ESI): 534.3 [M+H]⁺.

### Example 58

### Preparation of 2-(4-(4-((5-isopropyl-8-((2R,3S)-2-methyl-3-((methanesulfonyl)methyl)azetidin-1-yl)isoquinolin-3-yl)amino)pyrimidin-2-yl)-3-methyl-1H-pyrazol-1-yl)ethan-1-ol

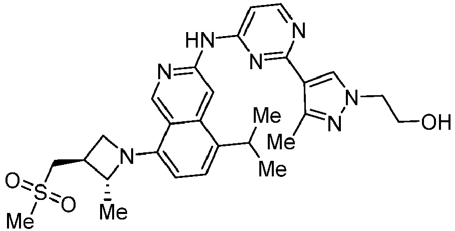

2-(4-(4-((5-Isopropyl-8-((2R,3S)-2-methyl-3-((methanesulfonyl)methyl) azetidin-1-yl)isoquinolin-3-yl)amino)pyrimidin-2-yl)-3-methyl-1H-pyrazol-1-yl)ethan-1-ol was prepared with reference to example 11 or 15.

¹H NMR (400 MHz, DMSO-*d*₆) *δ* 10.23 (s, 1H), 9.11 (s, 1H), 8.60 (s, 1H), 8.37 (d, *J* = 7.2 Hz, 1H), 8.13 (s, 1H), 7.46 (d, *J* = 7.2 Hz, 1H), 7.21 (br s, 1H), 6.59 (d, *J* = 7.2 Hz, 1H), 4.99-4.91 (m, 1H), 4.71-4.67 (m, 1H), 4.23-4.12 (m, 3H), 3.82-3.49 (m, 6H), 3.00 (s, 3H), 2.90-2.87 (m, 1H), 2.52 (s, 3H), 1.43 (d, *J* = 6.4 Hz, 3H), 1.39-1.36 (m, 6H);

MS m/z (ESI): 550.3 [M+H]⁺.

### Example 59

### Preparation of 2-(4-(4-((5-isopropyl-8-((2R,3S)-2-methyl-3-((methanesulfonyl)methyl)azetidin-1-yl)isoquinolin-3-yl)amino)pyrimidin-2-yl)-3-(trifluoromethyl)-1H-pyrazol-1-yl)ethan-1-ol

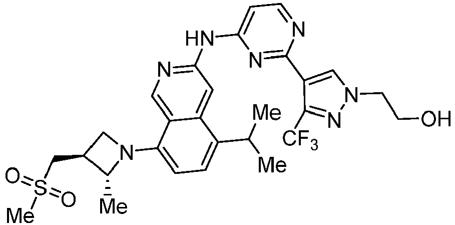

2-(4-(4-((5-Isopropyl-8-((2R,3S)-2-methyl-3-((methanesulfonyl)methyl) azetidin-1-yl)isoquinolin-3-yl)amino)pyrimidin-2-yl)-3-(trifluoromethyl)-1H-pyrazol-1-yl)ethan-1-ol was prepared with reference to example 11 or 15.

¹H NMR (400 MHz, DMSO-*d*₆) *δ* 10.20 (s, 1H), 9.11 (s, 1H), 8.49 (s, 1H), 8.43 (d, *J* = 6.2 Hz, 2H), 7.44 (d, *J* = 8.0 Hz, 1H), 7.36 (s, 1H), 6.58 (d, *J* = 8.0 Hz, 1H), 5.08 (s, 1H), 4.68-4.66 (m, 1H), 4.30-4.26 (m, 2H), 4.23-4.21 (m, 2H), 3.82 (s, 2H), 3.65-3.63 (m, 1H), 3.58-3.43 (m, 3H), 3.00 (s, 3H), 1.43 (d, *J=* 6.0 Hz, 3H), 1.36-1.32 (m, 6H);

MS m/z (ESI): 604.2 [M+H]⁺.

### Example 60

### Preparation of N-(1-(3-((2-(1,3-dimethyl-1H-pyrazol-4-yl)pyrimidin-4-yl)amino)-5-isopropylisoquinolin-8-yl)azetidin-3-yl)methanesulfonamide

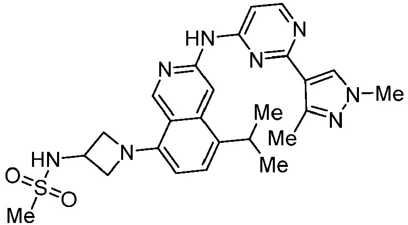

N-(1-(3-((2-(1,3-dimethyl-1H-pyrazol-4-yl)pyrimidin-4-yl)amino)-5-isopropylisoquinolin-8-yl)azetidin-3-yl)methanesulfonamide was prepared with reference to example 15.

¹H NMR (400 MHz, DMSO-*d*₆) *δ* 10.07 (s, 1H), 9.08 (s, 1H), 8.54 (s, 1H), 8.37 (d, *J* = 5.8 Hz, 1H), 8.18 (s, 1H), 7.84 (s, 1H), 7.44 (d, *J* = 8.0 Hz, 1H), 7.21 (d, *J* = 5.8 Hz, 1H), 6.46 (d, *J* = 8.0 Hz, 1H), 4.54 (t, *J* = 7.4 Hz, 2H), 4.37-4.33 (m, 1H), 3.92-3.89(m, 2H), 3.85 (s, 3H), 3.56-3.53 (m, 1H), 2.97 (s, 3H), 2.53 (s, 3H), 1.37-1.33 (m, 6H);

MS m/z (ESI): 507.2 [M+H]⁺.

### Example 61

### Preparation of 1-((2R,3S)-1-(3-((2-(1,3-dimethyl-1H-pyrazol-4-yl)pyrimidin-4-yl)amino)-5-isopropylisoquinolin-8-yl)-2-methyl azetidin-3-yl)-N,N-dimethyl methanesulfonamide

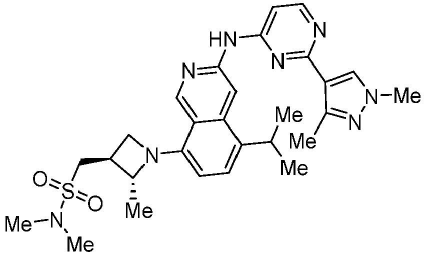

1-((2R,3S)-1-(3-((2-(1,3-dimethyl-1H-pyrazol-4-yl)pyrimidin-4-yl)amino)-5-isopropylisoquinolin-8-yl)-2-methyl azetidin-3-yl)-N,N-dimethyl methanesulfonamide was prepared with reference to example 15.

¹H NMR (400 MHz, DMSO-*d*₆) δ 10.09 (s, 1H), 9.10 (s, 1H), 8.57 (s, 1H), 8.36 (d, *J* = 5.8 Hz, 1H), 8.19 (s, 1H), 7.44 (d, *J* = 8.0 Hz, 1H), 7.18 (d, *J* = 6.0 Hz, 1H), 6.57 (d, *J* = 8.0 Hz, 1H), 4.66-4.63 (m, 1H), 4.20-4.15 (m, 1H), 3.85 (s, 3H), 3.64-3.61 (m, 1H), 3.57 -3.55(m, 1H), 3.52-3.39 (m, 2H), 2.88-2.80 (m, 1H), 2.77 (s, 6H), 2.53 (s, 3H), 1.44 (d, *J* = 6.0 Hz, 3H), 1.37-1.33 (m, 6H);

MS m/z (ESI): 549.3 [M+H]⁺.

### Example 62

### Preparation of N-(2-(3-fluoro-1-methyl-1H-pyrazolyl-4-yl)pyrimidin-4-yl)-5-isopropyl-8-((2R,3S)-2-methyl-3-((methanesulfonyl)methyl)azabut-1-yl)isoquinolin-3-amine

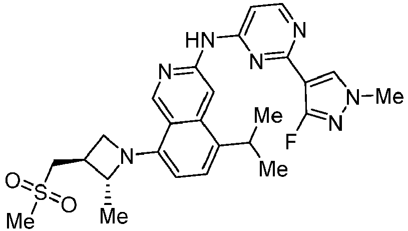

N-(2-(3-fluoro-1-methyl-1H-pyrazolyl-4-yl)pyrimidin-4-yl)-5-isopropyl-8-((2R,3 S)-2-methyl-3-((methanesulfonyl)methyl)azabut-1-yl)isoquinolin-3-amine was prepared with reference to example 15.

¹H NMR (400 MHz, DMSO-*d*₆) *δ* 10.20 (s, 1H), 9.09 (s, 1H), 8.66 (s, 1H), 8.35 (d, *J* = 5.9 Hz, 1H), 8.21 (s, 1H), 7.44 (d, *J* = 8.0 Hz, 1H), 7.15 (s, 1H), 6.58 (d, *J* = 8.2 Hz, 1H), 4.67 (t, *J* = 7.4 Hz, 1H), 4.24-4.14 (m, 1H), 3.82 (s, 3H), 3.66-3.50 (m, 4H), 3.00 (s, 3H), 2.92-2.86 (m, 1H), 1.43 (d, *J* = 6.0 Hz, 3H), 1.33-1.26 (m, 6H);

MS m/z (ESI): 524.2 [M+H]⁺.

### Example 63

### Preparation of N-(2-(3-chloro-1-methyl-1H-pyrazol-4-yl)pyrimidin-4-yl)-5-isopropyl-8-(3-((methanesulfonyl)methyl)cyclobutyl)isoquinolin-3-amine

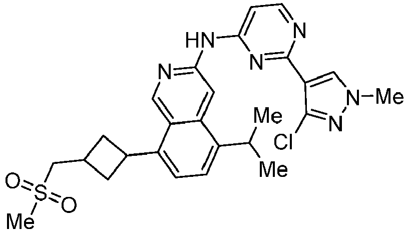

N-(2-(3-chloro-1-methyl-1H-pyrazol-4-yl)pyrimidin-4-yl)-5-isopropyl-8-(3-((methanesulfonyl)methyl)cyclobutyl)isoquinolin-3-amine was prepared with reference to example 15.

¹H NMR (400 MHz, DMSO-*d*₆) *δ* 10.24 (d, *J* = 5.9 Hz, 1H), 9.17 (d, *J* = 24.3 Hz, 1H), 8.71 (s, 1H), 8.41 (d, *J* = 5.9 Hz, 1H), 8.35 (s, 1H), 7.60-7.55 (m, 1H), 7.41-7.22 (m, 2H), 4.51-4.08 (m, 1H), 3.91 (s, 3H), 3.73-3.64 (m, 1H), 3.58 (d, *J* = 7.5 Hz, 1H), 3.31-3.27 (m, 3H), 2.97 (d, *J* = 10.9 Hz, 3H), 2.87-2.74 (m, 2H), 2.15-2.05 (m, 1H), 1.34 (dd, *J* = 6.7, 3.5 Hz, 6H);

MS m/z (ESI): 525.2 [M+H]⁺.

### Example 64

### Preparation of 5-isopropyl-8-((2R,3S)-2-methyl-3-((methanesulfonyl)methyl)azetidin-1-yl)-N-(2-(1-(methyl-d3)-3-nitro-1H-pyrazol-4-yl)pyrimidin-4-yl)isoquinolin-3-amine

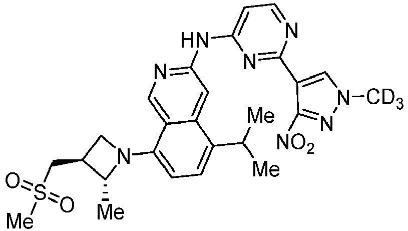

5-Isopropyl-8-((2R,3S)-2-methyl-3-((methanesulfonyl)methyl)azetidin-1-yl)-N-(2-(1-(methyl-d3)-3-nitro-1H-pyrazol-4-yl)pyrimidin-4-yl)isoquinolin-3-amine was prepared with reference to example 15.

MS m/z (ESI): 554.2 [M+H]⁺.

### Example 65 Preparation of N-(2-(3-fluoro-1-(methyl-d3)-1H-pyrazol-4-yl)pyrimidin-4-yl)-5-isopropyl-8-((2R,3S)-2-methyl-3-((methylsulfonyl)methyl)azabut-1-yl)isoquinolin-3-amine

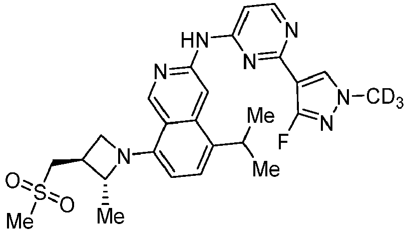

N-(2-(3-fluoro-1-(methyl-d3)-1H-pyrazol-4-yl)pyrimidin-4-yl)-5-isopropyl-8-((2R,3S)-2-methyl-3-((methylsulfonyl)methyl)azabut-1-yl)isoquinolin-3-amine was prepared with reference to example 15.

¹H NMR (400 MHz, DMSO-*d*₆) δ 10.20 (s, 1H), 9.09 (s, 1H), 8.66 (s, 1H), 8.35 (d, *J* = 5.9 Hz, 1H), 8.21 (d, *J* = 1.9 Hz, 1H), 7.44 (d, *J* = 8.0 Hz, 1H), 7.16 (d, *J* = 5.8 Hz, 1H), 6.58 (d, *J* = 8.1 Hz, 1H), 4.67 (t, *J* = 7.4 Hz, 1H), 4.20 (t, *J* = 6.3 Hz, 1H), 3.71-3.48 (m, 4H), 3.00 (s, 3H), 2.89 (dd, *J* = 14.3, 7.2 Hz, 1H), 1.43 (d, *J* = 6.0 Hz, 3H), 1.31 (dd, *J* = 6.5, 5.0 Hz, 6H);

MS m/z (ESI): 527.2 [M+H]⁺.

### Example 66

### Preparation of 5-isopropyl-N-(2-(1-((1-(methoxymethyl)cyclopropyl)methyl)-1H-pyrazol-4-yl)pyrimidin-4-yl)-8-((2R,3S)-2-methyl-3-((methanesulfonyl)methyl)azetidin-1-yl)isoquinolin-3-amine

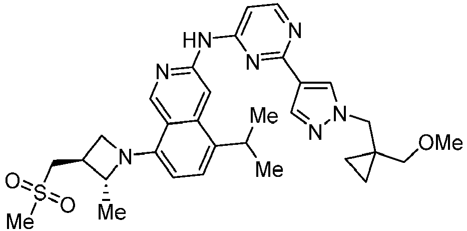

5-Isopropyl-N-(2-(1-((1-(methoxymethyl)cyclopropyl)methyl)-1H-pyrazol-4-yl)pyrimidin-4-yl)-8-((2R,3S)-2-methyl-3-((methanesulfonyl)methyl)azetidin-1-yl)isoquinolin-3-amine was prepared with reference to example 11 or 15.

¹H NMR (400 MHz, DMSO-*d*₆) *δ* 10.21 (s, 1H), 9.10 (s, 1H), 8.76 (s, 1H), 8.35 (d, *J* = 5.8 Hz, 1H), 8.25 (s, 1H), 8.09 (s, 1H), 7.45 (d, *J* = 8.0 Hz, 1H), 7.19-7.13 (m, 1H), 6.58 (d, *J* = 8.0 Hz, 1H), 4.69 (t, *J* = 7.6 Hz, 1H), 4.27-4.09 (m, 3H), 3.68-3.51 (m, 4H), 3.23 (s, 3H), 3.09 (s, 2H), 3.00 (s, 3H), 2.93-2.86 (m, 1H), 1.43 (d, *J* = 6.2 Hz, 3H), 1.40-1.33 (m, 6H), 0.74-0.65 (m, 2H), 0.61-0.48 (m, 2H);

MS m/z (ESI): 590.3 [M+H]⁺.

### Example 67

### Preparation of 3-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)propanenitrile

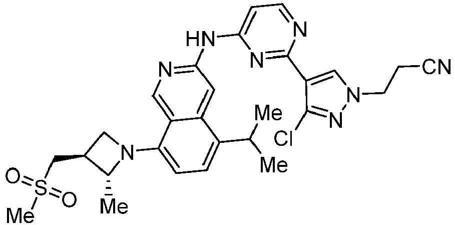

3-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl) propanenitrile was prepared with reference to example 11 or 15.

¹H NMR (400 MHz, DMSO-*d*₆) *δ* 10.20 (s, 1H), 9.10 (s, 1H), 8.60-8.37 (m, 3H), 7.51-7.30 (m, 2H), 6.58 (s, 1H), 4.75-4.64 (m, 1H), 4.45-4.39 (m, 2H), 4.25-4.12 (m, 1H), 3.69-3.54 (m, 4H), 3.17 (s, 2H), 3.00 (s, 3H), 2.92-2.85 (m, 1H), 1.43 (d, *J* = 6.0 Hz, 3H), 1.35-1.19 (m, 6H);

MS m/z (ESI): 579.2 [M+H]⁺.

### Example 68 Preparation of 2-(1-(3-((2-(3-chloro-1-methyl-1H-pyrazol-4-yl)pyrimidin-4-yl)amino)-5-isopropylisoquinolin-8-yl)azetidin-3-yl)isothiazolidine 1,1-dioxide

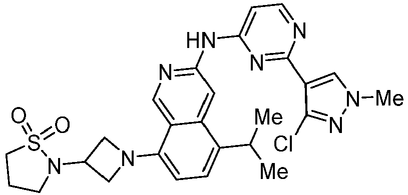

2-(1-(3-((2-(3-Chloro-1-methyl-1H-pyrazol-4-yl)pyrimidin-4-yl)amino)-5-isopropylisoquinolin-8-yl)azetidin-3-yl)isothiazolidine 1,1-dioxide was prepared with reference to example 15.

¹H NMR (400 MHz, DMSO-*d*₆) δ 10.18 (s, 1H), 9.10 (s, 1H), 8.57 (s, 1H), 8.41 (d, *J* = 5.9 Hz, 1H), 8.34 (s, 1H), 7.43 (d, *J* = 8.0 Hz, 1H), 7.28 (s, 1H), 6.47 (d, *J*= 8.1 Hz, 1H), 4.46-4.31 (m, 3H), 4.29-4.20 (m, 2H), 3.90 (s, 3H), 3.65-3.52 (m, 1H), 3.43 (t, *J* = 6.6 Hz, 2H), 3.33-3.23 (m, 2H), 2.30-2.25 (m, 2H), 1.30 (d, *J* = 6.8 Hz, 6H);

MS m/z (ESI): 553.2 [M+H]⁺.

### Example 69 Preparation of 2-(1-(3-((2-(1,3-dimethyl-1H-pyrazol-4-yl)pyrimidin-4-yl)amino)-5-isopropylisoquinolin-8-yl)azetidin-3-yl)isothiazolidine 1,1-dioxide

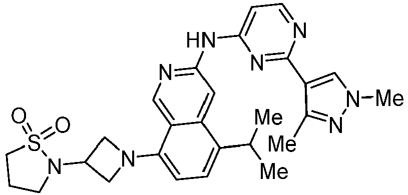

2-(1-(3-((2-(1,3-Dimethyl-1H-pyrazol-4-yl)pyrimidin-4-yl)amino)-5-isopropylisoquinolin-8-yl)azetidin-3-yl)isothiazolidine 1,1-dioxide was prepared with reference to example 15.

¹H NMR (400 MHz, DMSO-*d*₆) δ 10.08 (s, 1H), 9.10 (s, 1H), 8.55 (s, 1H), 8.37 (d, *J* = 5.9 Hz, 1H), 8.19 (s, 1H), 7.44 (d, *J* = 8.0 Hz, 1H), 7.22 (d, *J* = 5.6 Hz, 1H), 6.47 (d, *J* = 8.0 Hz, 1H), 4.41-4.30 (m, 3H), 4.26-4.22 (m, 2H), 3.85 (s, 3H), 3.62-3.50 (m, 1H), 3.43 (t, *J* = 6.6 Hz, 2H), 3.32-3.24 (m, 3H), 2.48-2.44 (m, 2H), 2.34-2.24 (m, 2H), 1.33 (d, *J* = 6.8 Hz, 6H);

MS m/z (ESI): 533.2 [M+H]⁺.

### Example 70

### Preparation of N-(2-(3-chloro-1-cyclopropyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

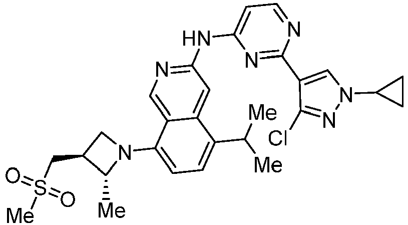

N-(2-(3-chloro-1-cyclopropyl-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine was prepared with reference to example 15.

¹H NMR (400 MHz, DMSO-*d*₆) δ 10.21 (s, 1H), 9.09 (s, 1H), 8.62 (s, 1H), 8.41 (d, *J* = 5.1 Hz, 2H), 7.44 (d, *J* = 8.0 Hz, 1H), 7.23 (d, *J* = 4.5 Hz, 1H), 6.58 (d, *J* = 8.1 Hz, 1H), 4.68 (s, 1H), 4.23-4.15 (m, 1H), 3.86-3.80 (m, 1H), 3.66-3.48 (m, 4H), 3.00 (s, 3H), 2.93-2.84 (m, 1H), 1.43 (d, *J* = 6.1 Hz, 3H), 1.32 (dd, *J* = 6.7, 4.9 Hz, 6H), 1.18-1.12 (m, 2H), 1.06-1.00 (m, 2H);

MS m/z (ESI): 566.2 [M+H]⁺.

### Example 71

### Preparation of N-(2-(1-(2-aminoethyl)-3-chloro-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

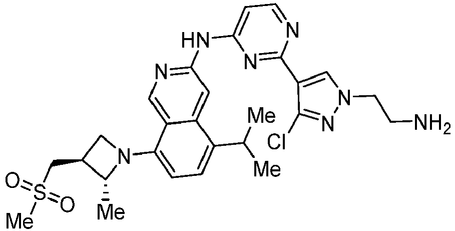

N-(2-(1-(2-aminoethyl)-3-chloro-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine was prepared with reference to example 11 or 15.

MS m/z (ESI): 569.2 [M+H]⁺.

### Example 72

### Preparation of methyl (1-(3-((2-(3-chloro-1-methyl-1H-pyrazol-4-yl)pyrimidin-4-yl)amino)-5-isopropylisoquinolin-8-yl)azetidin-3-yl)carbamate

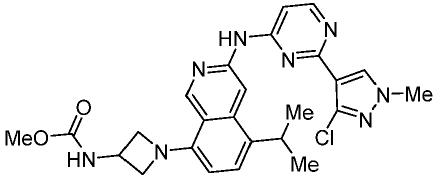

Methyl (1-(3-((2-(3-chloro-1-methyl-1H-pyrazol-4-yl)pyrimidin-4-yl)amino)-5-isopropylisoquinolin-8-yl)azetidin-3-yl)carbamate was prepared with reference to example 15.

¹H NMR (400 MHz, DMSO-*d*₆) *δ* 10.17 (s, 1H), 9.08 (s, 1H), 8.58 (s, 1H), 8.40 (d, *J* = 5.8 Hz, 1H), 8.33 (s, 1H), 7.85 (d, *J* = 7.2 Hz, 1H), 7.43 (d, *J* = 8.0 Hz, 1H), 7.28-7.23 (m, 1H), 6.44 (d, *J* = 8.0 Hz, 1H), 4.57-4.42 (m, 4H), 3.90 (s, 3H), 3.63-3.54 (m, 5H), 1.30 (d, *J* = 6.8 Hz, 6H);

MS m/z (ESI): 507.2 [M+H]⁺.

### Example 73

### Preparation of N-(2-(3-chloro-1-(2-methoxyethyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

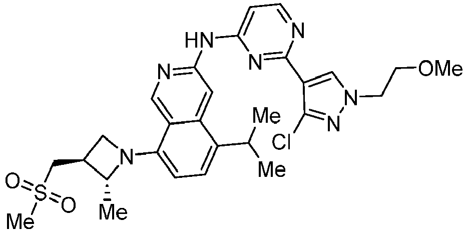

N-(2-(3-chloro-1-(2-methoxyethyl)-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine was prepared with reference to example 11 or 15.

¹H NMR (400 MHz, DMSO-*d*₆) *δ* 10.19 (s, 1H), 9.10 (s, 1H), 8.53 (s, 1H), 8.41 (d, *J* = 5.8 Hz, 1H), 8.33 (s, 1H), 7.43 (d, *J* = 8.0 Hz, 1H), 7.32-7.28 (m, 1H), 6.58 (d, *J* = 8.1 Hz, 1H), 4.68 (t, *J* = 7.4 Hz, 1H), 4.36-4.28 (m, 2H), 4.24-4.18 (m, 1H), 3.73 (t, *J* = 5.2 Hz, 2H), 3.68-3.62 (m, 1H), 3.61-3.50 (m, 3H), 3.25 (s, 3H), 3.00 (s, 3H), 2.94-2.85 (m, 1H), 1.43 (d, *J* = 6.0 Hz, 3H), 1.33-1.27 (m, 6H);

MS m/z (ESI): 584.2 [M+H]⁺.

### Example 74

### Preparation of 3-((2R,3S)-1-(3-((2-(3-chloro-1-methyl-1H-pyrazol-4-yl)pyrimidin-4-yl)amino)-5-isopropylisoquinolin-8-yl)-2-methylazetidin-3-yl)oxazolidin-2-one

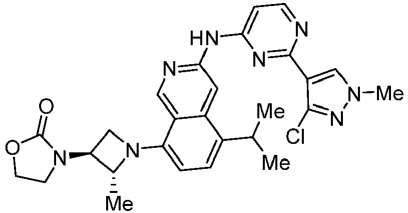

3-((2R,3S)-1-(3-((2-(3-chloro-1-methyl-1H-pyrazol-4-yl)pyrimidin-4-yl)amino)-5-isopropylisoquinolin-8-yl)-2-methyl azetidin-3-yl)oxazolidin-2-one was prepared with reference to example 15.

¹H NMR (400 MHz, DMSO-*d*₆) δ 10.26 (s, 1H), 9.08 (s, 1H), 8.52 (s, 1H), 8.35 (d, *J* = 6.0 Hz, 1H), 8.28 (s, 1H), 7.40 (d, *J* = 8.0 Hz, 1H), 7.20 (d, *J* = 5.2 Hz, 1H), 6.60 (d, *J* = 8.1 Hz, 1H), 4.58 (t, *J* = 7.5 Hz, 1H), 4.40-4.36 (m, 1H), 4.30-4.18 (m, 2H), 3.84 (s, 3H), 3.79 (t, *J* = 7.2 Hz, 1H), 3.68-3.58 (m, 2H), 3.55-3.48 (m, 2H), 1.34 (d, *J* = 6.0Hz, 3H), 1.24(t, *J* = 6.0Hz ,6H);

MS m/z (ESI): 533.2 [M+H]⁺.

### Example 75

### Preparation of N-(2-(3-chloro-1-methyl-1H-pyrazol-4-yl)pyrimidin-4-yl)-5-isopropyl-8-(3-(nitromethyl)azetidin-1-yl)isoquinolin-3-amine

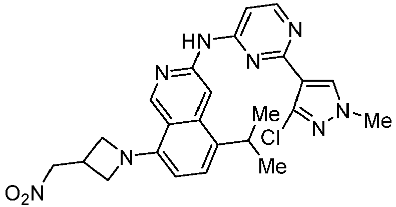

N-(2-(3-chloro-1-methyl-1H-pyrazol-4-yl)pyrimidin-4-yl)-5-isopropyl-8-(3-(nitromethyl)azetidin-1-yl)isoquinolin-3-amine was prepared with reference to example 15.

MS m/z (ESI): 493.2 [M+H]⁺.

### Example 76

### Preparation of N-(2-(3-chloro-1-methyl-1H-pyrazol-4-yl)pyrimidin-4-yl)-5-isopropyl-8-(3-((methylsulfinyl<sulfenyl>)methyl)azetidin-1-yl)isoquinolin-3-amine

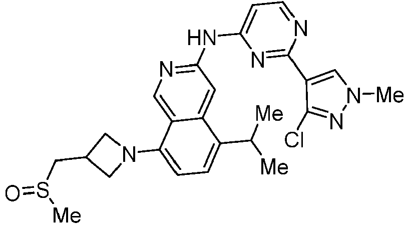

N-(2-(3-chloro-1-methyl-1H-pyrazol-4-yl)pyrimidin-4-yl)-5-isopropyl-8-(3-((methylsulfinyl<sulfenyl>)methyl)azetidin-1-yl)isoquinolin-3-amine was prepared with reference to example 15.

MS m/z (ESI): 510.2 [M+H]⁺.

### Example 77

### Preparation of N-(2-(5-fluoro-1,3-dimethyl-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

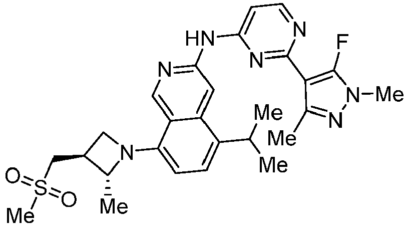

N-(2-(5-fluoro-1,3-dimethyl-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine was prepared with reference to example 15.

¹H NMR (400 MHz, DMSO-*d*₆) *δ* 10.12 (s, 1H), 9.09 (s, 1H), 8.50 (s, 1H), 8.39 (d, *J* = 5.9 Hz, 1H), 7.44 (d, *J* = 8.0 Hz, 1H), 7.26-7.18 (m, 1H), 6.58 (d, *J* = 8.1 Hz, 1H), 4.67 (t, *J* = 7.6 Hz, 1H), 4.20 (t, *J* = 6.2 Hz, 1H), 3.71 (s, 3H), 3.65 (t, *J* = 7.1 Hz, 1H), 3.58-3.49 (m, 3H), 3.00 (s, 3H), 2.93-2.85 (m, 1H), 2.45 (s, 3H), 1.43 (d, *J* = 6.0 Hz, 3H), 1.29 (t, *J* = 6.1 Hz, 6H);

MS m/z (ESI): 538.2 [M+H]⁺.

### Example 78

### Preparation of N-(2-(3-chloro-1-(tetrahydro-2H-pyran-4-yl)-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine

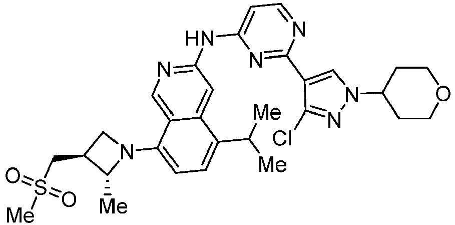

N-(2-(3-chloro-1-(tetrahydro-2H-pyran-4-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to example 11 or 15.

¹H NMR (400 MHz, DMSO-*d*₆) *δ* 10.19 (s, 1H), 9.10 (s, 1H), 8.52 (s, 1H), 8.42-8.40 (m, 2H), 7.43 (d, *J* = 7.2 Hz, 1H), 7.28 (br s, 1H), 6.59 (d, *J* = 7.2 Hz, 1H), 4.71-4.67 (m, 1H), 4.48-4.44 (m, 1H), 4.23-4.20 (m, 1H), 4.02-3.99 (m, 2H), 3.65-3.43 (m, 6H), 3.00 (s, 3H), 2.90-2.87 (m, 1H), 2.01-1.96 (m, 4H), 1.43 (d, *J* = 6.4 Hz, 3H), 1.39-1.36 (m, 6H);

MS m/z (ESI): 610.2 [M+H]⁺.

### Example 79

### Preparation of N-(1-(3-((2-(3-chloro-1-(2-hydroxyethyl)-1H-pyrazol-4-yl)pyrimidin-4-yl)amino)-5-isopropylisoquinolin-8-yl)azetidin-3-yl)-N-methyl methanesulfonamide

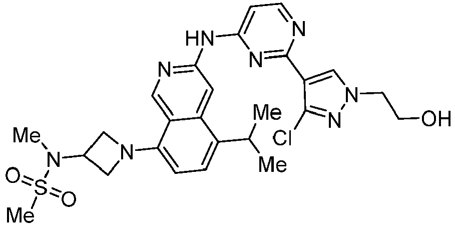

N-(1-(3-((2-(3-chloro-1-(2-hydroxyethyl)-1H-pyrazol-4-yl)pyrimidin-4-yl)amino)-5-isopropylisoquinolin-8-yl)azetidin-3-yl)-N-methyl methanesulfonamide was prepared with reference to example 11 or 15.

¹H NMR (400 MHz, DMSO-*d*₆) *δ* 10.15 (s, 1H), 9.13 (s, 1H), 8.50 (s, 1H), 8.41 (d, *J* = 5.8 Hz, 1H), 8.32 (s, 1H), 7.44 (d, *J* = 8.0 Hz, 1H), 7.34 (s, 1H), 6.47 (d, *J* = 7.6 Hz, 1H), 5.06-4.99 (m, 1H), 4.72-4.62 (m, 1H), 4.47-4.36 (m, 2H), 4.28-4.15 (m, 4H), 3.84-3.75 (m, 2H), 3.60-3.53 (m, 1H), 2.99-2.91 (m, 6H), 1.31 (d, *J* = 6.6 Hz, 6H);

MS m/z (ESI): 571.2 [M+H]⁺.

### Example 80

### Preparation of 1-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)propan-2-ol

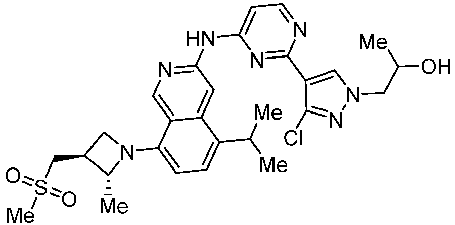

1-(3-Chloro-4-(4-((5-isopropyl-8-((2R,3 S)-2-methyl-3-((methanesulfonyl) methyl)azetidin-1-yl)isoquinolin-3-yl)amino)pyrimidin-2-yl)-1H-pyrazol-1-yl) propan-2-ol was prepared with reference to example 11 or 15.

¹H NMR (400 MHz, DMSO-*d*₆) *δ* 10.19 (s, 1H), 9.10 (s, 1H), 8.53 (s, 1H), 8.41 (d, *J* = 5.8 Hz, 1H), 8.30 (s, 1H), 7.43 (d, *J* = 8.0 Hz, 1H), 7.28 (s, 1H), 6.58 (d, *J* = 8.2 Hz, 1H), 5.10-5.03 (m, 1H), 4.68 (t, *J* = 7.6 Hz, 1H), 4.24-4.17 (t, *J* = 6.4 Hz, 1H), 4.15-4.07 (m, 1H), 4.05-3.96 (m, 2H), 3.67-3.62 (m, 1H), 3.60-3.49 (m, 3H), 3.00 (s, 3H), 2.93-2.86 (m, 1H), 1.43 (d, *J* = 6.0 Hz, 3H), 1.36-1.28 (m, 6H), 1.10 (d, *J* = 5.8 Hz, 3H);

MS m/z (ESI): 584.2 [M+H]⁺.

### Example 81

### Preparation of N-(2-(3-chloro-1-(oxetan-3-yl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

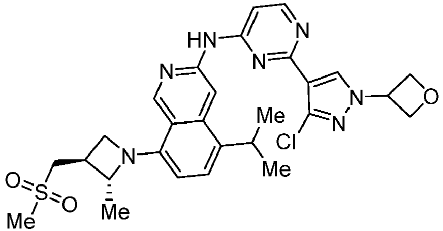

N-(2-(3-chloro-1-(oxetan-3-yl)-1H-pyrazol-4-yl)pyrimidin-4-yl)-5-isopropyl-8-((2R,3 S)-2-methyl-3-((methanesulfonyl)methyl)azetidin-1-yl)isoquinolin-3-amine was prepared with reference to example 11 or 15.

¹H NMR (400 MHz, DMSO-*d*₆) δ 10.13 (s, 1H), 9.03 (s, 1H), 8.45 (s, 1H), 8.41 (s, 1H), 8.35 (d, *J* = 5.8 Hz, 1H), 7.36 (d, *J* = 8.0 Hz, 1H), 7.24 (d, *J* = 5.8 Hz, 1H), 6.51 (d, *J* = 8.0 Hz, 1H), 5.61-5.50 (m, 1H), 4.93-4.82 (m, 4H), 4.61 (t, *J* = 7.6 Hz, 1H), 4.17-4.10 (m, 1H), 3.62-3.39 (m, 4H), 2.93 (s, 3H), 2.87-2.77 (m, 1H), 1.36 (d, *J* = 6.0 Hz, 3H), 1.25-1.14 (m, 6H);

MS m/z (ESI): 582.2 [M+H]⁺.

### Example 82

### Preparation of 1-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-3-fluoropropan-2-ol

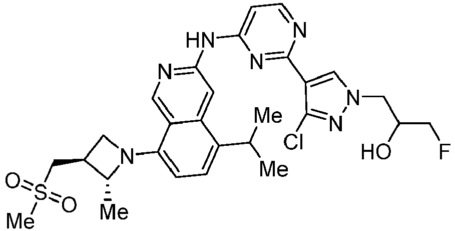

1-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)-3-fluoropropan-2-ol was prepared with reference to example 11.

¹H NMR (400 MHz, DMSO-*d*₆) *δ* 10.20 (s, 1H), 9.10 (s, 1H), 8.53 (s, 1H), 8.42 (d, *J* = 5.8 Hz, 1H), 8.32 (s, 1H), 7.44 (d, *J* = 8.0 Hz, 1H), 7.29 (s, 1H), 6.58 (d, *J* = 7.8 Hz, 1H), 5.62 (s, 1H), 4.67 (d, *J* = 7.6 Hz, 1H), 4.49-4.11 (m, 6H), 3.69-3.63 (m, 1H), 3.60-3.51 (m, 3H), 3.00 (s, 3H), 2.92-2.87 (m, 1H), 1.43 (d, *J=* 6.0 Hz, 3H), 1.35-1.27 (m, 6H);

MS m/z (ESI): 602.2 [M+H]⁺.

### Example 83

### Preparation of N-(((1-(3-((2-(1,3-dimethyl-1H-pyrazol-4-yl)pyrimidin-4-yl)amino)-5-isopropylisoquinolin-8-yl)azetidin-3-yl)methyl)(methyl)(carbonyl)-l6-sulfanylidene)cyanamide

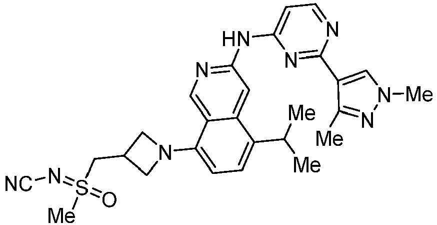

N-(((1-(3-((2-(1,3-dimethyl-1H-pyrazol-4-yl)pyrimidin-4-yl)amino)-5-isopropylisoquinolin-8-yl)azetidin-3-yl)methyl)(methyl)(carbonyl)-l6-sulfanylidene) cyanamide was prepared with reference to example 15.

¹H NMR (400 MHz, DMSO-*d*₆) *δ* 10.07 (s, 1H), 9.09 (s, 1H), 8.54 (s, 1H), 8.37 (d, *J* = 5.8 Hz, 1H), 8.18 (s, 1H), 7.44 (d, *J* = 8.0 Hz, 1H), 7.21 (d, *J* = 5.5 Hz, 1H), 6.44 (d, *J* = 8.1 Hz, 1H), 4.47-4.37 (m, 2H), 4.13 (d, *J* = 7.2 Hz, 2H), 4.06-3.98 (m, 2H), 3.85 (s, 3H), 3.58-3.50 (m, 4H), 3.43-3.38 (m, 1H), 2.49-2.45 (m, 3H), 1.33 (d, *J* = 6.8 Hz, 6H);

MS m/z (ESI): 530.2 [M+H]⁺.

### Example 84

### Preparation of (R)-3-(1-(3-((2-(3-chloro-1-methyl-1H-pyrazol-4-yl)pyrimidin-4-yl)amino)-5-isopropylisoquinolin-8-yl)azetidin-3-yl)-5-methyloxazolidin-2-one

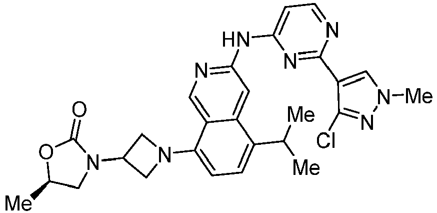

(R)-3-(1-(3-((2-(3-Chloro-1-methyl-1H-pyrazol-4-yl)pyrimidin-4-yl)amino)-5-isopropylisoquinolin-8-yl)azetidin-3-yl)-5-methyloxazolidin-2-one was prepared with reference to example 15.

¹H NMR (400 MHz, DMSO-*d*₆) δ 10.10 (s, 1H), 9.06 (s, 1H), 8.50 (s, 1H), 8.34 (d, *J* = 5.9 Hz, 1H), 8.26 (s, 1H), 7.37 (d, *J* = 8.0 Hz, 1H), 7.21 (d, *J* = 5.7 Hz, 1H), 6.41 (d, *J* = 8.0 Hz, 1H), 4.74-4.57 (m, 2H), 4.32 (dd, *J* = 16.5, 8.2 Hz, 2H), 4.25-4.12 (m, 2H), 3.87 (t, *J* = 8.4 Hz, 1H), 3.84 (s, 3H), 3.54-3.47 (m, 1H), 1.96-1.90 (m, 1H), 1.29 (d, *J* = 6.2 Hz, 3H), 1.23 (d, *J* = 6.8 Hz, 6H);

MS m/z (ESI): 533.2 [M+H]⁺.

### Example 85

### N-(2-(6-(fluoromethyl)-5,6-dihydro-4H-pyrrolo[1,2-b]pyrazol-3-yl)pyrimidin-4-yl)-5-isopropyl-8-((2R,3S)-2-methyl-3-((methanesulfonyl)methyl)azetidin-1-yl)isoquinolin-3-amine (comprising a pair of epimers)

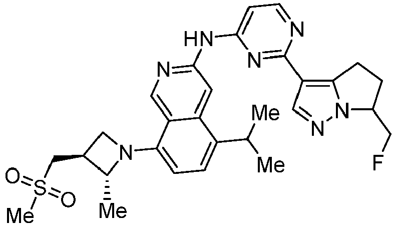

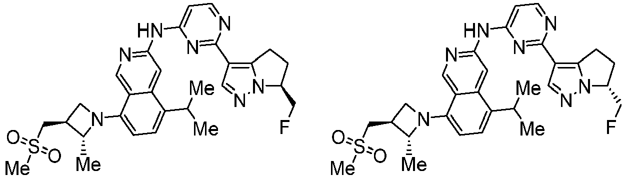

N-(2-(6-(fluoromethyl)-5,6-dihydro-4H-pyrrolo[1,2-b]pyrazol-3-yl)pyrimidin-4-yl)-5-isopropyl-8-((2R,3S)-2-methyl-3-((methanesulfonyl)methyl)azetidin-1-yl) isoquinolin-3-amine was prepared with reference to example 15.

MS m/z (ESI): 564.2 [M+H]⁺.

### Example 86

### N-(2-(6-(difluoromethyl)-5,6-dihydro-4H-pyrrolo[1,2-b]pyrazol-3-yl)pyrimidin-4-yl)-5-isopropyl-8-((2R,3S)-2-methyl-3-((methanesulfonyl)methyl)azetidin-1-yl)isoquinolin-3-amine (comprising a pair of epimers)

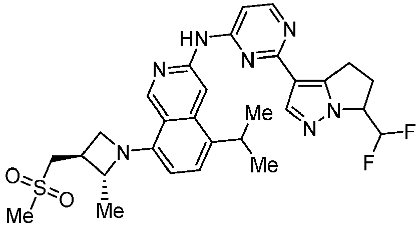

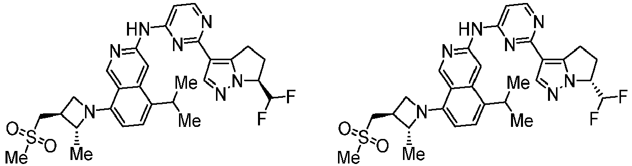

N-(2-(6-(difluoromethyl)-5,6-dihydro-4H-pyrrolo[1,2-b]pyrazol-3-yl)pyrimidin-4-yl)-5-isopropyl-8-((2R,3S)-2-methyl-3-((methanesulfonyl)methyl)azetidin-1-yl)isoquinolin-3-amine was prepared with reference to example 15.

MS m/z (ESI): 582.2 [M+H]⁺.

### Example 87

### 5-isopropyl-8-((2R,3S)-2-methyl-3-((methanesulfonyl)methyl)azetidin-1-yl)-N-(2-(6-(trifluoromethyl)-5,6-dihydro-4H-pyrrolo[1,2-b]pyrazol-3-yl)pyrimidin-4-yl)isoquinolin-3-amine (comprising a pair of epimers)

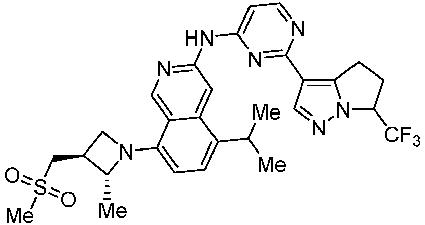

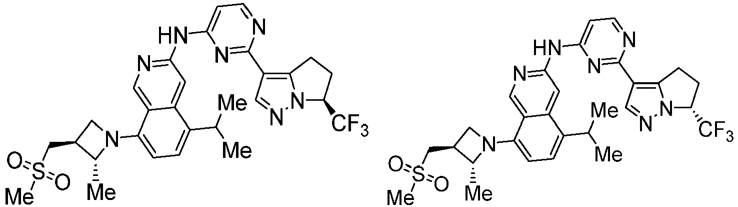

5-Isopropyl-8-((2R,3S)-2-methyl-3-((methanesulfonyl)methyl)azetidin-1-yl)-N-(2-(6-(trifluoromethyl)-5,6-dihydro-4H-pyrrolo[1,2-b]pyrazol-3-yl)pyrimidin-4-yl)isoquinolin-3-amine was prepared with reference to example 15.

MS m/z (ESI): 600.2 [M+H]⁺.

### Example 88

### 5-isopropyl-N-(2-(6-(methoxymethyl)-5,6-dihydro-4H-pyrrolo[1,2-b]pyrazol-3-yl)pyrimidin-4-yl)-8-((2R,3S)-2-methyl-3-((methanesulfonyl)methyl)azetidin-1-yl)isoquinolin-3-amine (comprising a pair of epimers)

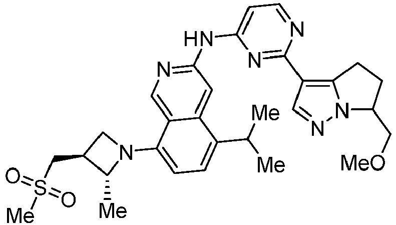

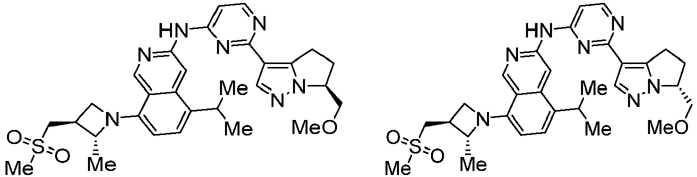

5-Isopropyl-N-(2-(6-(methoxymethyl)-5,6-dihydro-4H-pyrrolo[1,2-b]pyrazol-3-yl)pyrimidin-4-yl)-8-((2R,3S)-2-methyl-3-((methanesulfonyl)methyl)azetidin-1-yl)isoquinolin-3-amine was prepared with reference to example 15.

MS m/z (ESI): 576.2 [M+H]⁺.

### Example 89

Preparation of N-(2-(1-(cyclopropylsulfonyl)-1H-pyrazol-4-yl)pyridin-4-yl)-5-isopropyl-8-((2R,3S)-2-methyl-3-((methanesulfonyl)methyl)azetidin-1-yl)isoquinolin-3-amine 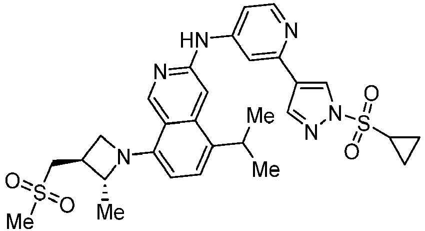 N-(2-(1-(cyclopropylsulfonyl)-1H-pyrazol-4-yl)pyridin-4-yl)-5-isopropyl-8-((2R,3S)-2-methyl-3-((methanesulfonyl)methyl)azetidin-1-yl)isoquinolin-3-amine was prepared with reference to example 1 or 15.

MS m/z (ESI): 595.2 [M+H]⁺.

### Example 90

### Preparation of 2-(1-(3-((2-(1-(cyclopropylsulfonyl)-1H-pyrazol-4-yl)pyridin-4-yl)amino)-5-isopropylisoquinolin-8-yl)azetidin-3-yl)acetonitrile

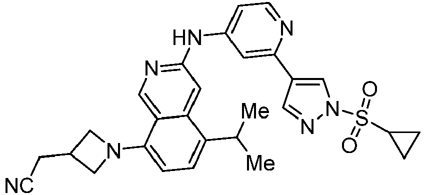

2-(1-(3-((2-(1-(Cyclopropylsulfonyl)-1H-pyrazol-4-yl)pyridin-4-yl)amino)-5-isopropylisoquinolin-8-yl)azetidin-3-yl)acetonitrile was prepared with reference to example 1 or 15.

MS m/z (ESI): 528.2 [M+H]⁺.

### Example 91

### Preparation of N-(2-(1-cyclopropyl-1H-pyrazol-4-yl)pyridin-4-yl)-5-isopropyl-8-((2R,3S)-2-methyl-3-((methanesulfonyl)methyl)azetidin-1-yl)isoquinolin-3-amine

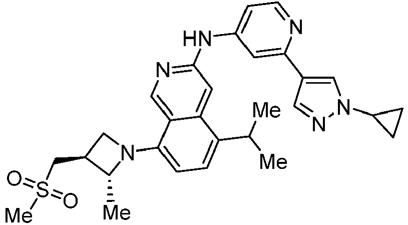

N-(2-(1-cyclopropyl-1H-pyrazol-4-yl)pyridin-4-yl)-5-isopropyl-8-((2R,3 S)-2-methyl-3-((methanesulfonyl)methyl)azetidin-1-yl)isoquinolin-3-amine was prepared with reference to example 15.

MS m/z (ESI): 531.2 [M+H]⁺.

### Example 92

### Preparation of 5-isopropyl-N-(2-(1-methyl-1H-pyrazol-4-yl)pyridin-4-yl)-8-((2R,3S)-2-methyl-3-((methanesulfonyl)methyl)azetidin-1-yl)isoquinolin-3-amine

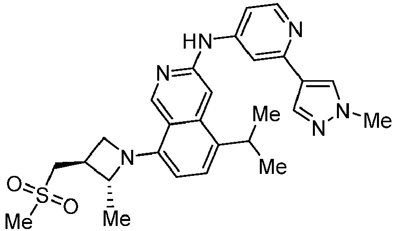

5-Isopropyl-N-(2-(1-methyl-1H-pyrazol-4-yl)pyridin-4-yl)-8-((2R,3 S)-2-methyl-3-((methanesulfonyl)methyl)azetidin-1-yl)isoquinolin-3-amine was prepared with reference to example 15.

MS m/z (ESI): 505.2 [M+H]⁺.

### Example 93

### Preparation of 2-(1-(5-isopropyl-3-((2-(1-methyl-1H-pyrazol-4-yl)pyridin-4-yl)amino)isoquinolin-8-yl)azetidin-3-yl)acetonitrile

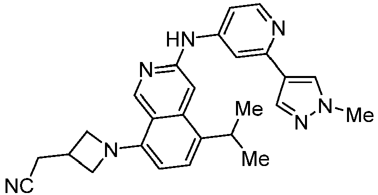

2-(1-(5-Isopropyl-3-((2-(1-methyl-1H-pyrazol-4-yl)pyridin-4-yl)amino) isoquinolin-8-yl)azetidin-3-yl)acetonitrile was prepared with reference to example 15.

MS m/z (ESI): 438.2 [M+H]⁺.

### Example 94

### Preparation of 2-((2R,3S)-1-(5-isopropyl-3-((2-(1-methyl-1H-pyrazol-4-yl)pyridin-4-yl)amino)isoquinolin-8-yl)-2-methylazetidin-3-yl)acetonitrile

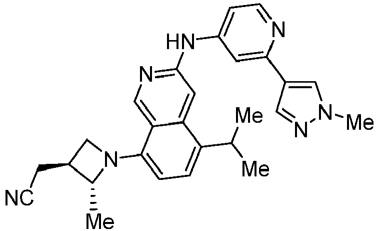

2-((2R,3 S)-1-(5-Isopropyl-3-((2-(1-methyl-1H-pyrazol-4-yl)pyridin-4-yl)amino)isoquinolin-8-yl)-2-methylazetidin-3-yl)acetonitrile was prepared with reference to example 15.

MS m/z (ESI): 452.2 [M+H]⁺.

### Example 95

### Preparation of N-(2-(3-chloro-1-(2,2-difluorocyclobutyl)-1H-pyrazol-4-yl)pyrimidin-4-yl)-5-isopropyl-8-((2R,3 S)-2-methyl-3-((methanesulfonyl)methyl)azetidin-1-yl)isoquinolin-3 -amine

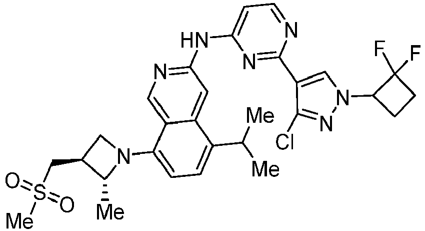

N-(2-(3-chloro-1-(2,2-difluorocyclobutyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to example 11.

MS m/z (ESI): 616.2 [M+H]⁺.

### Example 96

### Preparation of 4-(3-chloro-4-(4-((5-isopropyl-8-((2R,3 S)-2-methyl-3-((methanesulfonyl)methyl)azetidin-1-yl)isoquinolin-3-yl)amino)pyrimidin-2-yl)-1H-pyrazol-1 -yl)cyclohexan-1 -one

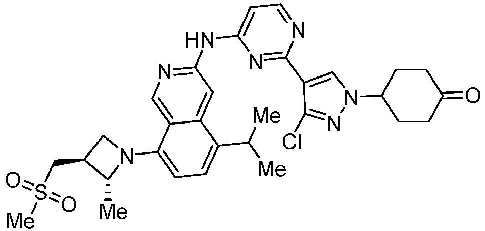

4-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)cyclohexan-1-one was prepared with reference to example 11.

MS m/z (ESI): 622.2 [M+H]⁺.

### Example 97

### Preparation of 2-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-1-(fluoromethyl)cyclobutan-1-ol

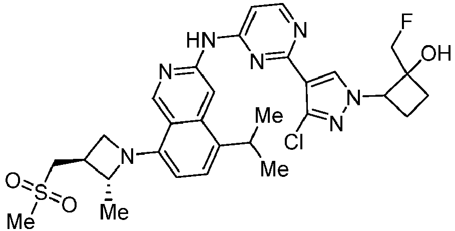

2-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)-1-(fluoromethyl)cyclobutan-1-ol was prepared with reference to example 11.

MS m/z (ESI): 628.2 [M+H]⁺.

### Example 98

### Preparation of N-(2-(3-chloro-1-(2-methoxy -2-methylcyclobutyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine

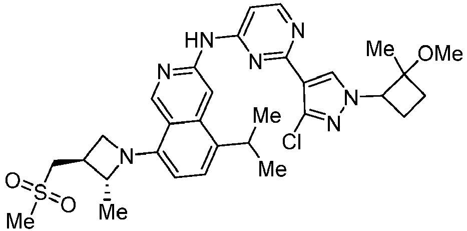

N-(2-(3-chloro-1-(2-methoxy-2-methylcyclobutyl)-1H-pyrazol-4-yl)pyrimidin-4-yl)-5-isopropyl-8-((2R,3S)-2-methyl-3-((methanesulfonyl)methyl)azetidin-1-yl)isoquinolin-3-amine was prepared with reference to example 11.

MS m/z (ESI): 624.2 [M+H]⁺.

### Example 99

### Preparation of 2-(3-chloro-4-(4-((5-isopropyl-8-((2R,3S)-2-methyl-3-((methanesulfonyl)methyl)azetidin-1-yl)isoquinolin-3-yl)amino)pyrimidin-2-yl)-1H-pyrazol-1-yl)-1-methylcyclobutane-1-carbonitrile

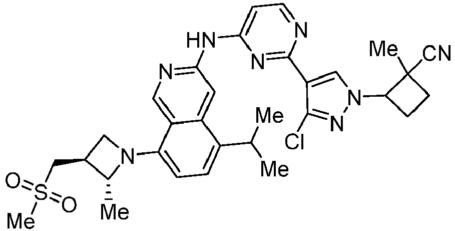

2-(3-Chloro-4-(4-((5-isopropyl-8-((2R,3S)-2-methyl-3-((methanesulfonyl) methyl)azetidin-1-yl)isoquinolin-3-yl)amino)pyrimidin-2-yl)-1H-pyrazol-1-yl)-1-methylcyclobutane-1-carbonitrile was prepared with reference to example 11.

MS m/z (ESI): 619.2 [M+H]⁺.

### Example 100

### Preparation of N-((2R,3S)-1-(3-((2-(3-chloro-1-(2-hydroxyethyl)-1H-pyrazol-4-yl)pyrimidin-4-yl)amino)-5-isopropylisoquinolin-8-yl)-2-methylazetidin-3-yl)-N-methyl methanesulfonamide

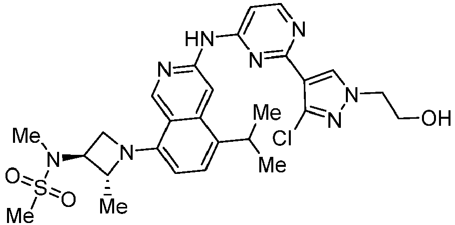

N-((2R,3S)-1-(3-((2-(3-chloro-1-(2-hydroxyethyl)-1H-pyrazol-4-yl)pyrimidin-4-yl)amino)-5-isopropylisoquinolin-8-yl)-2-methylazetidin-3-yl)-N-methyl methanesulfonamide was prepared with reference to example 15.

MS m/z (ESI): 585.2 [M+H]⁺.

### Example 101

### Preparation of N-((2R,3S)-1-(3-((2-(3-chloro-1-(2-hydroxyethyl)-1H-pyrazol-4-yl)pyrimidin-4-yl)amino)-5-isopropylisoquinolin-8-yl)-2-methylazetidin-3-yl)methanesulfonamide

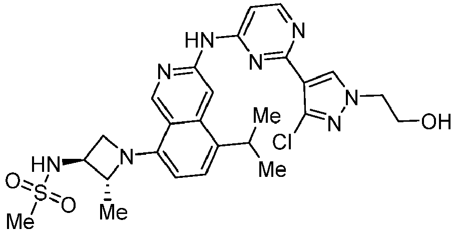

N-((2R,3S)-1-(3-((2-(3-chloro-1-(2-hydroxyethyl)-1H-pyrazo1-4-yl)pyrimidin-4-yl)amino)-5-isopropylisoquinolin-8-yl)-2-methylazetidin-3-yl)methanesulfonamide was prepared with reference to example 15.

MS m/z (ESI): 571.2 [M+H]⁺.

### Example 102

### Preparation of N-((2R,3S)-1-(3 -((2-(3-chloro-1-(2-hydroxyethyl)-1H-pyrazol-4-yl)pyrimidin-4-yl)amino)-5-(1-methoxyethyl)isoquinolin-8-yl)-2-methylazetidin-3-yl)-N-methyl methanesulfonamide

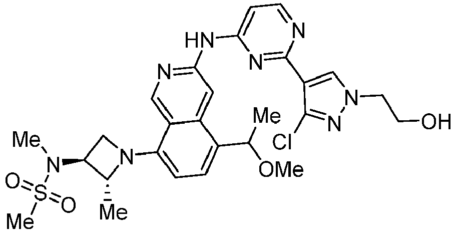

N-((2R,3S)-1-(3-((2-(3-chloro-1-(2-hydroxyethyl)-1H-pyrazol-4-yl)pyrimidin-4-yl)amino)-5-(1-methoxyethyl)isoquinolin-8-yl)-2-methylazetidin-3-yl)-N-methyl methanesulfonamide was prepared with reference to example 15.

MS m/z (ESI): 601.2 [M+H]⁺.

### Example 103

### Preparation of N-((2R,3S)-1-(3-((2-(3-chloro-1-(2-hydroxyethyl)-1H-pyrazol-4-yl)pyrimidin-4-yl)amino)-5-(dimethylamino)isoquinolin-8-yl)-2-methylazetidin-3-yl)-N-methyl methanesulfonamide

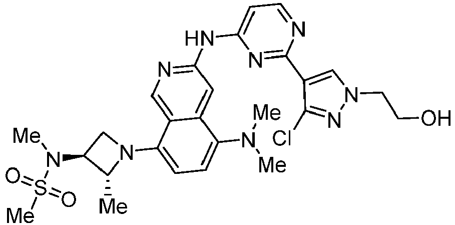

N-((2R,3S)-1-(3-((2-(3-chloro-1-(2-hydroxyethyl)-1H-pyrazol-4-yl)pyrimidin-4-yl)amino)-5-(dimethylamino)isoquinolin-8-yl)-2-methylazetidin-3-yl)-N-methyl methanesulfonamide was prepared with reference to example 15.

MS m/z (ESI): 586.2 [M+H]⁺.

### Biological test evaluation

The present invention will be further described and explained below in conjunction with test examples, but these examples are not meant to limit the scope of the present invention.

### Test example 1. Determination of the inhibitory effect of the compounds of the present invention on the activity of EGFR del19/T790M/C797S and EGFR L858R/T790M/C797S mutant kinases

Experimental objective: The objective of this test example is to test the inhibitory activity of the compounds on the activity of EGFR del19/T790M/C797S and EGFR L858R/T790M/C797S mutant kinases.

Experimental instruments: The centrifuge (5810R) was purchased from Eppendorf, the pipette was purchased from Eppendorf or Rainin, and the microplate reader was purchased from BioTek (United States) with a model of SynergyH1 multifunctional microplate reader.

Experimental method: This experiment used Cisbio's HTRF kinase assay method (Cisbio#62TK0PEB), wherein a catalytic reaction occurred between the substrate polypeptide TK and ATP in the presence of the tyrosine kinase with EGFR del19/T790M/C797S or EGFR L858R/T790M/C797S mutation, the substance was phosphorylated, and the activity of the kinase was characterized by measuring the content of the phosphorylated substrate generated during the reaction, and the half inhibitory concentration IC₅₀ of the compound on the activity of EGFR del19/T790M/C797S or EGFR L858R/T790M/C797S mutant kinase was obtained. Specific experimental operations were as follows:
the kinase reaction was carried out in a white 384-well plate (Perkin Elmer#6008280), and 1-5 µL of compounds of different concentrations which were diluted with 1% DMSO-containing ddH₂O were added; to the positive control wells were added 1-5 µL of 1% DMSO-containing ddH₂O, followed by 1-5 µL of 0.5-5 nM 4×EGFR del19/T790M/C797S or EGFR L858R/T790M/C797S mutant kinase solution which was diluted with Dilution buffer (5×kinase buffer, MgCl₂ 6.65 Mm, MnCl₂ 1.33 mM, DTT 1.33 mM); to the negative control wells were added 1-5 µL of Dilution buffer; to all the wells were added 1-5 µL of 4 µM 4× substrate TK solution which was prepared with 10× Dilution buffer, and finally added 1-5 µL, of 24 µM 4× ATP solution which was diluted with Dilution buffer to start the reaction; after reacting at room temperature for 120 minutes, 10 µL of detection solution (TK antibody 16 nM, XI,665 0.5 µM) was added to each well, same was reacted at room temperature in the dark for 20 minutes, and then the chemiluminescence value was detected using a BioTek Synergy H1 microplate reader.

### Experimental data processing method:

The percent inhibition data for compound-treated wells was calculated according to the positive control wells (DMSO control wells) and negative control wells (no kinase added) on the plate {% inhibition rate = 100-[(test compound value-negative control value)] / (positive control value - negative control value) × 100}. GraphPad prism was used to fit different concentrations and corresponding percent inhibition rate data to a 4-parameter nonlinear logistic equation to calculate IC₅₀ values. The specific data is shown in Table 1 and Table 2 below.

**Table 1**

| Example number | EGFR (L858R/T790M/C797S) |
|---|---|
| | IC₅₀ (nM) |
| 1 | 0.51 |
| 14 | 0.73 |
| 15 | 1.90 |
| 17 | 0.71 |
| 19 | 1.00 |
| 20 | 0.92 |
| 21 | 0.60 |
| 24 | 0.30 |
| 26 | 1.00 |
| 27 | 1.90 |
| 66 | 0.17 |

**Table 2**

| Example number | EGFR (del19/T790M/C797S) |
|---|---|
| | IC₅₀ (nM) |
| 1 | 0.58 |
| 3 | 9.40 |
| 14 | 3.60 |
| 15 | 2.50 |
| 17 | 3.00 |
| 18 | 9.10 |
| 19 | 2.50 |
| 20 | 6.20 |
| 21 | 3.90 |
| 24 | 3.60 |
| 27 | 8.90 |
| 48 | 4.20 |
| 54 | 7.50 |

The above data shows: The compounds in this example of the present invention have good inhibitory effects on EGFR L858R/T790M/C797S and EGFR del19/T790M/C797S drug-resistant mutant kinases.

### Test example 2: Cell proliferation inhibition assay

Experimental objective: The objective of this test example is to test the inhibitory activity of compounds on the proliferation of different EGFR C797S drug-resistant mutant cell lines Ba/F3 EGFR Del19/T790M/C797S, Ba/F3 EGFR L858R/T790M/C797S, and Ba/F3 EGFR Del19/C797S.

### Experimental instruments:

centrifuge (Eppendorf 5810R)
microplate reader (BioTek Synergy H1)
pipette (Eppendorf or Rainin)
carbon dioxide incubator (Thermo 311)
cell counter (Life Countess II)

### Experimental reagents and consumables:

Ba/F3 EGFR Del19/T790M/C797S cells were purchased from KYinno Biotechnology Co., Ltd.;
Ba/F3 EGFR Del19/C797S cells were purchased from CoBioer Biosciences Co., Ltd.;
Ba/F3 EGFR L858R/T790M/C797S cells were purchased from CoBioer Biosciences Co., Ltd.;
Cell Titer-Glo was purchased from Promega, with a catalog number of G7573;
RPMI 1640 was purchased from Gibco, with a catalog number of 22400089;
FBS was purchased from Gibco, with a catalog number of 10091148;
cell culture plates were purchased from Corning, with a catalog number of 3610.

### Experimental method:

The inhibitory activity of the compound on the proliferation of different EGFR C797S drug-resistant mutant cell lines was detected using the Cell Titer-Glo method. The different cell lines were cultured in RPMI 1640 complete culture medium containing 10% FBS under conditions of 37°C and 5% CO₂, the cells were collected by centrifugation when growing to a certain density, and adjusted to appropriate cell density after counting, the cells were spread on a white 96-well plate at 90 µL/well, and cultured in a 37°C, 5% CO₂ incubator overnight, prepared compound solutions of different concentrations were added at 10 µL/well, corresponding solvent controls were set, and all wells were cultured in a 37°C, 5% CO₂ incubator for 72 hours, 50 µL of CellTiter-Glo solution was added to each well, shook and mixed evenly, and incubated in the dark for 10 minutes, and a BioTek Synergy H1 microplate reader was used for reading.

### Experimental data processing method:

The luminescence signal value was used to calculate the inhibition rate. Graphpad Prism software was used to fit the concentration and inhibition rate to a nonlinear regression curve, so as to obtain the IC₅₀ value, as shown in Tables 3 and 4 below for details.

**Table 3**

| Example | Ba/F3 EGFR Del19_T790M_C797S IC₅₀ (nM) |
|---|---|
| 1 | 10.0 |
| 3 | 5.5 |
| 11 | 7.2 |
| 24 | 10.0 |
| 25 | 14.0 |
| 61 | 9.1 |
| 70 | 11.0 |
| 73 | 11.0 |
| 74 | 14.0 |
| 78 | 12.0 |
| 79 | 7.7 |
| 80 | 10.0 |

**Table 4**

| Example | Ba/F3 EGFR Del19_C797S IC₅₀ (nM) |
|---|---|
| 1 | 14 |
| 3 | 10 |
| 10 | 60 |
| 11 | 24 |
| 12 | 56 |
| 13 | 82 |
| 14 | 54 |
| 15 | 41 |
| 21 | 96 |
| 22A | 95 |
| 24 | 29 |
| 25 | 54 |
| 26 | 78 |
| 27 | 40 |
| 29 | 64 |
| 39 | 73 |
| 40 | 70 |
| 48 | 85 |
| 49 | 41 |
| 50 | 51 |
| 51 | 41 |
| 52 | 49 |
| 53 | 49 |
| 54 | 32 |
| 55 | 25 |
| 56 | 10 |
| 57 | 26 |
| 58 | 52 |
| 59 | 62 |
| 60 | 26 |
| 61 | 24 |
| 62 | 40 |
| 63 | 30 |
| 64 | 62 |
| 65 | 84 |
| 66 | 78 |
| 67 | 20 |
| 68 | 21 |
| 69 | 51 |
| 70 | 10 |
| 71 | 96 |
| 72 | 77 |
| 73 | 8.5 |
| 74 | 14 |
| 75 | 28 |
| 76 | 63 |
| 77 | 12 |
| 78 | 27 |
| 79 | 11 |
| 80 | 17 |
| 81 | 50 |
| 82 | 33 |
| 83 | 74 |
| 84 | 93 |

The above data shows: the compounds of this example all have excellent inhibitory activity on the proliferation of Ba/F3 EGFR Del19/T790M/C797S, Ba/F3 EGFR L858R/T790M/C797S and Ba/F3 EGFR Del19/C797S.

## Claims

1. A compound as represented by general formula (II-A), or a stereoisomer or pharmaceutically acceptable salt thereof: 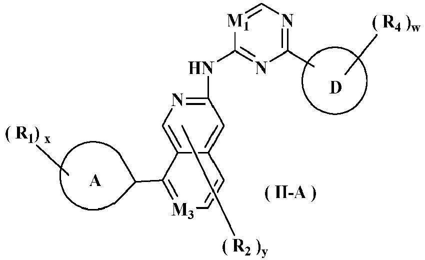 wherein:
M₁ is N or CH;
M₃ is N or CH;
ring D is selected from heteroaryl;
ring A is selected from cycloalkyl, heterocyclyl, aryl or heteroaryl;
R₁ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted; or, two R₁ are connected to the atoms therebetween to form cycloalkyl, heterocyclyl, aryl or heteroaryl, and the cycloalkyl, heterocyclyl, aryl and heteroaryl can be optionally further substituted;
R₂ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted;
R₄ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted;
each Rₐ is independently selected from hydrogen, deuterium, halogen, amino, hydroxyl, cyano, nitro, alkyl, alkenyl, alkynyl, alkoxy, hydroxyalkyl, cycloalkyl, heterocyclyl, aryl or heteroaryl, and the amino, alkyl, alkenyl, alkynyl, alkoxy, hydroxyalkyl, cycloalkyl, heterocyclyl, aryl and heteroaryl can be optionally further substituted;
x is 0, 1, 2, 3, 4, 5 or 6;
y is 0, 1, 2, 3, 4, 5 or 6;
w is 0, 1, 2, 3, 4, 5 or 6;
and p, m and n5 are each independently 0, 1, 2 or 3.

2. The compound or the stereoisomer or pharmaceutically acceptable salt thereof according to claim 1, wherein
ring A is selected from C₃₋₁₂ cycloalkyl, 3- to 12-membered heterocyclyl, C₆₋₁₄ aryl or 5- to 14-membered heteroaryl, the heteroatoms in the 3- to 12-membered heterocyclyl and 5- to 14-membered heteroaryl are independently selected from nitrogen, oxygen, sulfur and phosphorus, and the number of the heteroatoms is independently 1, 2, 3 or 4;
preferably, ring A is 4- to 10-membered heterocyclyl; more preferably, ring A is 4- to 6-membered monocyclic heterocyclyl, 7- to 9-membered spiro heterocyclyl or 8-to 10-membered fused heterocyclyl;
specifically and preferably, ring A is 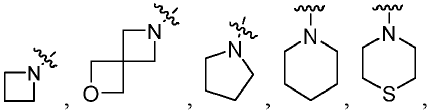 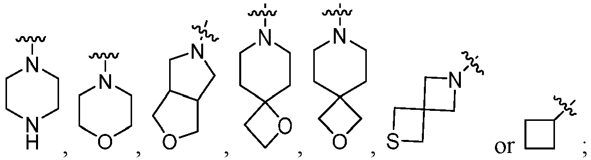
more preferably, ring A is 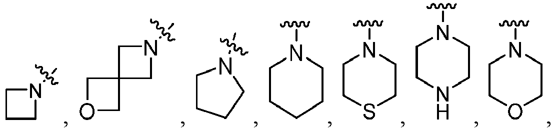 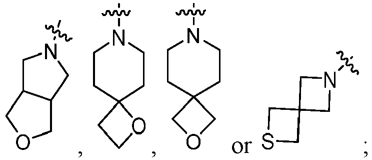
further preferably, ring A is 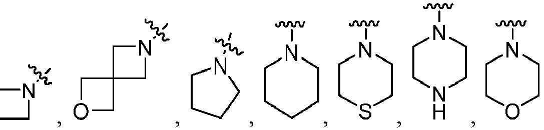 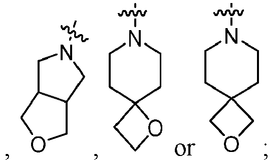
and even further preferably, ring A is

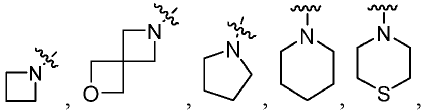

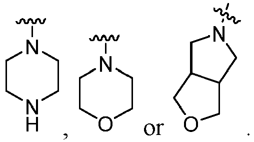

3. The compound or the stereoisomer or pharmaceutically acceptable salt thereof according to claim 1 or 2, wherein
ring D is selected from 5- to 8-membered heteroaryl; more preferably, ring D is 5-membered heteroaryl;
further preferably, ring D is 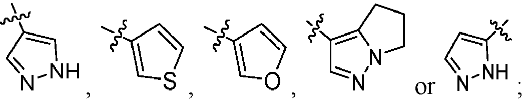
and even further preferably, ring D is

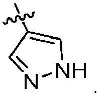

4. A compound as represented by general formula (VII), or a stereoisomer or pharmaceutically acceptable salt thereof: 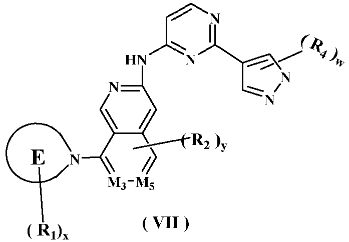 wherein,
M₃ is N or CH;
M₅ is N or CH;
ring E is 4- to 10-membered heterocyclyl; preferably, ring E is 4- to 10-membered heterocyclyl containing 1-4 heteroatoms selected from N, O, S or P;
R₁ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted; or, two R₁ are connected to the atoms therebetween to form cycloalkyl, heterocyclyl, aryl or heteroaryl, and the cycloalkyl, heterocyclyl, aryl and heteroaryl can be optionally further substituted;
R₂ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted;
R₄ is independently selected from hydrogen, deuterium, oxo, thio, halogen, amino, hydroxyl, cyano, nitro, alkyl, alkenyl, alkynyl, alkoxy, hydroxyalkyl, cycloalkyl, heterocyclyl, aryl, heteroaryl, -ORₐ, -P(O)ₚ(Rₐ)ₙ₅, -S(O)ₘRₐ or -C(O)Rₐ, and the amino, alkyl, alkenyl, alkynyl, alkoxy, hydroxyalkyl, cycloalkyl, heterocyclyl, aryl and heteroaryl can be optionally further substituted;
each Rₐ is independently selected from hydrogen, deuterium, halogen, amino, hydroxyl, cyano, nitro, alkyl, alkenyl, alkynyl, alkoxy, hydroxyalkyl, cycloalkyl, heterocyclyl, aryl or heteroaryl, and the amino, alkyl, alkenyl, alkynyl, alkoxy, hydroxyalkyl, cycloalkyl, heterocyclyl, aryl and heteroaryl can be optionally further substituted;
x is 0, 1, 2, 3, 4, 5 or 6;
y is 0, 1, 2, 3, 4, 5 or 6;
w is 0, 1, 2, 3, 4, 5 or 6;
and p, m and n5 are each independently 0, 1, 2 or 3.

5. The compound or the stereoisomer or pharmaceutically acceptable salt thereof according to claim 4, wherein ring E is selected from 4- to 6-membered monocyclic heterocyclyl, 7- to 9-membered spiro heterocyclyl or 8- to 10-membered fused heterocyclyl;
preferably, ring E is 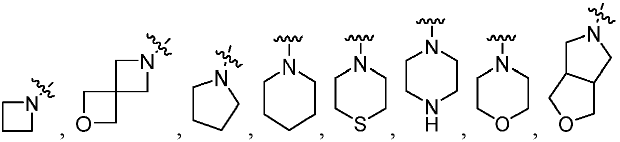 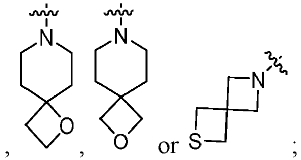
preferably, ring E is 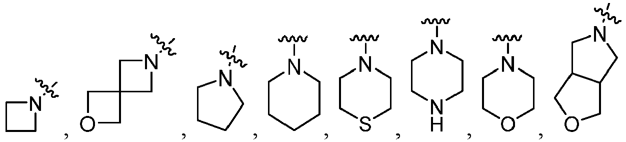 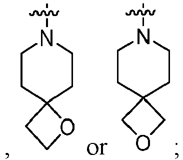
and further preferably, ring E is the group as follows:

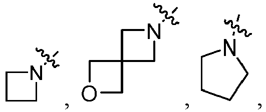

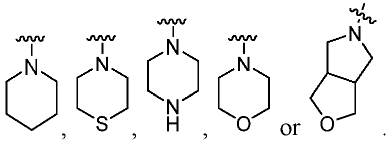

6. The compound or the stereoisomer or pharmaceutically acceptable salt thereof according to claim 4, wherein the compound is further as represented by general formula (VII-1): 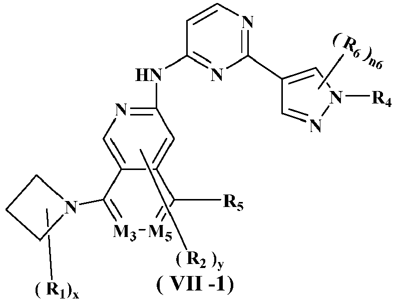 wherein,
R₅ is selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ haloalkoxy or C₁₋₆ hydroxyalkyl;
preferably, R₅ is selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ haloalkoxy or C₁₋₆ hydroxyalkyl;
R₆ is independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ haloalkoxy or C₁₋₆ hydroxyalkyl;
preferably, R₆ is independently selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ haloalkoxy or C₁₋₆ hydroxyalkyl;
and n6 is 0, 1 or 2.

7. The compound as represented by the general formula, or the stereoisomer or pharmaceutically acceptable salt thereof according to any one of claims 1-6, wherein
R₁ is independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or -C(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, - ORₐₐ, -P(O)ₚ(Rₐₐ)ₙ₅, -S(O)ₘRₐₐ, -Se(O)ₘRₐₐ or -C(O)Rₐₐ;
or, two R₁ are connected to the atoms therebetween to form C₃₋₁₂ cycloalkyl, 3- to 12-membered heterocyclyl, C₆₋₁₄ aryl or 5- to 14-membered heteroaryl, and the C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, or C₁₋₆ hydroxyalkyl;
preferably, R₁ is independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or -C(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, -P(O)ₚ(Rₐₐ)ₙ₅, -S(O)ₘRₐₐ or -C(O)Rₐₐ;
or, two R₁ are connected to the atoms therebetween to form C₃₋₁₂ cycloalkyl, 3- to 12-membered heterocyclyl, C₆₋₁₄ aryl and 5- to 14-membered heteroaryl, and the C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, or C₁₋₆ hydroxyalkyl;
more preferably, R₁ is independently selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₈ cycloalkyl, 3- to 10-membered heterocyclyl, C₆₋₁₀ aryl, 5- to 12-membered heteroaryl, -ORₐ, -P(O)ₚ(Rₐ)ₙ₅, -S(O)ₘRₐ or -C(O)Rₐ, and the amino, C₁₋₃ alkyl, C₂₋₃ alkenyl, C₂₋₃ alkynyl, C₁₋₆ alkoxy, C₁₋₆ hydroxyalkyl, C₃₋₈ cycloalkyl, 3- to 10-membered heterocyclyl, C₆₋₁₀ aryl and 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₈ cycloalkyl, 3- to 8-membered heterocyclyl, C₆₋₁₀ aryl, 5- to 10-membered heteroaryl, -ORₐₐ, -P(O)ₚ(Rₐₐ)ₙ₅, -S(O)ₘRₐₐ or -C(O)Rₐₐ;
even further preferably, R₁ is independently selected from -NHS(O)₂CH₃,-NCH₃S(O)₂CH₃, -CH₂S(O)₂N(CH₃)₂, -CH₂Se(O)₂CH₃, -CH₂S(O)₂CH₃, -CH₂SOCH₃,-CH₂NO₂, methyl, hydrogen, 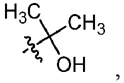 methoxy, cyano, -CH₂OCH₃, -CH₂CN,-CH(CN)₂, -S(O)₂CH₃, oxo, hydroxyl, - CH₂COCH₃, -COCH₃, -CH₂P(O)(CH₃)₂, 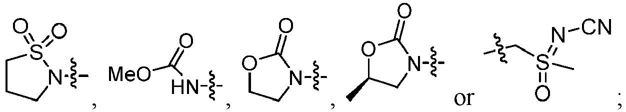
each Rₐₐ is independently selected from hydrogen, deuterium, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, and 5-to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl;
preferably, each Rₐₐ is independently selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 8-membered heterocyclyl, C₆₋₁₂ aryl, or 5- to 12-membered heteroaryl, and the amino, C₁₋₃ alkyl, C₂₋₃ alkenyl, C₂₋₃ alkynyl, C₁₋₃ alkoxy, C₁₋₃ hydroxyalkyl, C₃₋₈ cycloalkyl, 3- to 10-membered heterocyclyl, C₆₋₁₂ aryl, 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₈ cycloalkyl, 3- to 8-membered heterocyclyl, C₆₋₁₀ aryl, or 5- to 10-membered heteroaryl.

8. The compound as represented by the general formula, or the stereoisomer or pharmaceutically acceptable salt thereof according to any one of claims 1-7, wherein
R₂ is independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or -C(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, - ORₐₐ, -P(O)ₚ(Rₐₐ)ₙ₅, -S(O)ₘRₐₐ or -C(O)Rₐₐ;
preferably, R₂ is independently selected from hydrogen, deuterium, oxo, thio, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, -ORₐ, -P(O)ₚ(Rₐ)ₙ₅, -S(O)ₘRₐ or -C(O)Rₐ, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl and 5- to 12-membered heteroaryl can be optionally further substituted with one or more of hydrogen, deuterium, oxo, thio, halogen, amino, hydroxyl, cyano, nitro, C₁₋₂ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl, 5- to 10-membered heteroaryl, -ORₐₐ, -P(O)ₚ(Rₐₐ)ₙ₅, -S(O)ₘRₐₐ or -C(O)Rₐₐ;
each Rₐₐ is independently selected from hydrogen, deuterium, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl;
preferably, each Rₐₐ is independently selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₈ cycloalkyl, 3- to 8-membered heterocyclyl, C₆₋₁₂ aryl, or 5- to 12-membered heteroaryl, and the amino, C₁₋₃ alkyl, C₂₋₃ alkenyl, C₂₋₃ alkynyl, C₁₋₃ alkoxy, C₁₋₃ hydroxyalkyl, C₃₋₈ cycloalkyl, 3- to 8-membered heterocyclyl, C₆₋₁₂ aryl, 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₈ cycloalkyl, 3- to 8-membered heterocyclyl, C₆₋₁₀ aryl, or 5- to 10-membered heteroaryl.

9. The compound as represented by the general formula, or the stereoisomer or pharmaceutically acceptable salt thereof according to any one of claims 1-8, wherein
R₄ is independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or -C(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more R₄₋₁;
each R₄₋₁ is independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, -ORₐₐ, - P(O)ₚ(Rₐₐ)ₙ₅, -S(O)ₘRₐₐ or -C(O)Rₐₐ; optionally, R₄₋₁ is substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl, and the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, and C₁₋₆ hydroxyalkyl are optionally substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, or C₁₋₆ hydroxyalkyl;
preferably, R₄ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, -ORₐ, -P(O)ₚ(Rₐ)ₙ₅, -S(O)ₘRₐ or -C(O)Rₐ, and the amino, C₁₋₃ alkyl, C₂₋₄ alkenyl, C₂₋₄ alkynyl, C₁₋₃ alkoxy, C₁₋₃ hydroxyalkyl, C₃₋₁₀ cycloalkyl, 3- to 10-membered heterocyclyl, C₆₋₁₂ aryl and 5- to 12-membered heteroaryl can be optionally further substituted with one or more R₄₋₁;
each R₄₋₁ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl, -ORₐₐ, - P(O)ₚ(Rₐₐ)ₙ₅, -S(O)ₘRₐₐ or -C(O)Rₐₐ; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₂ aryl, or 5- to 12-membered heteroaryl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl;
or R₄ is independently selected from hydrogen, deuterium, oxo, thio, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl, -ORₐ, -P(O)ₚ(Rₐ)ₙ₅, -S(O)ₘRₐ or -C(O)Rₐ, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl and 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, 5- to 12-membered heteroaryl, - ORₐₐ, -P(O)ₚ(Rₐₐ)ₙ₅, -S(O)ₘRₐₐ or -C(O)Rₐₐ;
each Rₐₐ is independently selected from hydrogen, deuterium, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl;
preferably, each Rₐₐ is independently selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₈ cycloalkyl, 3- to 8-membered heterocyclyl, C₆₋₁₂ aryl, or 5- to 12-membered heteroaryl, and the amino, C₁₋₃ alkyl, C₂₋₃ alkenyl, C₂₋₃ alkynyl, C₁₋₃ alkoxy, C₁₋₃ hydroxyalkyl, C₃₋₈ cycloalkyl, 3- to 8-membered heterocyclyl, C₆₋₁₂ aryl, 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₈ cycloalkyl, 3- to 8-membered heterocyclyl, C₆₋₁₀ aryl, or 5- to 10-membered heteroaryl;
and even further preferably, R₄ is independently selected from fluorine, chlorine, cyano, trifluoromethyl, methyl, ethyl, nitro, hydroxyl, methoxy, -OCD₃, hydrogen, 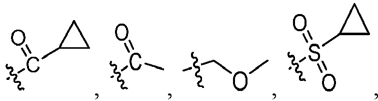 cyclopropyl, 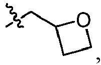 difluoromethyl, 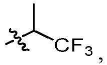 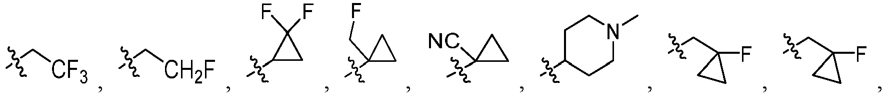 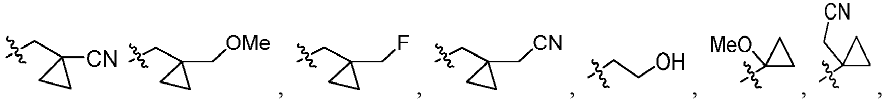 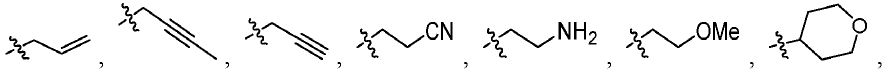 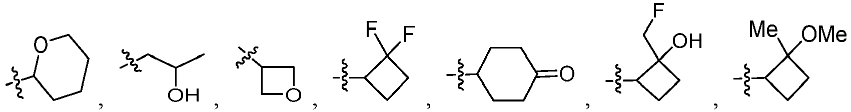 or

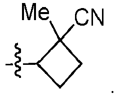

10. The compound as represented by the general formula, or the stereoisomer or pharmaceutically acceptable salt thereof according to any one of claims 1-9, wherein
each Rₐ is independently selected from hydrogen, deuterium, halogen, amino, hydroxyl, cyano, nitro, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ alkoxy, C₁₋₆ hydroxyalkyl, C₃₋₁₂ cycloalkyl, 3- to 12-membered heterocyclyl, C₆₋₁₄ aryl, 5- to 14-membered heteroaryl can be optionally further substituted with one or more of hydrogen, deuterium, oxo, thio, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halo C₁₋₆ alkoxy, C₁₋₆ hydroxyalkyl, C₃₋₁₂ cycloalkyl, 3- to 12-membered heterocyclyl, C₆₋₁₂ aryl, or 5- to 12-membered heteroaryl;
preferably, each Rₐ is independently selected from hydrogen, deuterium, halogen, amino, hydroxyl, cyano, nitro, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, or 5- to 12-membered heteroaryl, and the amino, C₁₋₃ alkyl, C₂₋₃ alkenyl, C₂₋₃ alkynyl, C₁₋₃ alkoxy, C₁₋₃ hydroxyalkyl, C₃₋₁₀ cycloalkyl, 3- to 10-membered heterocyclyl, C₆₋₁₂ aryl, 5- to 12-membered heteroaryl can be optionally further substituted with one or more of hydrogen, deuterium, oxo, thio, halogen, amino, hydroxyl, cyano, nitro, C₁₋₃ alkyl, C₂₋₃ alkenyl, C₂₋₃ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₁₀ cycloalkyl, 3- to 10-membered heterocyclyl, C₆₋₁₀ aryl, or 5- to 10-membered heteroaryl.

11. The compound or the stereoisomer or pharmaceutically acceptable salt thereof according to claim 1, wherein the compound is further as represented by general formula (VII-2): 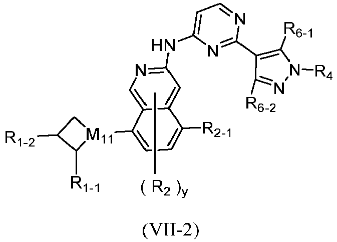 wherein,
M₁₁ is CH or N;
R₁₋₁ is selected from hydrogen, deuterium, fluorine, chlorine, bromine, methyl, ethyl, halomethyl, or haloethyl;
R₁₋₂ is selected from amino, hydroxyl, cyano, C₁₋₃ alkoxy, C₁₋₃ alkyl, C₃₋₆ cycloalkyl, or 3- to 8-membered heterocyclyl, and the amino, C₁₋₃ alkoxy, C₁₋₃ alkyl, C₃₋₆ cycloalkyl, and 3- to 8-membered heterocyclyl can be optionally further substituted with one or more of hydrogen, deuterium, oxo, thio, halogen, amino, hydroxyl, cyano, nitro, C₁₋₃ alkyl, C₁₋₃ deuteroalkyl, C₁₋₃ haloalkyl, C₁₋₃ alkoxy, halo C₁₋₃ alkoxy, C₁₋₃ hydroxyalkyl, -C(O)Rₑₑ, -ORₑₑ, -P(O)ₚ(Rₑₑ)ₙ₅, -S(O)ₘRₑₑ, - S(O)₂N(Rₑₑ)₂ and -S(=O)(=NCN)Rₑₑ; preferably, R₁₋₂ is selected from -NHS(O)₂CH₃, - NCH₃S(O)₂CH₃, -CH₂S(O)₂N(CH₃)₂, -CH₂S(O)₂CH₃, -CH₂SOCH₃, -CH₂NO₂, methyl, hydrogen, 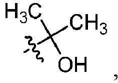 methoxy, cyano, -CH₂OCH₃, -CH₂CN, -CH(CN)₂, hydroxyl, - CH₂COCH₃, 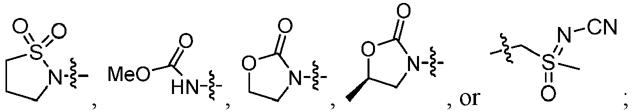
R₂ is independently selected from hydrogen, deuterium, fluorine, chlorine, bromine, methyl, ethyl, halomethyl, or haloethyl;
R₂₋₁ is selected from amino, C₁₋₃ alkyl, halo C₁₋₃ alkyl or deuterated C₁₋₃ alkyl, and the amino, C₁₋₃ alkyl, halo C₁₋₃ alkyl and deuterated C₁₋₃ alkyl can be optionally further substituted with one or more of deuterium, halogen, cyano, hydroxyl, nitro, C₁₋₃ alkyl, halo C₁₋₃ alkyl or deuterated C₁₋₃ alkyl;
R₄ is selected from halogen, amino, hydroxyl, cyano, nitro, C₁₋₃ alkyl, C₁₋₃ alkoxy, C₁₋₃ hydroxyalkyl, C₂₋₄ alkenyl, C₂₋₄ alkynyl, C₃₋₆ cycloalkyl, 3- to 8-membered heterocyclyl, -S(O)ₘR_{d} or -C(O)R_{d}, and the amino, C₁₋₃ alkyl, C₁₋₃ alkoxy, C₁₋₃ hydroxyalkyl, C₂₋₄ alkenyl, C₂₋₄ alkynyl, C₃₋₆ cycloalkyl and 3- to 8-membered heterocyclyl can be optionally further substituted with one or more R₄₋₁;
R₄₋₁ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl or 5- to 12-membered heteroaryl; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl, or 5- to 12-membered heteroaryl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl; preferably, R₄ is selected from fluorine, chlorine, cyano, trifluoromethyl, methyl, ethyl, nitro, hydroxyl, methoxy, -OCD₃, hydrogen, 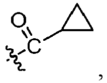 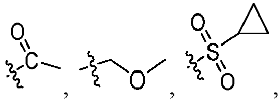 cyclopropyl, 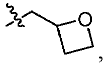 difluoromethyl, 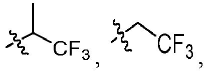 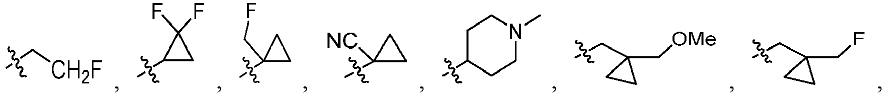 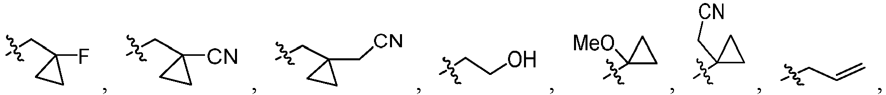 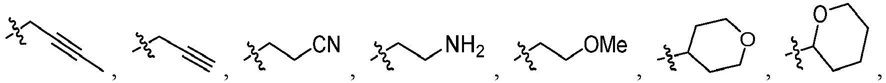 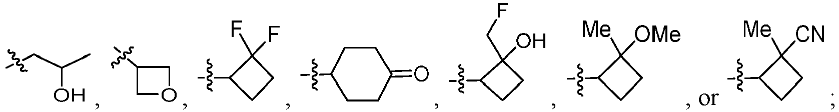
R₆₋₁ is selected from hydrogen, deuterium, oxo, thio, halogen, amino, hydroxyl, cyano, nitro, C₁₋₃ alkyl, C₁₋₃ haloalkyl, C₁₋₃ deuteroalkyl, C₁₋₃ alkoxy, or C₁₋₃ hydroxyalkyl;
R₆₋₂ is selected from hydrogen, deuterium, oxo, thio, halogen, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, -S(O)ₘRₑ or -C(O)Rₑ, and the amino, C₁₋₃ alkyl, C₁₋₃ alkoxy, C₁₋₃ hydroxyalkyl, C₃₋₆ cycloalkyl and 3- to 6-membered heterocyclyl can be optionally further substituted with one or more of deuterium, halogen, amino, hydroxyl, cyano, nitro and C₁₋₃ alkyl;
R_{d} is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
Rₑ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
Rₑₑ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl or C₁₋₃ deuteroalkyl;
m is 0, 1 or 2;
p is 0, 1 or 2;
n5 is 0, 1 or 2;
and y is 0, 1, 2, 3 or 4.

12. The compound as represented by the general formula, or the stereoisomer or pharmaceutically acceptable salt thereof according to claim 11, wherein the compound is further as represented by general formula (VII-3): 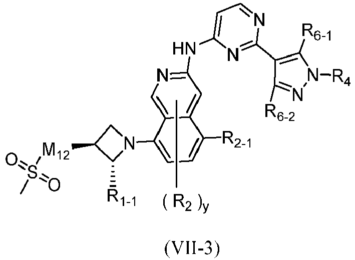 wherein,
M₁₂ is selected from a bond, NR₉ or CR₁₀R₁₁;
R₉ is selected from hydrogen, deuterium, methyl, ethyl, monofluoromethyl, difluoromethyl or trifluoromethyl;
R₁₀ is selected from hydrogen, deuterium, fluorine, chlorine, bromine, methyl, ethyl, monofluoromethyl, difluoromethyl or trifluoromethyl;
R₁₁ is selected from hydrogen, deuterium, fluorine, chlorine, bromine, methyl, ethyl, monofluoromethyl, difluoromethyl or trifluoromethyl;
R₁₋₁ is selected from hydrogen, deuterium, fluorine, chlorine, bromine, methyl, ethyl, monofluoromethyl, difluoromethyl or trifluoromethyl;
R₂ is independently selected from hydrogen, deuterium, fluorine, chlorine, bromine, methyl, ethyl, monofluoromethyl, difluoromethyl or trifluoromethyl;
R₂₋₁ is selected from amino, C₁₋₃ alkyl, halo C₁₋₃ alkyl or deuterated C₁₋₃ alkyl, and the amino, C₁₋₃ alkyl, halo C₁₋₃ alkyl or deuterated C₁₋₃ alkyl can be optionally further substituted with one or more of deuterium, halogen, cyano, hydroxyl, nitro, C₁₋₃ alkyl, halo C₁₋₃ alkyl and deuterated C₁₋₃ alkyl;
preferably, R₂₋₁ is selected from isopropyl, -CH(Me)OMe, or -N(Me)₂;
R₄ is selected from halogen, amino, hydroxyl, cyano, nitro, C₁₋₃ alkyl, C₁₋₃ alkoxy, C₁₋₃ hydroxyalkyl, C₂₋₄ alkenyl, C₂₋₄ alkynyl, C₃₋₆ cycloalkyl, 3- to 8-membered heterocyclyl, -S(O)ₘR_{d} or -C(O)R_{d}, and the amino, C₁₋₃ alkyl, C₁₋₃ alkoxy, C₁₋₃ hydroxyalkyl, C₂₋₄ alkenyl, C₂₋₄ alkynyl, C₃₋₆ cycloalkyl and 3- to 8-membered heterocyclyl can be optionally further substituted with one or more R₄₋₁;
R₄₋₁ is independently selected from hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl or 5- to 12-membered heteroaryl; optionally, R₄₋₁ is substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, C₁₋₃ hydroxyalkyl, C₃₋₆ cycloalkyl, 3- to 8-membered heterocyclyl, C₆₋₁₂ aryl, or 5- to 12-membered heteroaryl, and the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, and C₁₋₃ hydroxyalkyl are optionally substituted with one or more of hydrogen, deuterium, oxo, thio, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halo C₁₋₃ alkoxy, or C₁₋₃ hydroxyalkyl; preferably, R₄ is independently selected from fluorine, chlorine, cyano, trifluoromethyl, methyl, ethyl, nitro, hydroxyl, methoxy, -OCD₃, hydrogen, 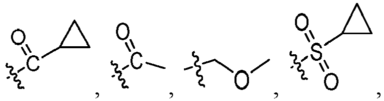 cyclopropyl, 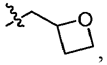 difluoromethyl, 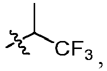 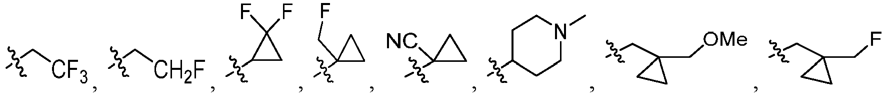 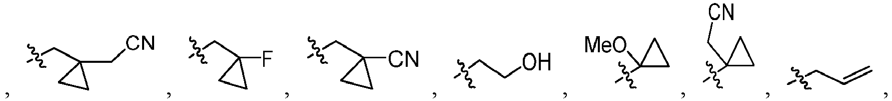 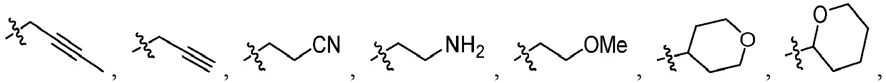 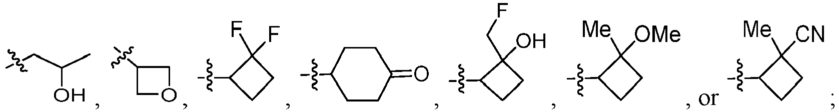
R₆₋₁ is selected from hydrogen, deuterium, oxo, thio, halogen, amino, hydroxyl, cyano, nitro, C₁₋₃ alkyl, C₁₋₃ alkoxy, or C₁₋₃ hydroxyalkyl;
R₆₋₂ is selected from hydrogen, deuterium, oxo, thio, halogen, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, -S(O)ₘRₑ or -C(O)Rₑ, and the amino, C₁₋₃ alkyl, C₁₋₃ alkoxy, C₁₋₃ hydroxyalkyl, C₃₋₆ cycloalkyl and 3- to 8-membered heterocyclyl can be optionally further substituted with one or more of deuterium, halogen, amino, hydroxyl, cyano, nitro, or C₁₋₃ alkyl;
R_{d} is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl, or C₁₋₃ deuteroalkyl;
Rₑ is independently selected from hydrogen, deuterium, amino, hydroxyl, cyano, nitro, C₁₋₃ alkyl, C₁₋₃ alkoxy, C₁₋₃ hydroxyalkyl, C₃₋₆ cycloalkyl, 3- to 6-membered heterocyclyl, C₁₋₃ haloalkyl, or C₁₋₃ deuteroalkyl;
m is 0, 1 or 2;
and y is 0, 1, 2, 3 or 4.

13. The compound or the stereoisomer or pharmaceutically acceptable salt thereof according to claim 1, wherein the specific compounds are as follows: 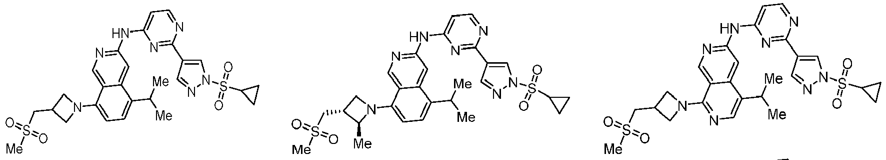 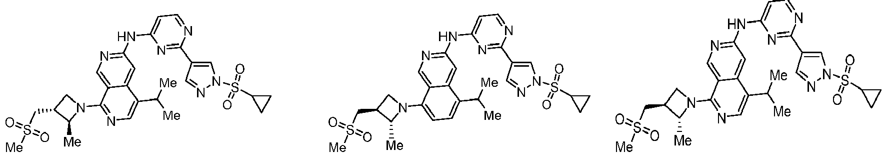 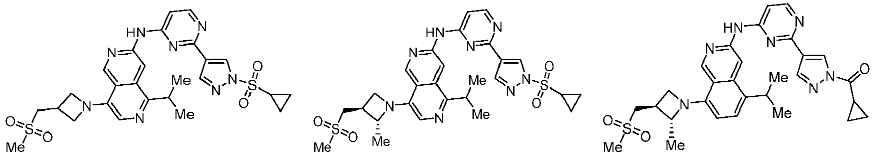 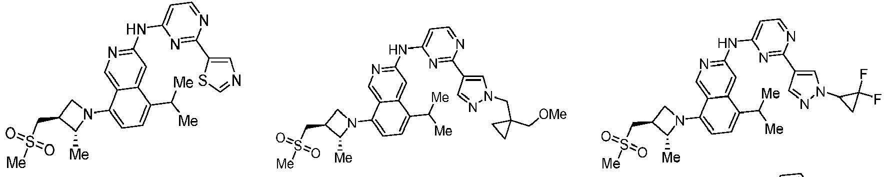 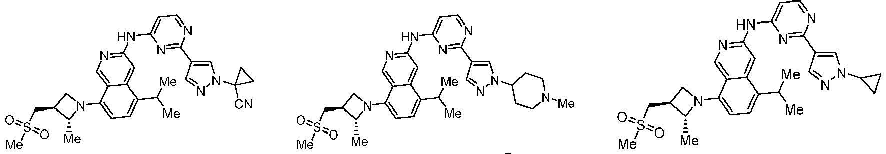 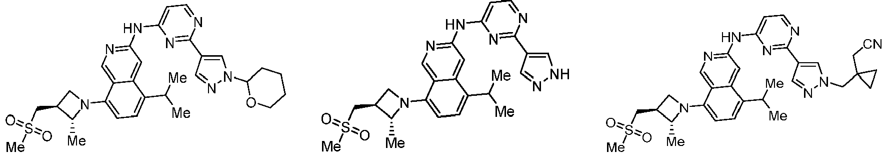 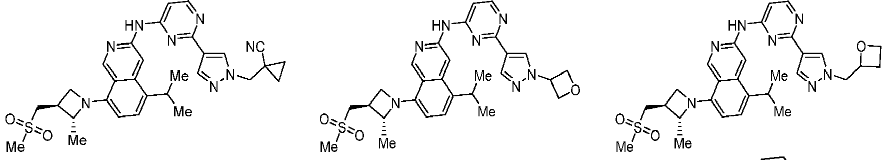 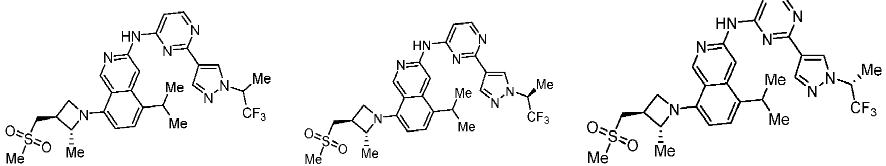 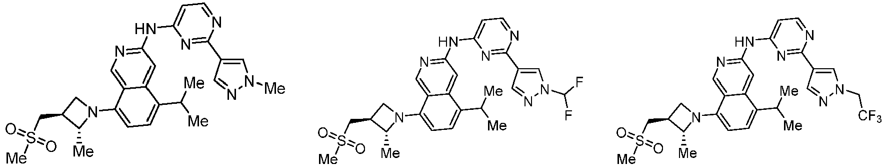 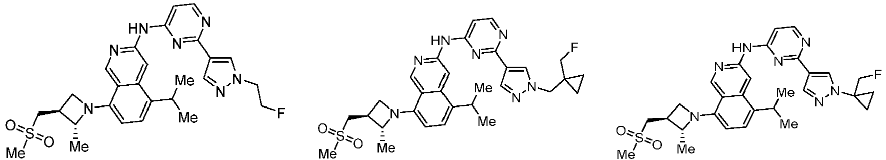 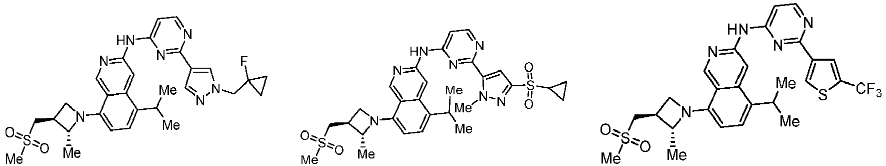 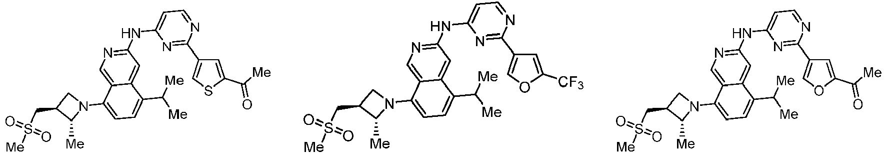 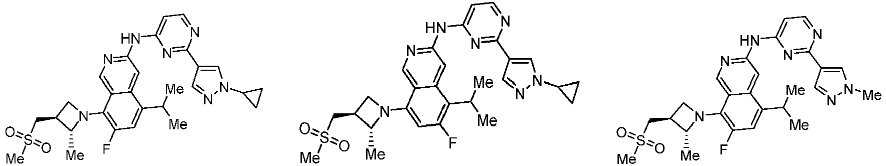 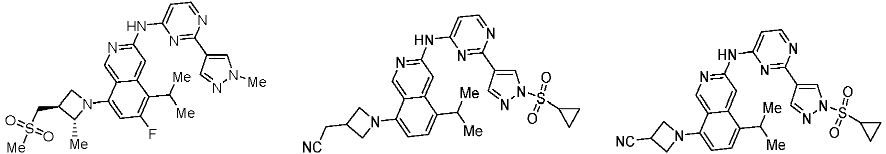 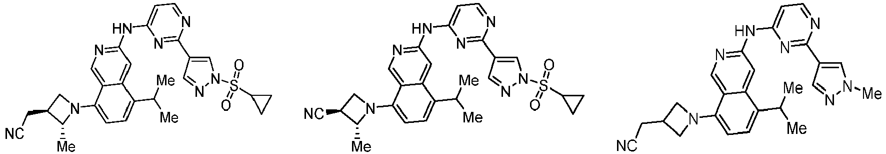 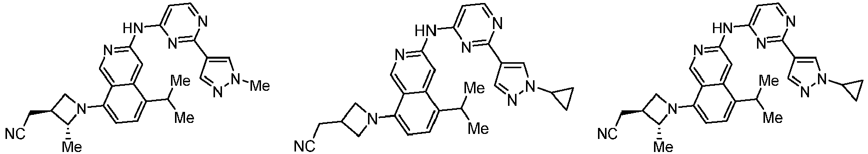 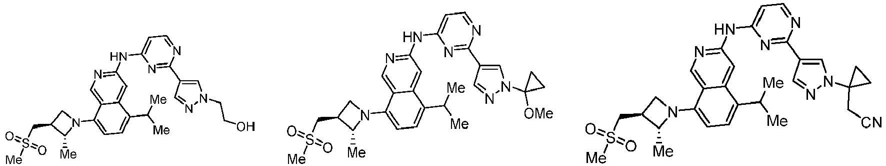 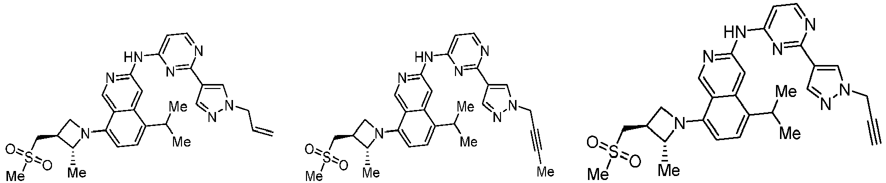 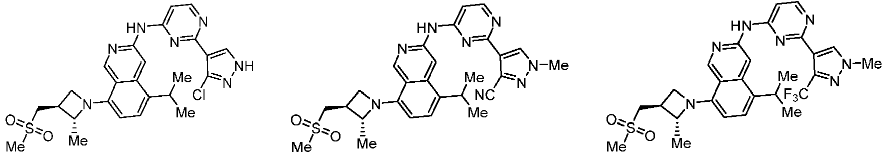 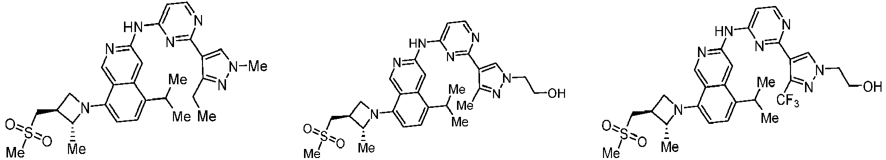 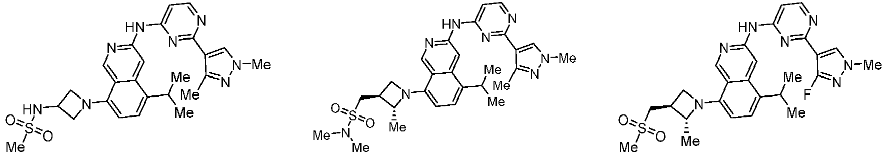 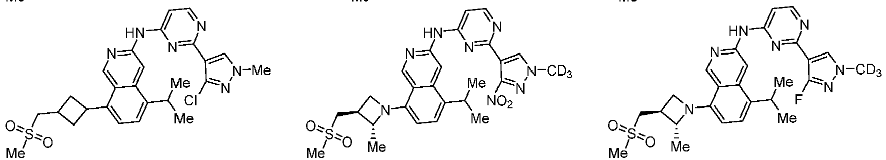 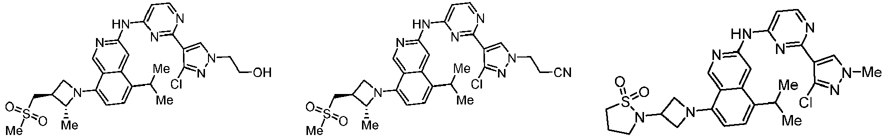 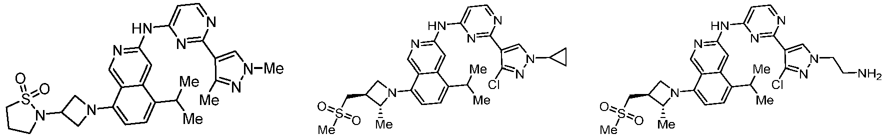 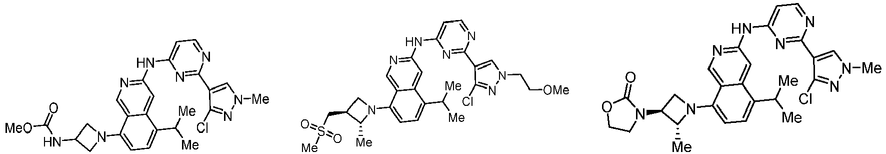 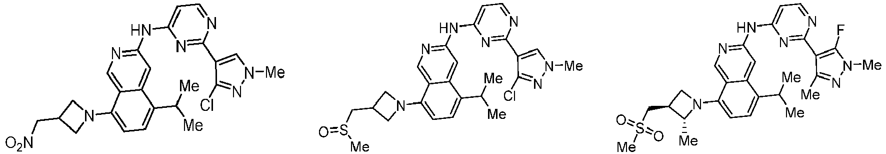 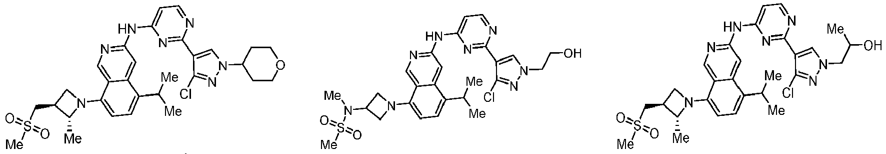 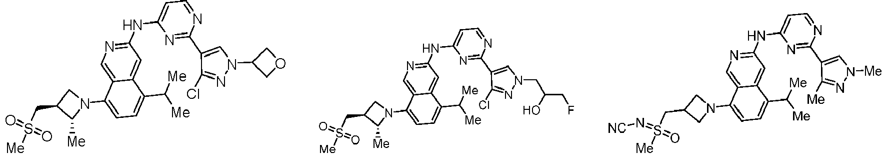 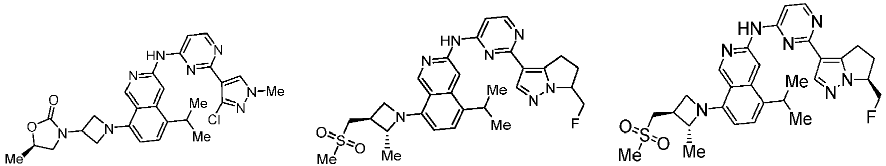 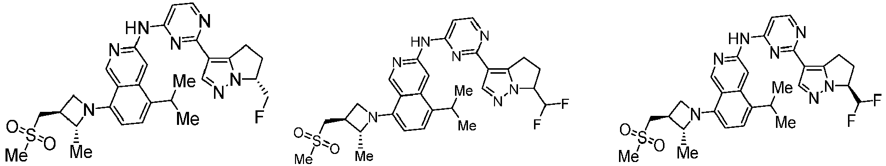 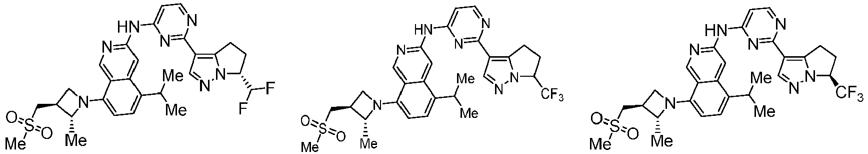 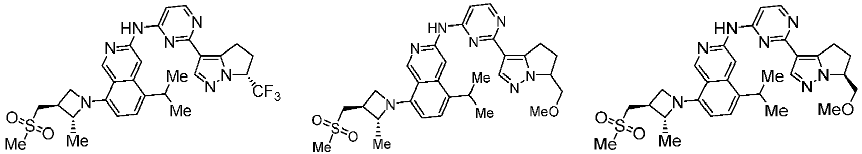 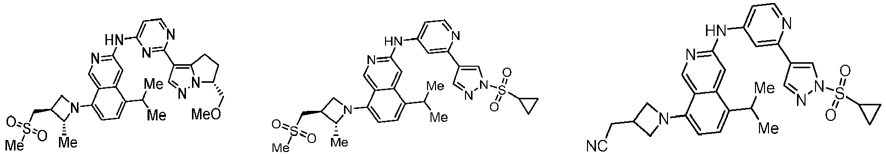 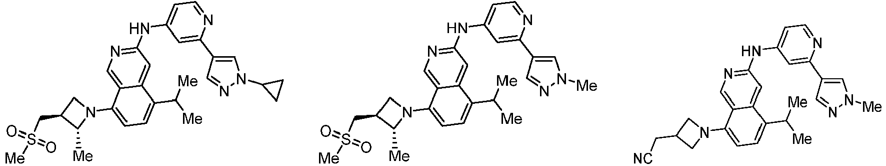 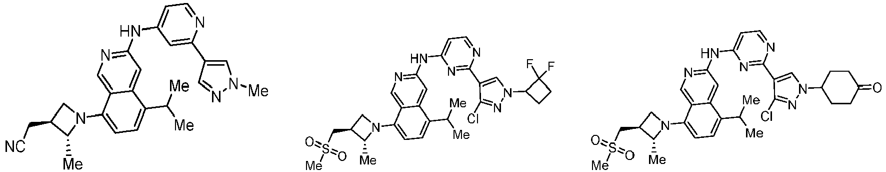 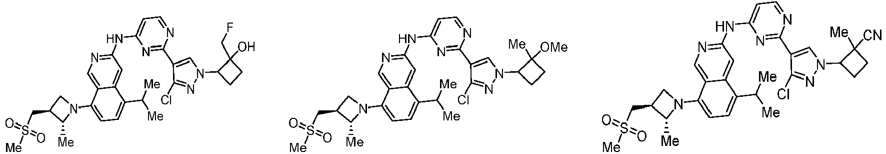 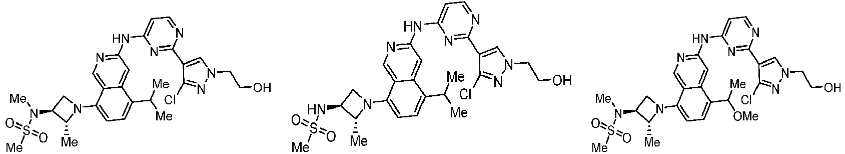 or

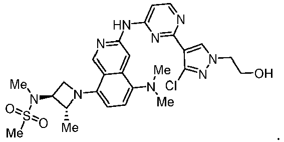

14. Use of the compound or the stereoisomer or pharmaceutically acceptable salt thereof according to any one of claims 1-13 in the preparation of an EGFR inhibitor.

15. The use according to claim 14, wherein the EGFR is a mutated EGFR, preferably with one or more mutations of Del19, L858R, T790M or C797S, and more preferably with L858R/T790M, Del19/T790M, Del19/C797S, L858R/C797S, Del19/T790M/C797S or L858R/T790M/C797S mutation.

16. Use of the compound or the stereoisomer or pharmaceutically acceptable salt thereof according to any one of claims 1-13 in the preparation of a drug for treating cancer, wherein preferably, the cancer is non-small cell lung cancer.
